# EUROPÄISCHE PATENTSCHRIFT

(11) **EP 2 049 671 B1**
(45) Veröffentlichungstag und Bekanntmachung des Hinweises auf die Patenterteilung: **19.12.2012**
(21) Anmeldenummer: 07730192.7
(22) Anmeldetag: 15.06.2007
(51) Int. Cl.: C07K 14/47, C12N 15/63, C12N 15/85, C12N 15/67

(54) **REGULATORISCHE NUKLEINSÄUREELEMENTE**
REGULATORY NUCLEIC ACID ELEMENTS
ÉLÉMENTS RÉGULATEURS D'ACIDE NUCLÉIQUE

(30) Priorität: 26.07.2006 EP 06117862
(43) Veröffentlichungstag der Anmeldung: 22.04.2009
(73) Patentinhaber: Boehringer Ingelheim Pharma GmbH & Co. KG, 55216 Ingelheim am Rhein (DE)
(72) Erfinder: ENENKEL, Barbara, 88447 Warthausen (DE); SAUTTER, Kerstin, 88400 Biberach (DE)
(74) Vertreter: Simon, Elke Anna Maria
(86) Internationale Anmeldenummer: PCT/EP2007/055954
(87) Internationale Veröffentlichungsnummer: WO 2008/012142

(56) Entgegenhaltungen:
- WO-A-97/15664
- WO-A-2004/050879
- DATABASE EMBL 4. März 2003 (2003-03-04), "Mouse DNA sequence from clone RP23-92B18 on chromosome 11." XP002409732 gefunden im EBI Database accession no. BX284634

## Beschreibung

### HINTERGRUND DER ERFINDUNG

### TECHNISCHES GEBIET

Die Erfindung betrifft cis-aktive Nukleinsäuresequenzen, sogenannte TE-Elemente. Die TE-Elemente stammen vorzugsweise aus dem CHO-Genom. Deren Einsatz in z.B. Expressionsvektoren ermöglicht in stabilen Zellpopulationen eine im Vergleich zu bisher verwendeten Vektoren eine mindestens doppelt so hohe Expression eines Gens von Interesse in einem beliebigen Chromosomenlokus.

### HINTERGRUND

Säugerzellen sind die bevorzugten Wirtszellen zur Produktion komplexer biopharmazeutischer Proteine, da die post-translational durchgeführten Modifikationen sowohl in funktionaler als auch in pharmakokinetischer Hinsicht humankompatibel sind. Vornehmlich relevante Zelltypen sind Hybridomas, Myelomas, CHO- (*Chinese Hamster Ovary*) Zellen und BHK- (*Baby Hamster Kidney*) Zellen. Die Kultivierung der Wirtszellen erfolgt zunehmend unter serum- und proteinfreien Produktionsbedingungen. Gründe hierfür sind die damit verbundene Kostenreduktion, die geringere Interferenz bei der Aufreinigung des rekombinanten Proteins sowie die Reduktion des Potentials zur Einführung von Pathogenen (z.B. Prionen, Viren). Der Einsatz von CHO-Zellen als Wirtszellen findet immer weitere Verbreitung, da diese Zellen sich an Suspensionswachstum in serum- und proteinfreiem Medium adaptieren lassen und zudem von den regulatorischen Behörden als sichere Produktionszellen angesehen und akzeptiert werden.

Zur Erzeugung einer stabilen Säugerzelllinie, die ein heterologes Gen von Interesse exprimiert, wird das heterologe Gen in der Regel gemeinsam mit einem selektierbaren Markergen, wie z.B. Neomycin-Phosphotransferase (NPT), durch Transfektion in die gewünschte Zelllinie eingebracht. Das heterologe Gen und das selektierbare Markergen lassen sich in einer Wirtszelle, ausgehend von einem einzelnen oder von separaten, co-transfizierten Vektoren, exprimieren. Zwei bis drei Tage nach der Transfektion werden die transfizierten Zellen in Medium überführt, das ein selektives Agens enthält, z.B. G418 bei Verwendung des Neomycin-Phosphotransferase-Gens (NPT-Gen), und für einige Wochen unter diesen selektiven Bedingungen kultiviert. Die hochwachsenden resistenten Zellen, die die exogene DNA integriert haben, können isoliert und hinsichtlich der Expression des gewünschten Genprodukts (Gen von Interesse) untersucht werden.

Für die biopharmazeutische Produktion sind Zelllinien mit hoher und stabiler Produktivität notwendig. Die Expressionsvektoren für Produktionszellen werden mit starken, meist konstitutiv exprimierenden Promotoren und Enhancem wie beispielsweise CMV-Enhancer und -Promotor ausgestattet, um eine hohe Produktexpression zu ermöglichen. Da die Expression des Produktes über einen möglichst langen Zeitraum gewährleistet werden muss, werden Zellen selektioniert, die das Produktgen stabil in ihr Genom integriert haben. Dies erfolgt mit Selektionsmarkern wie z.B. Neomycinphosphotransferase (NPT) und Dihydrofolatreduktase (DHFR).

Durch die zufällige Integration der Expressionsvektoren in das Wirtszellgenom erhält man Zellen mit unterschiedlich hoher Expression des gewünschten Genprodukts, da dessen Expression nicht alleinig durch die Stärke des vorgeschalteten Promotors oder der Promotor/Enhancer-Kombination bedingt wird. Die am Integrationsort vorliegende Chromatinstruktur kann die Höhe der Expression sowohl in negativer als auch in positiver Weise beeinflussen. Zunehmend werden deshalb auch cis-aktive Elemente, die die Expression auf Chromatinebene positiv beeinflussen, in Expressionsvektoren integriert. Dazu gehören Locus Control Regions (LCR), die z.B. in der 5'-Region der β-Globingene (Li et al., 2002) und in der 3'-Region des TCRa-Gens vorkommen. Sie verursachen eine hohe gewebespezifische Expression eines gekoppelten Transgens im Chromatin, die sich durch Positionsunabhängigkeit und Kopienzahlabhängigkeit auszeichnet. Diese Eigenschaften deuten darauf hin, dass LCRs fähig sind, in ihrem nativen Gewebe Chromatin zu öffnen (Ortiz et al., 1997). Es gibt verschiedene Formen von β-Thalassämie, bei denen der β-Globin-Lokus intakt ist, aber nicht exprimiert wird. Der Grund für die fehlende Expression ist eine große Deletion in 5'-Richtung der β-Globingene. Die Deletion dieser β-Globin-LCR führt zu einer geschlossenen Chromatinkonformation, die sich über den ganzen Lokus erstreckt und zu einer Unterdrückung der Genexpression führt (Li et al., 2002). LCRs kolokalisieren mit DNAse I-hypersensitiven Stellen (HS) im Chromatin exprimierender Zellen. Das Vorkommen von HS deutet ebenfalls auf offenes Chromatin hin. Die HS enthalten eine Reihe verschiedener allgemeiner und gewebespezifischer Bindungsstellen für Transkriptionsfaktoren. Durch die Interaktion der Transkriptionsfaktoren mit der DNA entsteht die offene Chromatinstruktur der HS (Li et al., 2002). Von manchen LCRs ist bekannt, dass sie sich aus mehreren HS zusammensetzen, deren Funktionen mehr oder weniger voneinander abgegrenzt werden können. Das TCRα-Gen zum Beispiel wird unter endogener Kontrolle nur in T-Zellgewebe exprimiert. Der Lokus existiert je nach Gewebe und Expressionsstatus in verschiedenen Chromatinmodi. Er besitzt im 3'-Bereich eine Locus Control Region, die acht HS aufweist. HS 2 - 6, ein 6 kb großes Teilfragment der LCR, wirkt chromatinöffnend und ist nicht gewebespezifisch. Die Gewebespezifität wird der T-zellspezifischen Expression im Thymus durch die HS 7, 8 und 1 (3 kb) verliehen. Nur in der vollständigen Kombination aller HS ist die TCRαLGR funktionell komplett (Ortiz et al., 1997). Eine genauere Unterteilung und Spezifikation der einzelnen HS-Funktionen der TCRαLCR ist in (Ortiz et al., 1999) nachzulesen. Dieses Beispiel zeigt, dass LCRs funktionell sehr komplex sind und sich aus verschiedenen Kontrollelementen wie Enhancem, Silencem und Isolatoren zusammensetzen können. Weitere Beispiele für eine Aufteilung der LCR-Funktionen auf verschiedene Domänen sind der TCRγ-Lokus und der β-Globin-Lokus. Ersterer setzt sich aus der DNAse I-hypersensitiven Stelle HsA und dem Enhancer 3'E_{Cγ1} zusammen. Der TCRy-LCR wird außer den üblichen Aufgaben noch eine Rolle bei der Rekombination der TCRγ-Gene zugeschrieben (Baker et al., 1999). Der β-Globin-Lokus weist fünf HS mit unterscheidbaren Funktionen auf, die für ihr vollständiges Funktionieren außerdem den gewebespezifischen Promotor benötigen. Eine weitere wichtige Rolle könnten LCRs bei der gewebespezifischen Demethylierung der DNA spielen, da DNA-Methylierung eine geschlossene Chromatinstruktur und die Inaktivierung von Genen verursacht. Möglich wäre auch ein Wirkmechanismus, der durch eine erhöhte Histonacetylierung die Genexpression aktiviert (Li et al., 2002).

Scaffold/Matrix Attachment Regions (S/MARs) sind DNA-Sequenzen, die mit hoher Affinität *in vitro* an Bestandteile der Matrix oder des Gerüsts des Zellkerns binden. Sie bilden die strukturellen und möglicherweise auch funktionellen Grenzen von Chromatindomänen (Zahn-Zabal et al., 2001). S/MARs sind fähig, mit Enhancem zu interagieren sowie die Zugänglichkeit der DNA im Chromatin lokal zu erhöhen und können auf diese Weise die Expression stabil integrierter, heterologer Gene in Zelllinien, transgenen Tieren und Pflanzen steigern (Klehr et al., 1991; Stief et al., 1989; Jenuwein et al., 1997; Zahn-Zabal et al., 2001). Sie können allerdings einen chromosomalen Lokus nicht vollständig von naheliegenden Elementen abschirmen, um eine positionsunabhängige Expression zu ermöglichen (Poljak et al., 1994). Der Effekt der MARs kann dazu genutzt werden, den Anteil (hoch-) exprimierender Zellklone bzw. transgener Tiere in einem Transfektionsexperiment zu erhöhen (McKnight et al., 1992; Zahn-Zabal et al., 2001). Es wurde jedoch auch von MARs berichtet, die keine Hochexpression vermitteln, aber eine wichtige Rolle in der korrekten Regulierung entwicklungsspezifischer Gene spielen (McKnight et al., 1992).

Isolatoren sind definiert als neutrale Grenze zwischen sich gegenseitig beeinflussenden Nachbarregionen, z. B. zwischen aktivem und inaktivem Chromatin (Grenzelemente). Sie können die Wirkung von Enhancern abgrenzen bzw. ganze DNA-Domänen dagegen isolieren und stabil transfizierte Reportergene gegen Positionseffekte abschirmen (Bell and Felsenfeld, 1999; Udvardy, 1999). So verleihen diese Elemente eine Unabhängigkeit der Expression von der genomischen Position. Außerdem können sie das Silencing von Transgenen bei Abwesenheit von Selektionsdruck verhindern (Pikaart et al., 1998). Eine weitere vermutete Funktion von Isolatoren ist die Abgrenzung von Replikationstenitorien (Bell and Felsenfeld, 1999). Die ersten Isolatoren, die beschrieben wurden, sind *scs* und *scs* aus Drosophila. Sie stellen die Grenze für die *hsp70* Hitzeschockgene dar und unterdrücken Positionseffekte (Udvardy et al., 1985).
Als ein weiteres Element mit isolierender Funktion wurde ein GC-reiches Fragment aus dem *dhfr*-Gen (Chinesischer Hamster) gefunden, das CpG-Inseln enthält (Poljak et al., 1994). Das Fragment allein zeigte keinerlei Einfluss auf die Reportergenexpression. Zwischen einem expressionsfördernden SAR und dem Reportergen gelegen, konnte das Fragment jedoch die expressionssteigernde Wirkung des SAR-Elements weitestgehend verhindern. Möglicherweise blockiert dieses GC-reiche Fragment den chromatihöffmenden Mechanismus des SAR-Elements und fungiert folglich als Isolator. Elemente mit ausgedehnten CpG-Inseln werden mit einer höheren Wahrscheinlichkeit methyliert, da sie von einer DNA-Methyltransferase erkannt werden, die Cytosin zu 5-Methylcytosin umwandelt. Als Folge wird inaktives Chromatin gebildet (Poljak et al., 1994).

Aronow und Mitarbeiter definierten im ersten Intron des humanen ADA-Gens (Adcnosindeaminase) ein neues regulatorisches Element, das wesentlich zur Genkopienzahl-abhängigen und positionsunabhängigen Expression beiträgt (Aronow et al., 1995). Das Element ist bis zu 1 kb groß und nur funktionell, wenn es einen 200 bp großen T-zellspezifischen Enhancer flankiert. Ist nur eines der beiden Segmente vorhanden oder sind die Segmente bezüglich der Reihenfolge und Orientierung falsch angeordnet, ist das Element funktionslos, da dadurch die Bildung von DNase I-hypersensitiven Stellen am Enhancer verhindert wird.

Die Firma Cobra Therapeutics beschreibt im Patent WO 02/081677 ein weiteres chromatinbecinflussendes Element. Die Ubiquitous Chromatin Opening Elements (UCOEs) sind verantwortlich für eine offene Chromatinstruktur in chromosomalen Regionen mit ubiquitär exprimierten Haushaltsgenen (humanes hnRNP A2-Gen, humanes β-Actin-Gen, humanes PDCD2-Gen). Alle diese Gene besitzen CpG-reiche Inseln in den untranslatierten Bereichen, die verhältnismäßig schwach methyliert sind. Die fehlende Methylierung von CpG-Inseln deutet darauf hin, dass es sich an dieser Stelle um aktives Chromatin handelt. Die UCOEs verhelfen zu einer Expressionsstärke, die von der genomischen Umgebung und von der Zell- oder Gewebeart unabhängig ist.

Die Firma Immunex beschreibt ebenfalls cis-aktive DNA-Sequenzen, die eine Erhöhung der Expression bewirken (US 6,027,915, US 6,309,851). Das als Expression Augmenting Scquence Element (EASE) bezeichnete Element fördert eine hohe Expression von rekombinanten Proteinen in Säugerzellen, ist in transienten Expressionssystemen nicht aktiv und besitzt nicht die typischen Sequenzeigenschaften wie sie in LCRs und S/MARs gefunden wurden. Es handelt sich auch nicht um eine Sequenz, die für ein transaktivierendes Protein kodiert, da sie kein offenes Leseraster enthält. Das Fragment ist 14,5 kb groß, stammt aus der genomischen DNA von CHO-Zellen und kann die Expression eines stabil integrierten Reportergens achtfach steigern. Über 50% der Aktivität des Elements sind auf ein 1,8 kb großes Segment begrenzt, wobei die ersten 600 Basenpaare dieses Segments für die korrekte Funktion unentbehrlich sind. Eine zusätzliche Eigenschaft von Sequenzabschnitten mit hoher EASE-Aktivität ist das Vorhandensein von mehreren HMG-I(Y)-Bindungsstellen. HMG-I(Y)-Proteine gehören zur Familie der High Mobility Group Nicht-Histon-Chromatin-Proteine. Sie werden auch als "architektonische Transkriptionsfaktoren" bezeichnet und bilden eine neue Kategorie der trans-Regulatoren von Säugergenen. HMG-I(Y)-Proteine erkennen AT-reiche Sequenzen und binden mit ihren sogenannten AT-Haken (DNA-bindende Domänen) in der kleinen DNA-Furche. Das kann zu lokalen Veränderungen der DNA-Topologie und in Folge dessen zu einer veränderten Genexpression führen. Die Autoren des Patents US 6,309,841 vermuten, dass die Auswirkungen von EASE mit der MTX-induzierten Amplifikation des integrierten Plasmids in Verbindung stehen. Bei der MTX-induzierten Gen-Amplification kommt es zu sogenannten Breakage-Fusion-Bridge-Zyklen. Dabei ist eine Rolle der HMG-I(Y)-Proteine bei den strukturellen Veränderung der DNA, die zur Entstehung und Behebung der DNA-Brüche führen, gut vorstellbar.

Weitere Elemente zur Steigerung der Genexpression in Säugetierzellen wurden in Kwaks et al., 2003 beschrieben. Diese so genannten STAR-Elemente (Stimulatory and Anti-Repressor Elements) stammen aus dem Screening einer humanen Genbibliothek mit 500 bis 2100 bp großen DNA-Fragementen. Das Screening wurde mit einem speziell konstruierten Reporterplasmid durchgeführt. Die Expression des Reportergens war nur dann möglich, wenn es funktionell mit einem Anti-Repressor-Element aus der humanen Genbank verknüpft war. Mit den derart erhaltenen STAR-Elementen konnten die Autoren Transgene vor Positionseffekten im Genom von Säugerzellen schützen. Ein Vergleich mit dem Mausgenom zeigte, dass die meisten dieser STAR-Elemente sowohl im humanen als auch im murinen Genom vorkommen und innerhalb dieser beiden Spezies hoch konserviert sind.

Ein großes Problem bei der Etablierung von Zelllinien mit hoher Expression des gewünschten Proteins ergibt sich aus der willkürlichen und ungerichteten Integration des rekombinanten Vektors in transkriptionsaktive oder -inaktive Loci des Wirtszellgenoms. Dadurch erhält man eine Population von Zellen, die völlig unterschiedliche Expressionsraten des heterologen Gens aufweisen, wobei die Produktivität der Zellen in der Regel einer Normalverteilung folgt. Zur Identifizierung von Zellklonen, die eine sehr hohe Expression des heterologen Gens von Interesse aufweisen, muss deshalb eine Vielzahl von Klonen überprüft und getestet werden, resultierend in einem hohen Zeit-, Arbeits- und Kostenaufwand. Optimierungen des zur Transfektion eingesetzten Vektorsystems zielen deshalb darauf ab, die Transkription eines stabil integrierten Transgens durch die Verwendung geeigneter *cis*-aktiver Elemente überhaupt erst zu ermöglichen oder auch zu steigern. Zu den cis-aktiven Elementen, die ihre Wirkung auf Chromatinebene ausführen, gehören z.B. die bereits beschriebenen Locus Control Regions, Scaffold/Matrix Attachment Regions, Isolatoren u.a.. Manche dieser Elemente schirmen bcstimmte Gene gegen Einflüsse des umliegenden Chromatins ab. Andere zeigen eine Enhancer-ähnliche Wirkung, wobei diese jedoch auf stabil integrierte Konstrukte beschränkt ist. Wieder andere Elemente vereinen mehrere dieser Funktionen in sich. Oft ist die genaue Zuordnung zu einer bestimmten Gruppierung nicht eindeutig möglich.
In stabilen Zelllinien unterliegt die Expression des transgenen Produktgens also in erheblicher Weise chromosomalen Positionseffekten. Dieses Phänomen beruht auf dem Einfluss der Chromatinstruktur und/oder dem Vorhandensein intrinsischer regulatorischer Elemente am Integrationsort der Fremd-DNA. Dies führt zu sehr variablen Expressionsieveln. Bei der Selektion von Zellen entstehen deshalb häufig Klone mit sehr geringer oder komplett fehlender Produktexpression. Diese chromosomalen Positionseffekte sind auch die Ursache dafür, dass die Generierung von stabilen Produktionszelllinien, die einen hohen Level eines therapeutischen Proteins exprimieren, in der Regel ein zeit-, kapazitäts- und kostenaufwändiger Prozess ist. Stabile Zelllinien mit hoher Produktivität werden üblicherweise durch Selektion mit positiven Selektionsmarkem, häufig kombiniert mit Agens-induzierter Genamplifikation (z.B. Dihydrofolatreduktase/Methotrexat oder Glutaminsynthetase/Methioninsulfoximin) erzeugt. Die Pools und Klone, die bei dieser Selektionsstrategie entstehen, werden in einem aufwändigen Screeningprozess auf hohe und stabile Expression untersucht. Die Mehrzahl der Klone produziert kein oder nur durchschnittliche Mengen an Produkt, nur wenige sind Hochproduzenten. Der Anteil an Hochproduzenten in einer gemischten Population kann beispielsweise durch eine Mutation im Selektionsmarker erhöht werden (Sautter und Enenkel, 2005, WO 2004/050884). Eine weitere Erhöhung der spezifischen Produktivität jedes einzelnen Klons sowie des Anteils an Hochproduzenten innerhalb einer transfizierten Zellpopulation ist jedoch wünschenswert.

Die spezifische Produktivität von stabil transfizierten Zellen, insbesondere CHO- oder anderen produktionsrelevanten Zellen, und der Anteil an Hochproduzenten eines Transfektionsansatzes sollen gesteigert werden. Dies soll letztendlich zu einer effizienteren Zelllinienentwicklung führen. Dadurch könnten in kürzerer Zeit mehr und höher produzierende Zelllinien etabliert werden und somit Arbeitsaufwand, Zeit und Kosten eingespart werden.

### ZUSAMMENFASSUNG DER ERFINDUNG

Die vorliegende Erfindung betrifft regulatorische Nukleinsäuren (genannt .,TE-Elemente"), insbesondere eine Nukleinsäure, welche TE-13 (SEQ ID Nr. 15) enthält oder ein Fragment von TE-13 (SEQ ID Nr. 15) oder deren komplementäre Nukleotidsequenzen oder ein Derivat von TE-13 (SEQ ID Nr. 15) oder deren komplementäre Nukleotidsequenzen, wobei die Nukleinsäure, das Fragment, das Derivat oder deren komplementäre Nukleotidsequenzen bei chromosomaler Integration zu einer Steigerung der Transkription bzw. Expression eines Gens von Interesse in einem Expressionssystem führt. und wobei das Derivat mindestens 85% Sequenzidentität aufweist. Die vorliegende Erfindung betrifft insbesondere eine Nukleinsäure mit der SEQ ID Nr. 1. Es konnte überraschenderweise gezeigt werden, dass der Einsatz eines solchen TE-Elements auf einem Expressionsvektor in Verbindung mit einem Promotor, einem Produktgen, einem Selektionsmarker und optional einem Enhancer bei stabiler Integration in ein Wirtsgenom, wie beispielsweise dem CHO-DG44-Genom, die chromosomalen Positionseffekte überkommt, abschirmt oder aufhebt. Dadurch wird sowohl der Anteil an Hochproduzenten eines Transfektionsansatzes als auch der absolute Expressionslevel gesteigert.

Die Erfindung betrifft ferner eukaryontische Expressionsvektoren, die eine erfindungsgemäße Nukleinsäure. enthalten, bevorzugt die TE-Elemente TE-01 (SEQ ID Nr. 3), TE-02 (SEQ ID Nr. 4), TE-07 (SEQ ID Nr. 9), TE-08 (SEQ ID Nr. 10), TE-10 (SEQ ID Nr. 12), TE-11 (SEQ ID Nr. 13), TE-12 (SEQ ID Nr. 14), TE-13 (SEQ ID Nr. 15), TE-15 (SEQ ID Nr. 17), TE-17 (SEQ ID Nr. 19), TE-18 (SEQ ID Nr. 20). Aufgrund ihrer geringeren Größe besonders bevorzugt sind TE-07 TE-08 sowie TE-13 (SEQ ID Nr. 15).
Die SEQ ID Nr. 1 entstammt einem stromaufwärts der Kodierregion des Ubiquitin/S27aGens gelegenen Sequenzbereichs, der aus CHO-Zellen isoliert wurde, wobei das Gen für ein essentielles Protein im Ribosomenstoffwechsel der Zelle kodiert.

Im Vergleich zu den bisher verwendeten Expressionsvektoren ermöglicht die zusätzliche Einbringung der cis-aktiven TE-Elemente in Expressionsvektoren eine überraschenderweise bis um Faktor 7 höhere spezifische Produktivität von stabil transfizierten Zellpools, insbesondere von CHO-DG44 Zellpools. In transienten Transfektionen von CHO-DG44 Zellpools ist hingegen bei Einbringung der TE-Elemente keine Produktivitätssteigerung zu erzielen. Somit beruht die beobachtete Produktivitätssteigerung in den stabilen Zellpools nicht auf einem in den TE-Elementen vorhandenen Enhancer. Für die durch TE-Elemente bedingte Steigerung der Produktivität ist eine chromsomale Integration also zwingend erforderlich. Dies ist ein Hinweis darauf, dass TE-Elemente negative chromosomale Positionseffekte unterdrücken, abschirmen oder aufheben können. Es konnten somit *cis-*aktive Elemente erzeugt und identifiziert werden, die sich durch ihre besondere Eignung zur Selektion und Anreicherung von hochproduzierenden Zellen auszeichnen und somit den Zeit-, Kosten- und Kapazitätsaufwand in der Isolierung und Identifizierung von Hochproduzentenklonen reduzieren können.

Anwendungsmöglichkeiten der Erfindung bieten sich z.B. in der Entwicklung von hochproduzierenden Zelllinien, die beispielsweise bei der Herstellung von Biopharmazeutika, in analytischen zellbasierten Assays, in High Throughput Screenings von Substanzen oder bei der Erzeugung von rekombinanten Proteinprodukten für NMR-Spektroskopie, andere Assays etc. benötigt werden. Durch die höhere spezifische Produktivität und die Reduktion von Zellen, die kein oder wenig Produkt exprimieren, können in kürzerer Zeit mehr und höher produzierende Zelllinien etabliert werden und somit Arbeitsaufwand und Kosten eingespart werden. Andere Anwendungsmöglichkeiten liegen in der Erzeugung von robusten, verbesserten Wirtszelllinien (z.B. Einbringen von Anti-Apoptose- oder Glykosylierungsgenen), von transgenen Tieren oder Pflanzen sowie in der Gentherapie.

Die Erfindung ergibt sich nicht aus dem Stand der Technik.

Bei der Nukleinsäure mit der SEQ ID Nr. 1 handelt es sich um eine Nukleinsäuresequenz, die aus dem Genom von Chinesischen Hamstern (*Cricetulus griseus*) isoliert wurde. Sie entstammt einem stromaufwärts der Kodierregion des Ubiquitin/S27a-Gens gelegenen Sequenzbereichs.
Die Nukleinsäure mit der SEQ ID Nr.1 weist einen durchschnittlichen GC-Gehalt von 44% auf und enthält keine längeren Passagen von GC-Wiederholungen. Dieser GC-Gehalt ist vergleichbar mit dem für genomische DNA von Säugetieren beschriebenen durchschnittlichen GC-Gehalt von etwa 40% (Delgado et al., 1998). Die Suche mit newcpgseek (EMBOSS sequence analysis package) nach CpG-reichen Regionen resultierte lediglich in fünf, sehr kurzen Sequenzregionen, die eine absolut erhöhte Frequenz von CpG Dimeren und/oder einen erhöhten Anteil von CpG im Verhältnis zu GpC aufweisen: Nukleotide 2242 - 2259 (18 bp), 3129 - 3146 (18 bp), 3215 - 3240 (26 bp), 3417 - 3461 (44 bp) und 3658 - 3788 (131 bp) der SEQ ID Nr. 1. Die Rechercheergebnisse zeigen, dass die Nukleinsäuresequenz keine ausgedehnten CpG Inseln enthält.
Des Weiteren enthält die SEQ ID Nr. 1 zwei Tandem Repeats (Nukleotide 2179 - 2244 und Nukleotide 1027 - 1080) und zwei Inverted Repeats (Nukleotide 8 - 47 und Nukleotide 1726 - 1766), die mit den EMBOSS-Porgrammen etandem und einverted identifiziert wurden. TE-08 enthält demgemäß einen inverted repeat. Im Sequenzbereich zwischen 1 bp und 1578bp befinden sich demgemäß ein tandem repeat und ein inverted repeat.

Teile der Nukleinsäure mit der SEQ ID Nr.1 wurden auch schon in WO 97/15664 beschrieben: die Nukleotide 1579 bis 3788 der SEQ ID Nr. 1 entsprechen den Nukleotiden 1 bis 2201 der SEQ ID Nr.5 aus WO 97/15664, jedoch mit einer Abweichung. Bei der Herstellung der erfindungsgemäßen SEQ ID Nr.1 wurden klonierungsbedingt zusätzliche 4 Nukleotide eingefügt, die aus einer Auffüllreaktion einer vorhandenen EcoRI-Schnittstelle entstanden sind. Diese Insertion der zusätzlichen 4 Nukleotide erfolgte zwischen Nukleotid 357 und 358 der SEQ ID Nr.5 aus WO 97/15664. Die Nukleotide 1 bis 1578 der Nukleinsäuresequenz mit der SEQ ID Nr. 1 aus der vorliegenden Erfindung stellen jedoch neue, bisher unbekannte Sequenzbereiche dar, die im Rahmen dieser Erfindung isoliert wurden. In WO 97/15664 wurde auch nicht offenbart, dass die SEQ ID Nr.1 aus der vorliegenden Erfindung, oder andere erfindungsgemäße Nukleinsäuren, die Transkription bzw. Expression eines Gens von Interesse unabhängig vom chromosomalen Integrationsort steigern, wenn sie funktionell mit einer Promotor/Enhancer-Kombination verknüpft werden, die die Transkription des funktionell verknüpften Gens von Interesse ermöglicht. In WO 97/15664 wird vielmehr die Verwendung von 5'UTR-Sequenzen des Ubiquitin/S27a-Gens als Promotor offenbart, wobei der Sequenzbereich von Position - 161 bis - 45 gemäß Abbildung 5 in WO 97/15664 für eine Promotoraktivität essentiell ist. Dieser Sequenzbereich ist in der erfindungsgemäßen Nukleinsäure der SEQ ID Nr. 1 und einem davon abgeleiteten Fragment mit der SEQ ID Nr.2 nur noch partiell erhalten (Position - 161 bis -89 gemäß Abbildung 5 in WO 97/15664). Andere erfindungsgemäße Nukleinsäuren enthalten diesen Sequenzbereich überhaupt nicht mehr.
Weiterhin weist die erfindungsgemäße Nukleinsäure der SEQ ID Nr. 1 bei Anwendung von Standard Alignment-Algorithmen, wie beispielsweise BLAST, keinerlei Sequenzhomologien zu den in den folgenden Patentanmeldungen beschriebenen Nukleinsäuresequenzen auf, die ebenfalls in *cis* die Expression auf Chromatinebene positiv beeinflussen können:
a) UCOE-Nukleinsäuresequenzen aus WO 00/05393
b) EASE-Nukleinsäuresequenzen aus US 6,309,841
c) STAR-Nukleinsäuresequenzen aus WO 03/004704
Auch mit komplexeren Alignment-Strategien finden sich keine ausgedehnten Sequenzhomologien zu diesen Nukleinsäuresequenzen a) bis c).

### BESCHREIBUNG DER ABBILDUNGEN

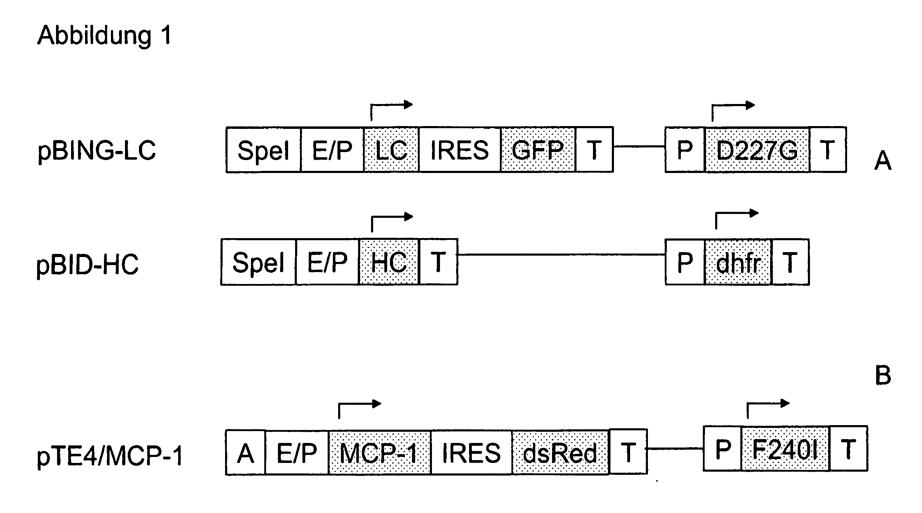

### ABBILDUNG 1: SCHEMATISCHE DARSTELLUNG DER BASISVEKTOREN

Die unter A dargestellten Vektoren wurden zur Expression eines rekombinanten, monoklonalen IgG1 Antikörpers in CHO-DG44-Zellen verwendet. Bei "E/P" handelt es sich in diesem Falle um eine Kombination aus CMV-Enhancer und Hamster Ubiquitin/S27a-Promotor, bei "P" lediglich um ein Promotorelement und bei "T" um ein Terminationssignal für die Transkription, das zur Polyadenylierung der transkribierten mRNA benötigt wird. Die Position und Richtung der Transkriptionsinitiation innerhalb jeder Transkriptionseinheit wird durch einen Pfeil angezeigt. Zur Klonierung von TE-Elementen vor der Promotor/Enhancer-Kombination eine SpeI-Schnittstelle vorhanden ("Spel"). Der amplifizierbare Selektionsmarker Dihydrofolat-Reduktase ist mit "dhfr" abgekürzt. Der Selektionsmarker Neomycin-Phosphotransferase enthält die Punktmutation D227G und ist in der Abbildung entsprechend mit "D227G" abgekürzt. Das aus dem Enccphalomyocarditis-Virus stammende "IRES"-Element dient als interne Ribosomenbindungsstelle innerhalb der bicistronischen Transkriptionseinheit und ermöglicht die Translation des nachfolgenden grünen fluoreszierenden Proteins "GFP". "HC und "LC" kodieren für die schwere bzw. leichte Kette eines humanisierten monoklonalen IgG1 Antikörpers.
Der unter B dargestellte Vektor wurde zur Expression des rekombinanten Proteins MCP-1 in CHO-DG44-Zellen verwendet. Bei "E/P" handelt es sich um eine Kombination aus CMV-Enhancer und CMV-Promotor, bei "P" lediglich um ein Promotorelement und bei "T" um ein Terminationssignal für die Transkription, das zur Polyadenylierung der transkribierten mRNA benötigt wird. Die Position und Richtung der Transkriptionsinitiation innerhalb jeder Transkriptionseinheit wird durch einen Pfeil angezeigt. Zur Klonierung der TE-Element ist vor dem Promotor ein Sequenzbereich "A" mit Schnittstellen für Restriktionsendonukleasen eingefügt (Adapter). Der Selektionsmarker Neomycin-Phosphotransferase enthält die Punktmutation F240I und ist in der Abbildung entsprechend mit "F2401" abgekürzt. Das aus dem Encephalomyocarditis-Virus stammende "IRES"-Element dient als interne Ribosomenbindungsstelle innerhalb der bicistronischen Transkriptionseinheit und ermöglicht die Translation des nachfolgenden roten fluoreszierenden Proteins "dsRed". "MCP-1" kodiert für das humane Monocyte Chemoattractant Protein-1.

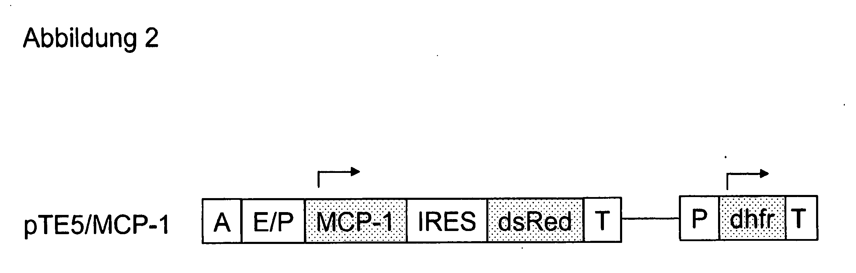

### ABBILDUNG 2: SCHEMATISCHE DARSTELLUNG EINES MCP-1 BASIS-VEKTORS

Der hier dargestellte Vektor wurde zur Expression des rekombinanten Proteins MCP-1 in CHO-DG44-Zellen verwendet. Bei "E/P" handelt es sich um eine Kombination aus CMV-Enhancer und CMV-Promotor, bei "P" lediglich um ein Promotorelement und bei "T" um ein Terminationssignal für die Transkription, das zur Polyadenylierung der transkribierten mRNA benötigt wird. Die Position und Richtung der Transkriptionsinitiation innerhalb jeder Transkriptionseinheit wird durch einen Pfeil angezeigt. Zur Klonierung der TE-Element ist vor dem Promotor ein Sequenzbereich "A" mit Schnittstellen für Restriktionsendonukleasen eingefügt (Adapter). Der Selektionsmarker Dihydrofolatreduktase ist mit "dhfr" abgekürzt. Das aus dem Encephalomyocarditis-Virus stammende "IRES"-Element dient als interne Ribosomenbindungsstelle innerhalb der bicistronischen Transkriptionseinheit und ermöglicht die Translation des nachfolgenden roten fluoreszierenden Proteins "dsRed". "MCP-1" kodiert für das humane Monocyte Chemoattractant Protein-1.

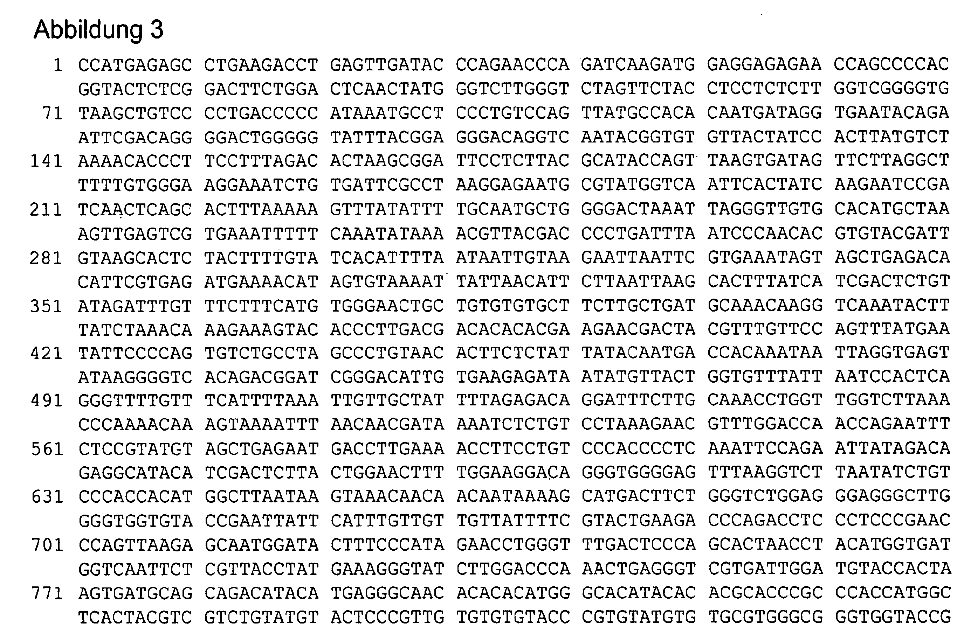

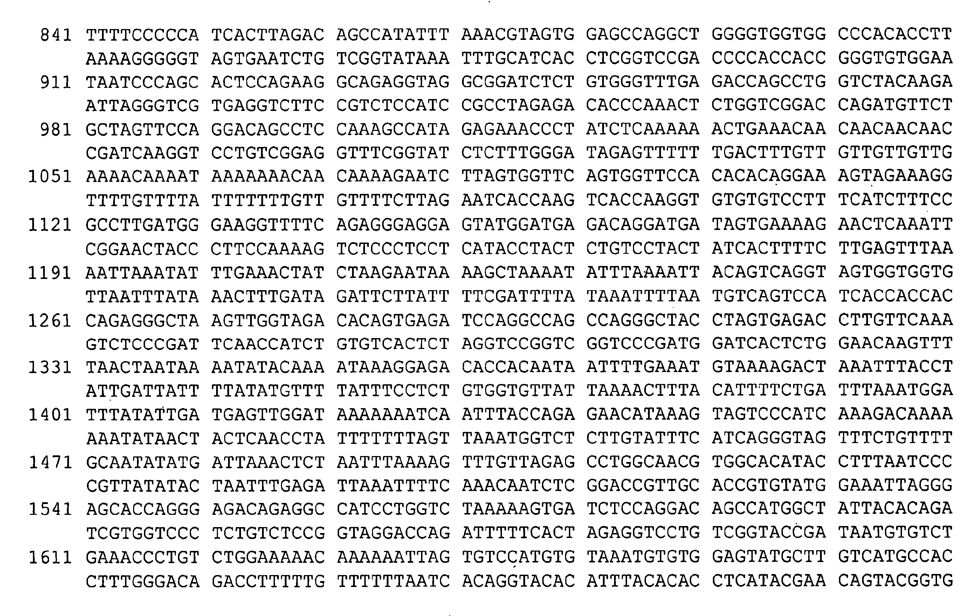

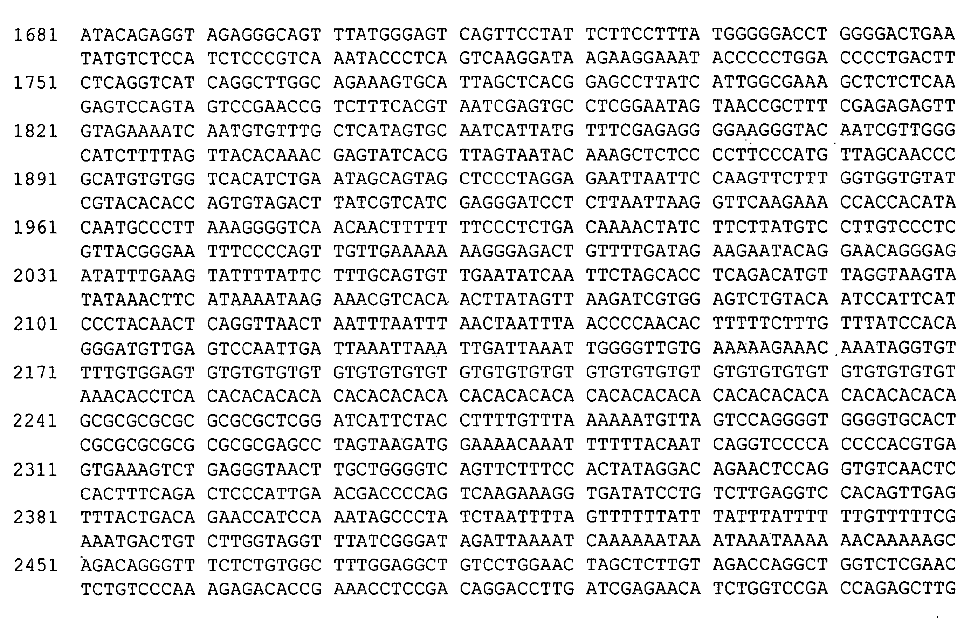

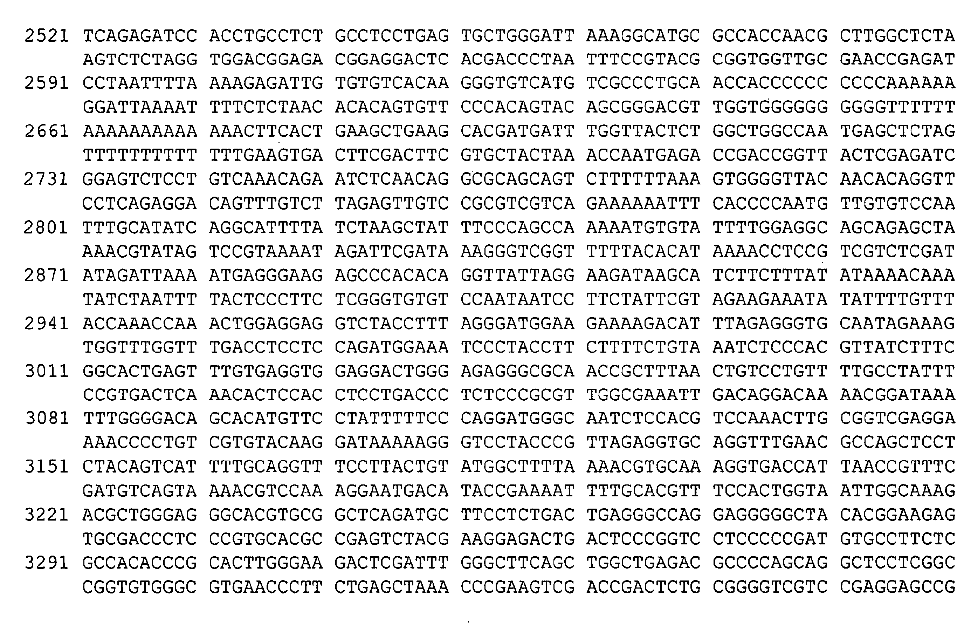

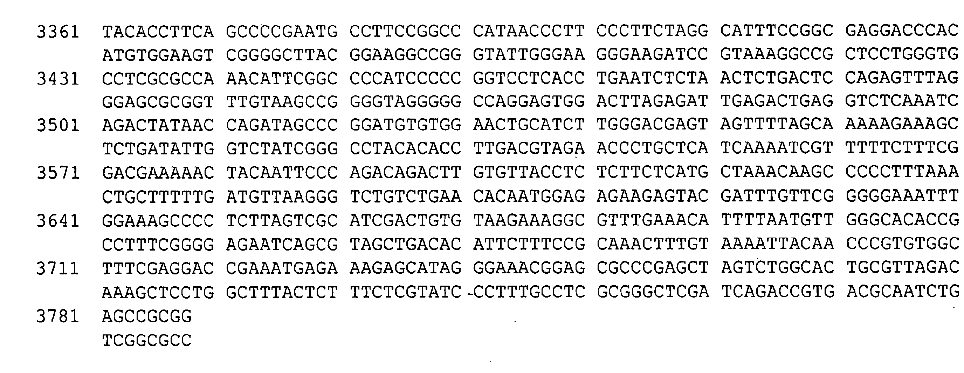

### ABBILDUNG 3: 5'-SEQUENZ DES CHO UBIQUITIN/S27A-GENS

Der 3788 bp umfasssende Sequenzbereich (SEQ ID Nr.1) wurde aus dem Genom von CHO- (*Chinese Hamster Ovary*) Zellen isoliert und liegt stromaufwärts von der Kodierregion des Ub/S27a-Gens, bei dem es sich um eine Fusion zwischen einer Ubiquitineinheit (Ub) und einem ribosomalen Protein der kleinen Ribosomenuntereinheit (S27a) handelt.

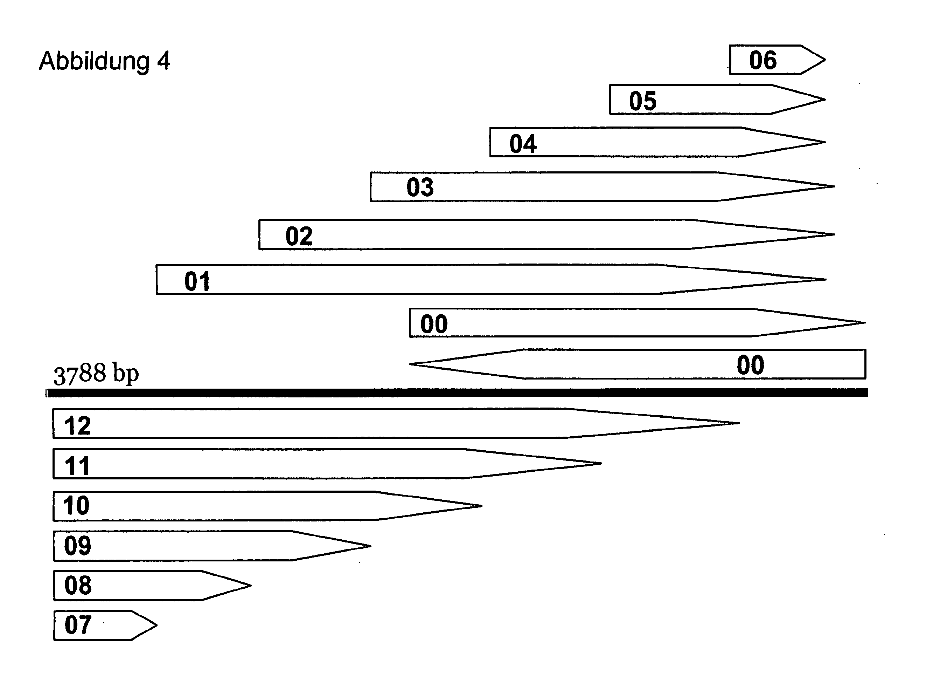

### ABBILDUNG 4: GRAPHISCHE DARSTELLUNG DER TE-ELEMENTE 00 BIS 12

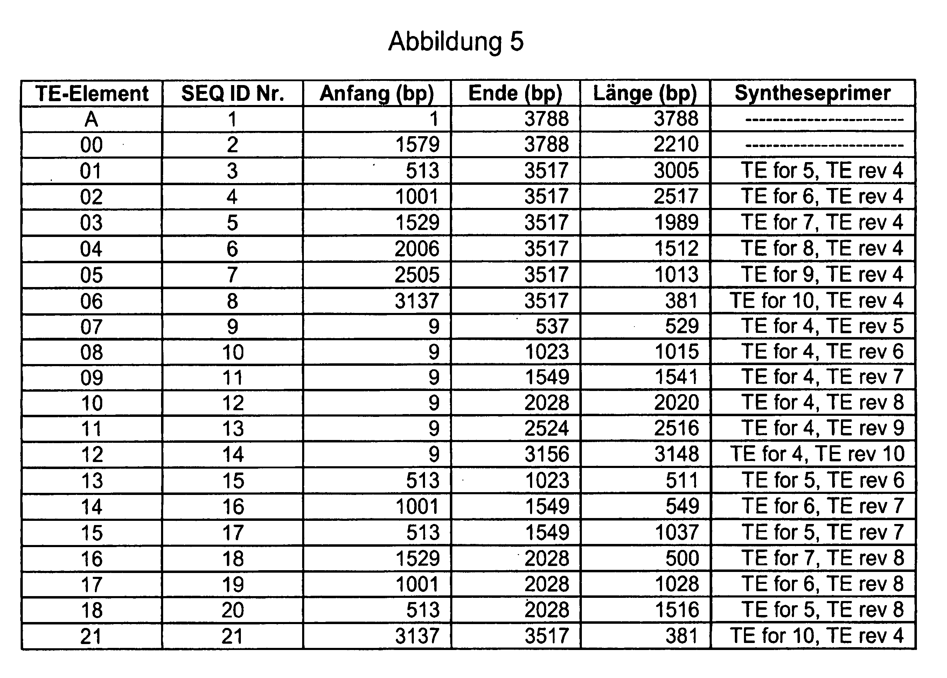

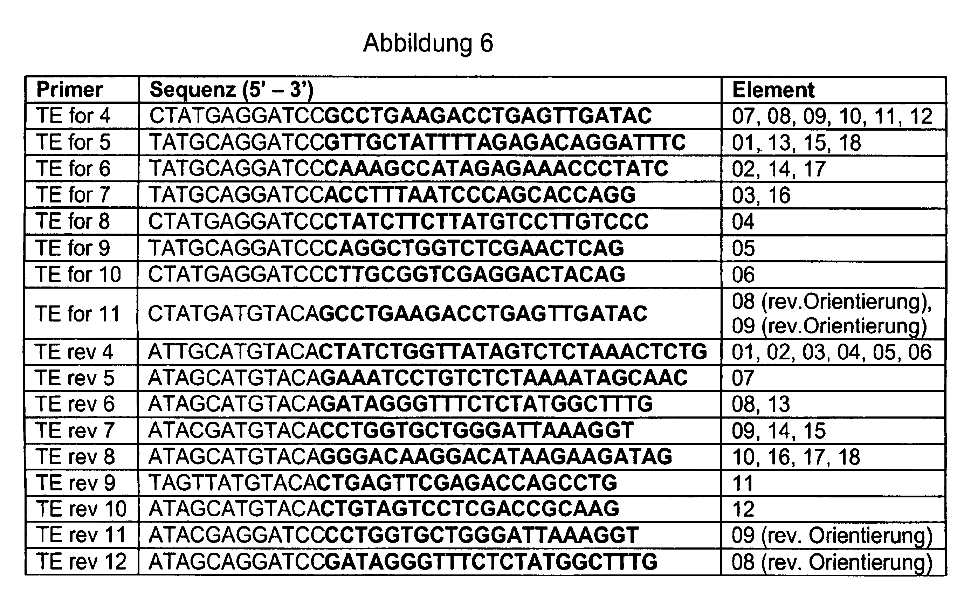

Diese Abbildung zeigt schematisch den stromaufwärts von der Kodierregion des CHO Ubiquitin/S27a-Gens gelegenen genomischen Sequenzbereich von 3788 bp, der subkloniert in einem Plasmid vorlag. Aus dieser auch als TE-Element A bezeichneten Genomsequenz (SEQ ID Nr. 1) wurden unterschiedlich lange Teilfragmente, im folgenden TE-Elemente genannt, hergestellt. Das TE-Element 00 (SEQ ID Nr. 2) wurde aus einem Subklon dieser Sequenz als SacII-Restriktionsfragment isoliert und in die SpeI-Schnittstelle der Zielvektoren pBID-HC und pBING-LC einkloniert. Diese enthielten entweder das Gen für die schwere (HC) oder die leichte Kette eines IgG1 (siehe Abbildung 1A). Dadurch entstanden Expressionsvektoren, in denen das TE-Element 00 in direkter und reverser Orientierung stromaufwärts des Promotors positioniert ist. Die TE-Elemente 01 bis 12 wurden durch PCR mit verschiedenen Primerpaaren hergestellt (siehe Abbildung 5 und 6) und über BamHI/BsrGI in die Basisplasmide pTE4/MCP-1 (Abb. 1B) und pTE5/MCP-1 (Abb. 2) kloniert.

### ABBILDUNG 5: TE-ELEMENTE 00 BIS 12

In dieser Tabelle sind die Größe sowie die Anfangs- und Endposition der TE-Elemente 00 bis 21 dargestellt, die aus der TE-A Sequenz (SEQ ID Nr. 1) erzeugt wurden. Für die Fragmente, die via PCR erzeugt wurden, sind zusätzlich noch die verwendeten Primer angegeben. Die Größenabstufungen der Elemente betragen ca. 500 bp und weisen im Vergleich zur Ausgangssequenz TE-A (SEQ ID Nr.1) Deletionen am 5'- oder 3'-Ende auf.

### ABBILDUNG 6: PRIMER FÜR DIE SYNTHESE DER TE-ELEMENTE 01 BIS 12

Die Primer sind in 5'-3'-Richtung dargestellt. Primer mit "for" in der Bezeichnung kennzeichnen Primer in direkter Orientierung der SEQ ID Nr. 1, Primer mit "rev" die in reverser Orientierung. Jeder Primer besteht am 5' Ende aus sechs beliebigen Nukleotiden, gefolgt von einer BamHI- oder BsrGI-Schnittstelle und einer Sequenz von ca. 20 bis 30 Nukleotiden, die 100 % homolog zu einem Sequenzabschnitt in SEQ ID Nr. 1 ist. Der zur SEQ ID Nr. 1 homologe Bereich der Primer wurde hervorgehoben. Je ein for- und ein rev-Primer wurden zur Amplifikation eines Sequenzbereichs der SEQ ID Nr.1 eingesetzt. Das jeweils resultierende PCR-Produkt wurde über BamHI und BsrGI in das Basisplasmid pTE4/MCP-1 (Abbildung 1B) oder pTE5/MCP-1 (Abbildung 2) einkloniert.

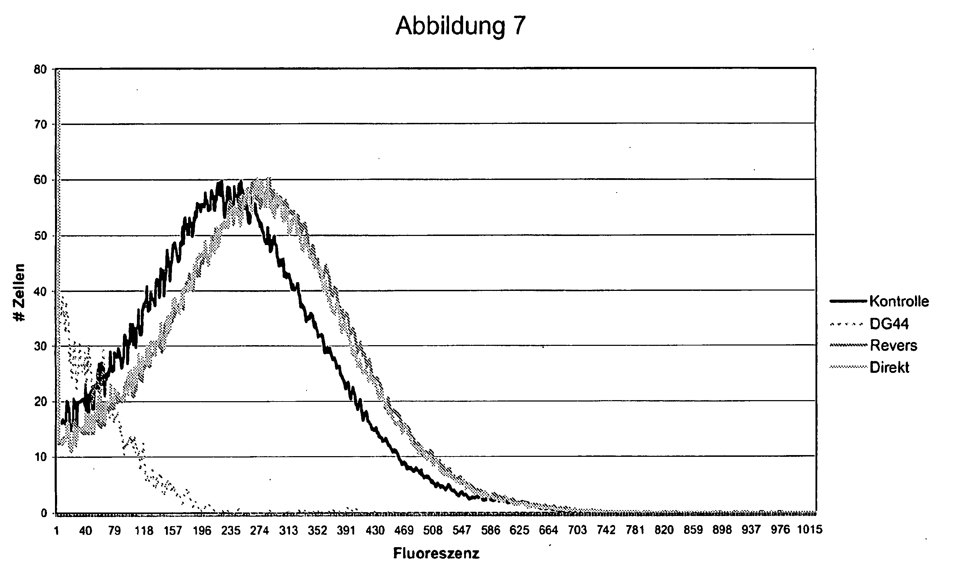

### ABBILDUNG 7: FACS-MESSUNG DER TRANSFEKTIONSSERIE B

Die Abbildung zeigt die relative Steigerung der GFP-Expression in Zellen mit dem TE-Element 00 im Vergleich zu Zellen ohne das TE-Element 00. Dazu wurden CHO-DG44-Zellen mit den Plasmidkombinationen pBING-LC und pBID-HC transfiziert, die sich lediglich im Vorhandensein und der orientierung des TE-Elements 00 voneinander unterschieden. Nach einer zwei- bis dreiwöchigen Selektion der transfizierten Zellpools in HT-freiem Medium mit Zusatz von G418 wurde die GFP-Fluoreszenz per FACS-Analyse gemessen. Jeder Graph, mit Ausnahme der als Negativkontrolle dienenden, nichttransfizierten CHO-DG44-Zellen ("DG44"), stellt den Mittelwert der GFP-Fluoreszenz aus jeweils zehn Pools der Transfektionsserie B dar. Pro Pool wurden 20000 Zellen berücksichtigt. "Kontrolle" steht für die Basisplasmide pBING-LC und pBID-HC, "Revers" bedeutet eine reverse Orientierung des TE-Elements 00 in den Basisvektoren, "Direkt" eine direkte Orientierung des TE-Elements 00 in den Basisvektoren.

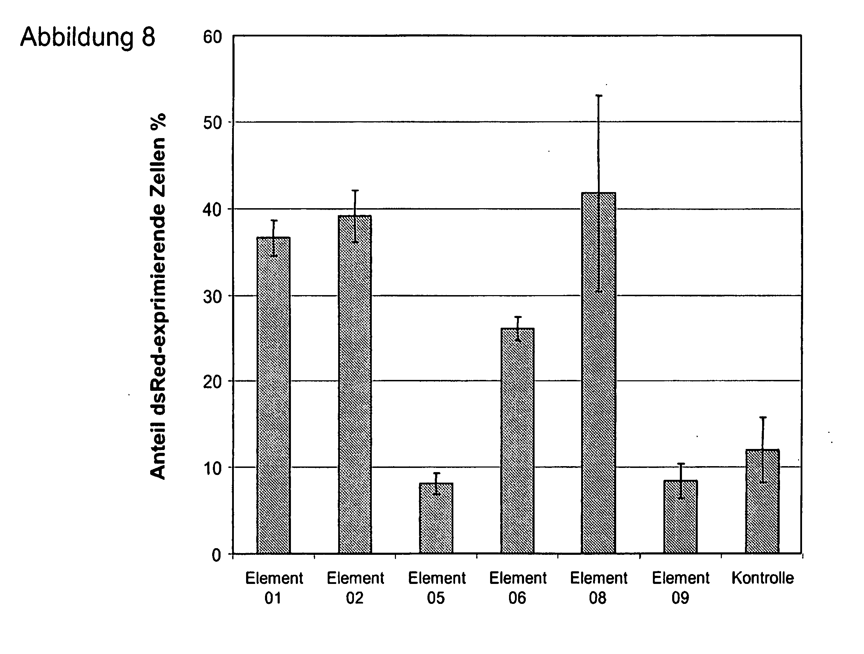

### ABBILDUNG 8: FACS-MESSUNG DER TRANSFEKTIONSSERIE C

Die Abbildung zeigt den Anteil von dsRed2-exprimierenden Zellen in stabilen Zellpopulationen, die die TE-Elemente 01, 02, 05, 06, 08 oder 09 enthielten, im Vergleich zu Zellen in Zellpopulationen, die kein TE-Element enthielten. Dazu wurden CHO-DG44-Zellen mit dem Plasmid pTE4/MCP-1 oder daraus abgeleiteten Derivaten, die zusätzlich jeweils eines der oben aufgeführten TE-Elemente enthielten, transfiziert. Nach einer ca. dreiwöchigen Selektion der transfizierten Zellpools in Medium mit Zusatz von G418 wurde die dsRed2-Fluoreszenz per FACS-Analyse gemessen. Es wurden pro Pool jeweils 10000 Zellen gemessen und die Eigenfluoreszenz der untransfizierten CHO-DG44 Zellen subtrahiert. Jeder Wert stellt den Mittelwert des prozentualen Anteils dsRed2-expimierender Zellen aus 6 Pools der Transfektionsserie C dar.

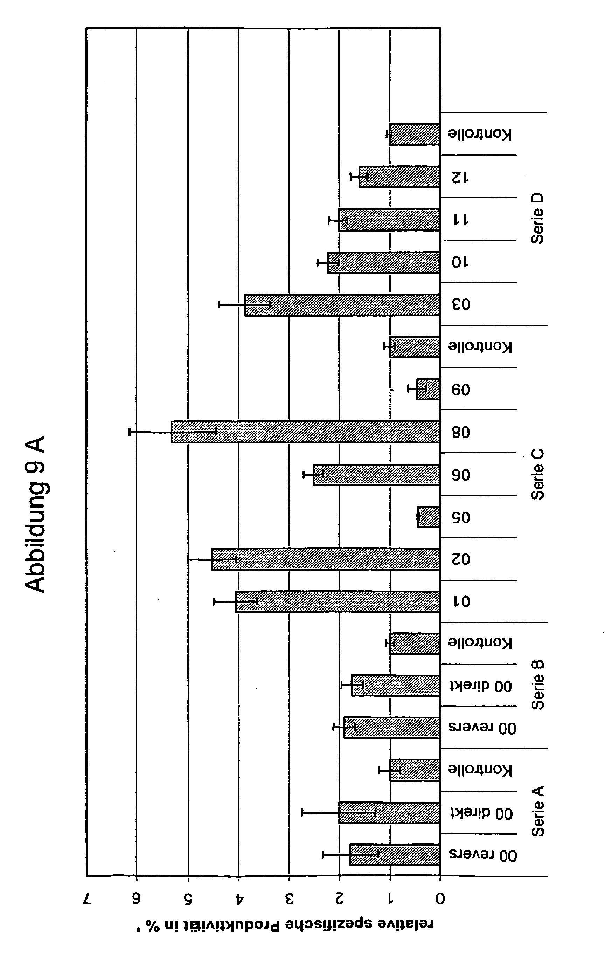

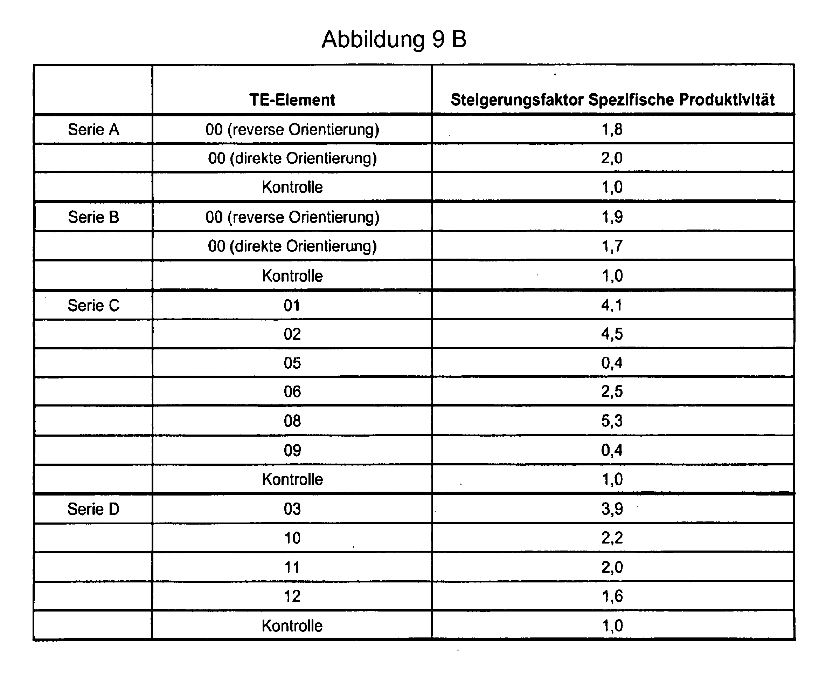

### ABBILDUNG 9: EINFLUSS DER TE-ELEMENTE AUF DIE SPEZIFISCHE PRODUKTIVITÄT

In dieser Abbildung sind die Änderungen der Expressionslevel von IgG1 bzw. MCP-1 dargestellt, die sich durch das Vorhandensein der TE-Elemente im Vergleich zu Kontrollpools ohne TE-Element ergeben, graphisch (A) bzw. tabellarisch (B) dargestellt. Die Zellpools wurden durch stabile Transfektion von CHO-DG44-Zellen mit den Basisplasmiden pBING-LC und pBID-HC bzw. pTE4/MCP-1 ("Kontrolle") und den daraus abgeleiteten Derivaten, die zusätzlich jeweils ein TE-Element enthielten ("00" in direkter ("00 direkt") und in reverser Orientierung ("00 revers"), "01" bis "12"), erzeugt. Nach einer zwei- bis dreiwöchigen Selektion der transfizierten Zellpools in HT-freiem Medium mit Zusatz von G418 (Serie A und B) bzw. in HT-haltigem Medium mit G418-Zusatz (Serie C und D) wurde die Proteinexpression per ELISA im Zellkulturüberstand gemessen und die spezifische Produktivität pro Zelle und Tag berechnet. Die Kultivierung der stabil transfizierten CHO-DG44-Zellen erfolgte über mehrere Passagen in 75 cm² T-Flasks mit einem Passagierungsrhythmus von 2-2-3 Tagen. In Serie A waren von den Plasmidkombinationen "00 reverse" und "00 direkt" je 4 Pools und von der Kontrolle 3 Pools über 8 Passagen in Kultur, in Serie B von jeder Plasmidkombination je 10 Pools über 6 Passagen und in den Serien C und D von jeder Plasmidsorte je 6 Pools über 6 Passagen. Die spezifischen Produktivitäten der Pools einer Plasmidkombination und Serie wurden gemittelt und der Mittelwert der Kontrollen in jeder Serie gleich 1 gesetzt. Die gemittelten spezifischen Produktivitäten der Pools mit TE-Element wurden dazu ins Verhältnis gesetzt.

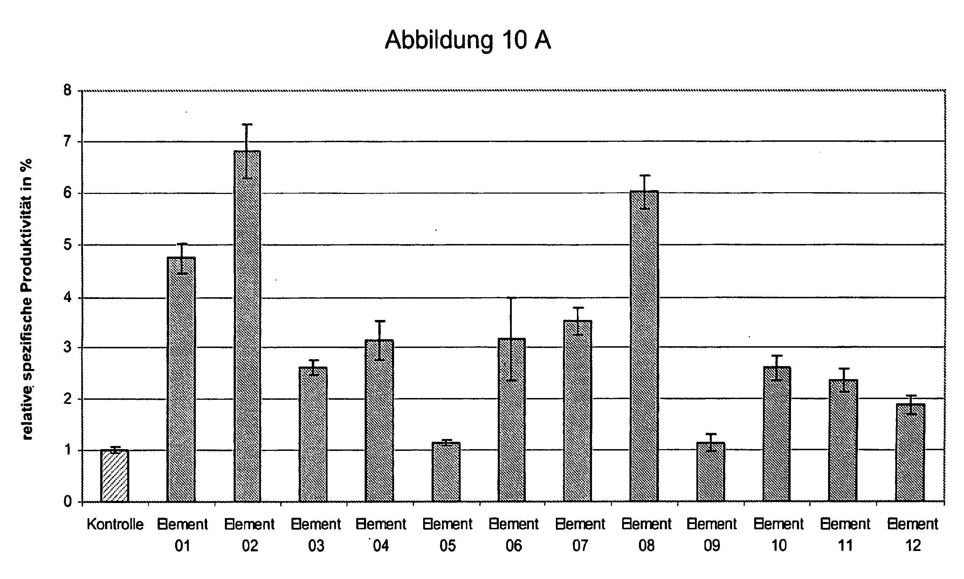

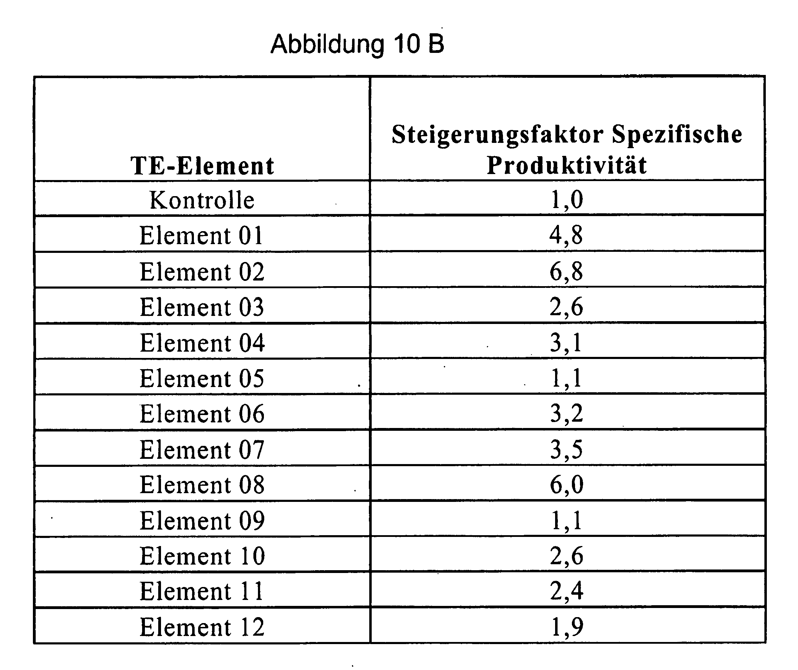

### ABBILDUNG 10: EINFLUSSS DER TE-ELEMENTE AUF DIE SPEZIFISCHE PRODUKTIVITÄT IN DHFR-SELEKTIONIERTEN ZELLPOOLS

In dieser Abbildung sind die Änderungen der Expressionslevel von MCP-1, die sich durch das Vorhandensein der TE-Elemente im Vergleich zu Kontrollpools ohne TE-Element ergeben, graphisch (A) bzw. tabellarisch (B) dargestellt. Die Zellpools wurden durch stabile Transfektion von CHO-DG44-Zellen mit dem Basisplasmid pTE5/MCP-1 ("Kontrolle") oder daraus abgeleiteten Derivaten, die zusätzlich jeweils ein TE-Elemente enthielten ("01" bis "12") (Serie E), erzeugt. Nach einer zwei- bis dreiwöchigen Selektion der transfizierten Zellpools in HT-freiem Medium wurde die Proteinexpression per ELISA im Zellkulturüberstand gemessen und die spezifische Produktivität pro Zelle und Tag berechnet. Die Kultivierung der stabil transfizierten CHO-DG44-Zellen erfolgte über mehrere Passagen in 75 cm² T-Flasks mit einem Passagierungsrhythmus von 2-2-3 Tagen. Von jeder Plasmidvariante waren je 6 Pools über 6 Passagen in Kultur. Die spezifischen Produktivitäten der Pools einer Plasmidvariante wurden gemittelt und der Mittelwert der Kontrollen gleich 1 gesetzt. Die gemittelten spezifischen Produktivitäten der Pools mit TE-Element wurden dazu ins Verhältnis gesetzt.

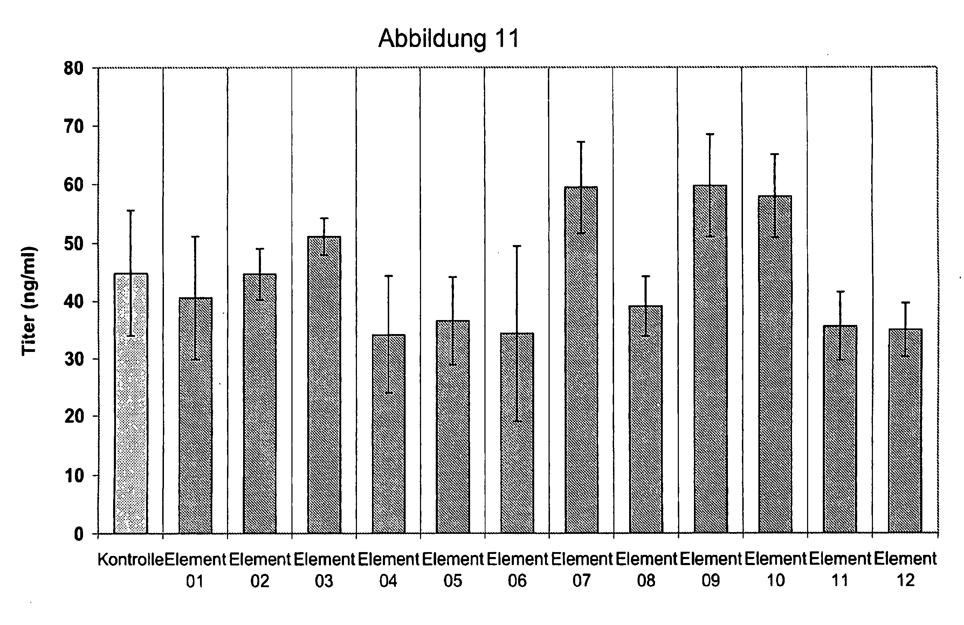

### ABBILDUNG 11: TEST DER TE-ELEMENTE AUF ENHANCERAKTIVITÄT

Die transiente Transfektion von CHO-DG44 Zellen zeigte bei Verwendung von Expressionsvektoren mit TE-Elementen keine signifikante Steigerung des MCP-1 Titers gegenüber Kontrollvektoren ohne TE-Element. Die TE-Elemente 01 bis 12 wirken somit nicht als Enhancer und können somit nur bei chromosomaler Integration eine signifikante Steigerung der Expression hervorrufen. Es wurden jeweils 6 Pools mit dem Basisvektor pTE4/MCP-1 ("Kontrolle") und den daraus abgeleiteten Derivaten, die zusätzlich jeweils ein TE-Elemente enthielten ("01" bis "12"), transfiziert. Gleichzeitig wurde ein SEAP-Expressionsplasmid co-transfiziert, um die Transfektionseffizienz zu bestimmen (SEAP = sezernierte alkalische Phosphatase). Nach 48 h Kultivierung in einem Gesamtvolumen von 3 ml wurde der Zellkulturüberstand abgenommen und der MGP-1-Titer mittels ELISA sowie die SEAP-Aktivität bestimmt. Der MCP-1-Titer wurde hinsichtlich der Transfektionseffizienz, ermittelt durch die SEAP-Expression, korrigiert. Die Abbildung zeigt den Mittelwert aus den jeweils 6 Parallelpools mit Standardabweichung.

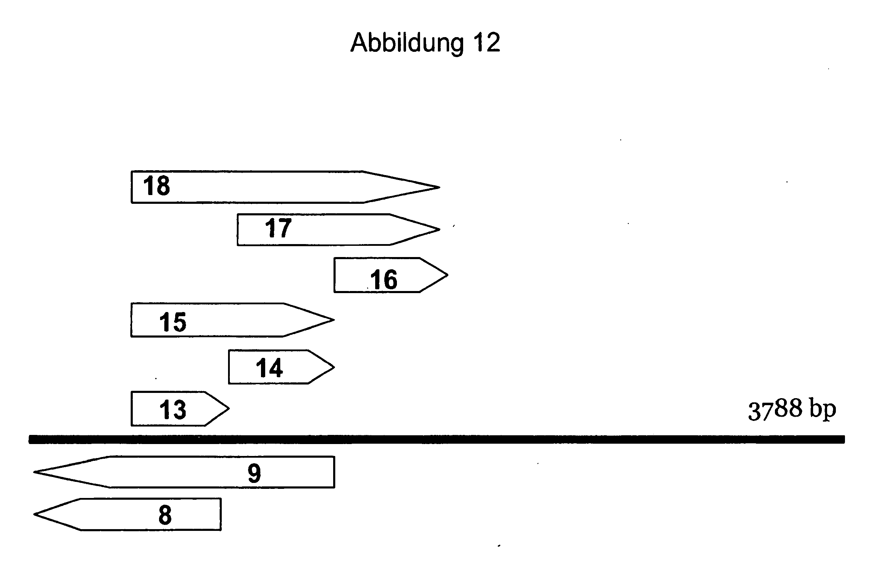

### ABBILDUNG 12: WEITERE TE-ELEMENTE

Die bisherigen Ergebnisse deuten darauf hin, dass die in dieser Abbildung gezeigte Auswahl von Teilstücken der Sequenz ID Nr. 1 ebenfalls eine Steigerung der Genexpression hervorrufen könnten. Durch Klonierung und stabile Transfektion dieser weiteren TE-Elemente soll die Sequenz ID Nr. 1 noch näher charakterisiert werden, um die für die Funktion wichtigen Sequenzbereiche exakter zu lokalisieren.

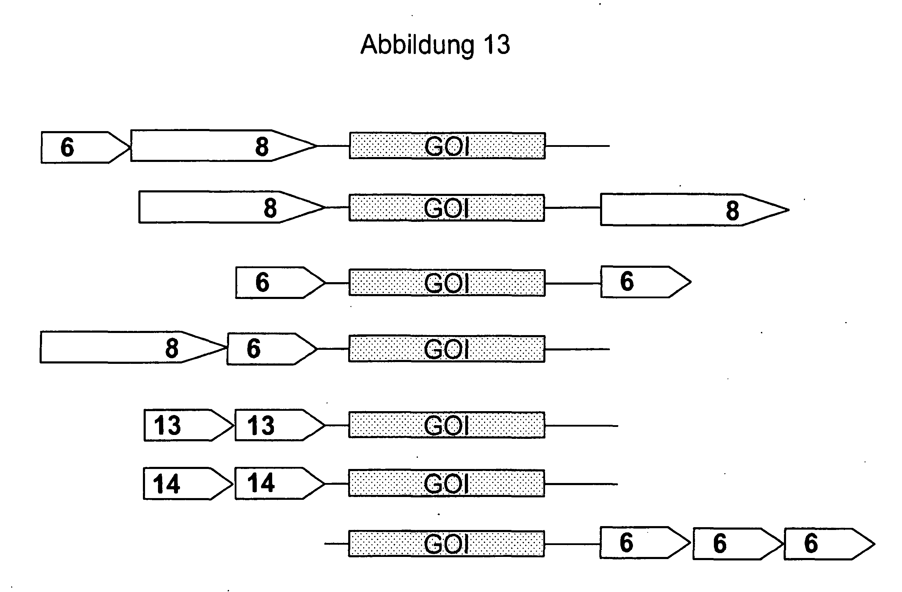

### ABBILDUNG 13: TESTUNG UNTERSCHIEDLICHER POSITIONEN UND KOMBINATIONEN DER TE-ELEMENTE

Diese Abbildung stellt eine Auswahl möglicher Expressionsvektoren dar, in denen unterschiedliche Positionen, Orientierung und Kombinationen von TE-Elementen verwendet werden, um zu untersuchen, ob dadurch eine zusätzliche Steigerung der Expression erreicht werden kann. Neben der Flankierung des Produktgens durch TE-Elemente werden auch mehrere identische oder verschiedene kurze TE-Elemente hintereinander geschaltet, wie z.B. die, TE-Elemente 06 und 08 oder die neuen TE-Elemente 13 und 14.

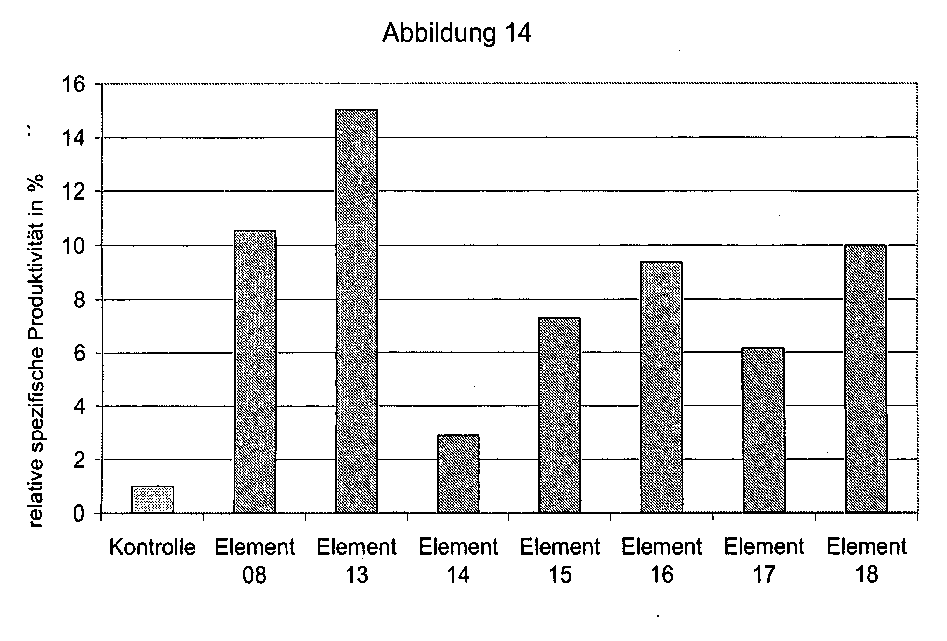

### ABBILDUNG 14: EINFLUSS DER TE-ELEMENTE TE-13 BIS TE-18 AUF DIE SPEZIFISCHE MCP-1-EXPRESSION

In dieser Abbildung sind die Änderungen der Expressionslevel von MCP-1, die sich durch das Vorhandensein der TE-Elemente im Vergleich zu Kontrollpools ohne TE-Element ergeben, graphisch dargestellt. Die Zellpools wurden durch stabile Transfektion von CHO-DG44-Zellen mit dem Basisplasmid pTE4/MCP-1 ("Kontrolle") oder daraus abgeleiteten Derivaten, die zusätzlich jeweils ein TE-Elemente enthielten ("13" bis "18") (Serie F), erzeugt. Nach einer zwei- bis dreiwöchigen Selektion der transfizierten Zellpools in HT-supplementiertem Medium +G418 (400 µg/ml) wurde die Proteinexpression per ELISA im Zellkulturüberstand gemessen und die spezifische Produktivität pro Zelle und Tag berechnet. Die Kultivierung der stabil transfizierten CHO-DG44-Zellen erfolgte über mehrere Passagen in 75 cm² T-Flasks mit einem Passagierungsrhythmus von 2-2-3 Tagen. Von jeder Plasmidvariante waren je 4 Pools über 5 bis 6 Passagen in Kultur. Die spezifischen Produktivitäten der Pools einer Plasmidvariante wurden gemittelt und der Mittelwert der Kontrollen gleich 1 gesetzt. Die gemittelten spezifischen Produktivitäten der Pools mit TE-Element wurden dazu ins Verhältnis gesetzt.

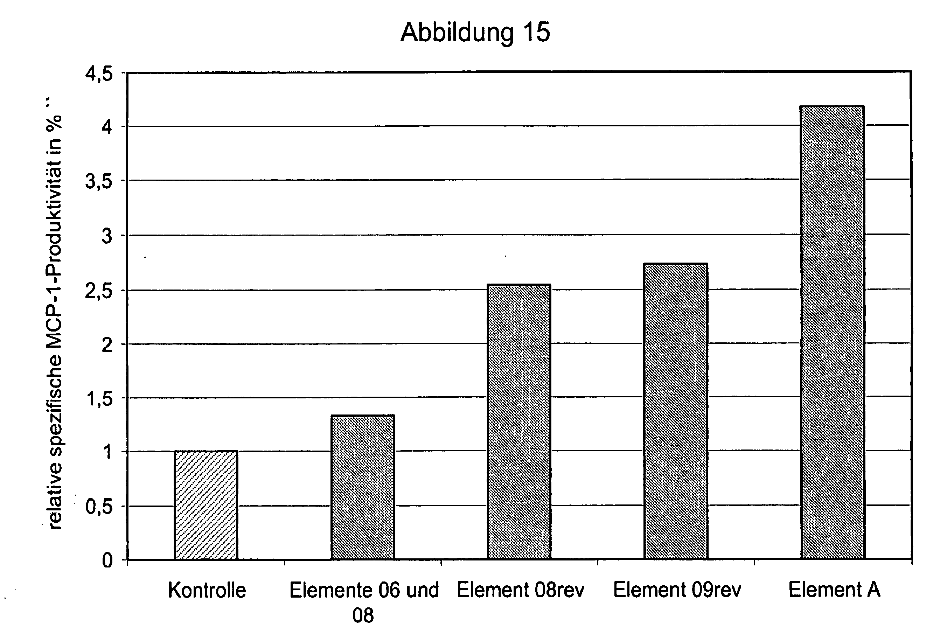

### ABBILDUNG 15: EINFLUSS DER TE-ELEMENTE AN VERSCHIEDENEN POSITIONEN UND IN VERSCHIEDENEN KOMBINATIONEN AUF DIE EXPRESSION VON MCP- 1

In dieser Abbildung sind die Änderungen der Expressionslevel von MCP-1, die sich durch das Vorhandensein und Kombination verschiedener TE-Elemente im Vergleich zu Kontrollpools ohne TE-Element ergeben, graphisch dargestellt. Die Zellpools wurden durch stabile Transfektion von CHO-DG44-Zellen mit dem Basisplasmid pTE4/MCP-1 ("Kontrolle") oder daraus abgeleiteten Derivaten, die zusätzlich jeweils ein oder zwei TE-Elemente enthielten ("06 und 08, 08rev, 09rev, A") (Serie G), erzeugt. Nach einer zweibis dreiwöchigen Selektion der transfizierten Zellpools in HT-supplementiertem Medium +G418 (300 µg/ml) wurde die Proteinexpression per ELISA im Zellkulturüberstand gemessen und die spezifische Produktivität pro Zelle und Tag berechnet. Die Kultivierung der stabil transfizierten CHO-DG44-Zellen erfolgte über mehrere Passagen in 6-Well Plates (MAT6) mit einem Passagierungsrhythmus von 2-2-3 Tagen. Von jeder Plasmidvariante waren je 6 Pools über 6 Passagen in Kultur. Die spezifischen Produktivitäten der Pools einer Plasmidvariante wurden gemittelt und der Mittelwert der Kontrollen gleich 1 gesetzt. Die gemittelten spezifischen Produktivitäten der Pools mit TE-Element wurden dazu ins Verhältnis gesetzt.

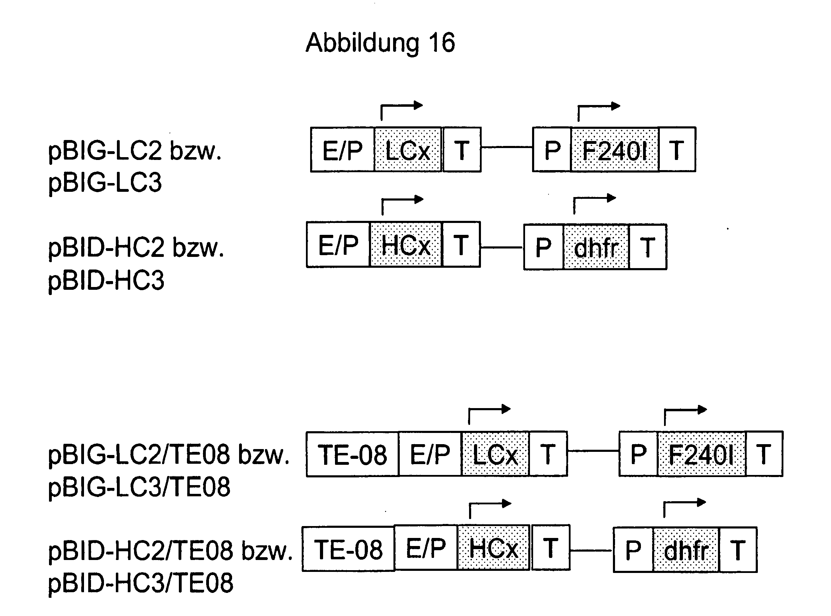

### ABBILDUNG 16: TESTUNG DES TE-ELEMENTS TE-08 MIT IGG4-ANTIKÖRPERN

Die hier dargestellten Vektoren wurden zur Expression von rekombinanten, monoklonalen IgG4 Antikörpern in CHO-DG44-Zellen verwendet. Bei "E/P" handelt es sich in diesem Falle um eine Kombination aus CMV-Enhancer und Promotor, bei "P" lediglich um ein Promotorelement und bei "T" um ein Terminationssignal für die Transkription, das zur Polyadenylierung der transkribierten mRNA benötigt wird. Die Position und Richtung der Transkriptionsinitiation innerhalb jeder Transkriptionseinheit wird durch einen Pfeil angezeigt. Die Gene für die leichte (LC2 bzw. LC3) und schwere (HC2 bzw. HC3) Kette wurden im Austausch gegen die MCP-1 - IRES - dsRed2-Kassette einkloniert (Abb. 1B und 2). Sie kodieren für die schwere bzw. leichte Kette humanisierter monoklonaler IgG4 Antikörper. Der amplifizierbare Selektionsmarker Dihydrofolat-Reduktase ist mit "dhfr" abgekürzt. Der Selektionsmarker Neomycin-Phosphotransferase enthält die Punktmutation F2401 und ist in der Abbildung entsprechend mit "F240I" abgekürzt.

### DETAILLIERTE BESCHREIBUNG DER ERFINDUNG

Im Rahmen dieser Erfindungsbeschreibung verwendete Begriffe und Bezeichnungen haben folgende im Anschluss definierte Bedeutungen. Die allgemeinen Ausführungsformen "enthaltend" oder "enthält" schließt die speziellere Ausführungsform "bestehend aus" mit ein. Femer werden "Einzahl" und "Mehrzahl" nicht begrenzend verwendet.

Der Begriff "TE-Element" bezeichnet regulatorische Nukleinsäuren.

Die Begriffe "TE-Element" oder "expressionssteigerndes Element" oder "transkriptionssteigerndes Element" oder "expressions- bzw. transkriptionssteigerndes Nukleinsäure Element" werden im Text synonym verwendet. Diese Begriffe bezeichnen alle regulatorische Nukleinsäuresequenzen.
Mit "TE-Element" oder "expressionssteigerndes Element" oder "transkriptionssteigerndes Element" oder "expressions- bzw. transkriptionssteigerndes Nukleinsäure Element" ist speziell die Sequenz ID Nr. 1, inklusive deren komplementärer Sequenz, die aus dem Genom des Chinesischen Hamsters (*Cricetulus griseus*) isoliert wurde, oder ein beliebiges Teilstück, Fragment oder Bereich davon oder ein Derivat der Sequenz ID Nr. 1 oder eines seiner Teilstücke, Fragmente oder Bereiche gemeint, welches bei stabiler chromosomaler Integration zu einer Steigerung der Transkription bzw. Expression eines Gens von Interesse führt, wobei das Fragment bzw. Derivat mindestens einen Sequenzbereich aus dem Nukleinsäurebereich zwischen 1 bp und 1578 bp mit umfasst und wobei das Derivat mindestens 85% Sequenzidentität aufweist. Ebenfalls gemeint sind beliebige Kombinationen aus erfindungsgemäßen Nukleinsäuren, die aus mehreren identischen oder unterschiedlichen erfindungsgemäßen Nukleinsäuren bestehen, die wiederum in beliebiger Orientierung und beliebigem Abstand zueinander angeordnet sein oder auch mit anderen regulatorischen Sequenzen kombiniert werden können und die zu einer Steigerung der Transkription bzw. Expression eines Gens von Interesse führen. Der Begriff TE-Element kann sowohl die SEQ ID Nr. 1 selbst als auch andere erfindungsgemäße Nukleinsäuren bezeichnen.
Des Weiteren umfasst der Begriff "TE-Element", "transkriptionssteigerndes bzw. expressionssteigerndes Nukleinsäure Element" bzw. Fragmente, Teilstücke, Bereiche oder Derivate davon neben Sequenzbereichen aus dem Chinesischen Hamster (*Cricetulus griseus*) auch entsprechende funktionelle homologe Nukleotidsequenzen aus anderen Organismen. Solche anderen Organismen sind beispielsweise Mensch, Maus, Ratte, Affe, sowie andere Säuge- und Nagetiere, Reptilien, Vögel, Fische und Pflanzen.

Mit einem "Fragment" oder "Teilstück" oder "Bereich" (Begriffe werden synonym verwendet) ist ein Nukleinsäuremolekül (Einzel- oder Doppelstrang) gemeint, welches mit einem Teilbereich von SEQ ID Nr. 1 oder deren komplementärer Sequenz 100% Sequenzidentität aufweist. Es ist bekannt, dass die Klonierung von Fragmenten, die entweder über Verdau mit Restriktionsenzymen oder via PCR erzeugt werden, zu Modifikationen in den Endbereichen des Fragmentes führen kann, also beispielsweise aus Auffüll- oder Abbaureaktionen resultierende zusätzliche oder fehlende Nukleotide oder über Primer zusätzlich eingeführte Nukleotide. Diese Variationen in den Endbereichen der Fragmente sind in der Definition eines Fragments eingeschlossen, selbst wenn diese Sequenzbereiche eine Sequenzidentität von weniger als 100% zur SEQ ID Nr. 1 aufweisen. "Teilstücke" bzw. "Fragmente" bzw. "Bereiche" der Sequenz ID Nr. 1 sind z.B. TE-00 (Sequenz ID Nr. 2), TE-01 (Sequenz ID Nr. 3), TE-02 (Sequenz ID Nr. 4), TE-03 (Sequenz ID Nr. 5), TE-04 (Sequenz ID Nr. 6), TE-05 (Sequenz ID Nr. 7), TE-06 (Sequenz ID Nr. 8), TE-07 (Sequenz ID Nr. 9), TE-08 (Sequenz ID Nr. 10), TE-09 (Sequenz ID Nr. 11), TE-10 (Sequenz ID Nr. 12), TE-11 (Sequenz ID Nr. 13), TE-12 (Sequenz ID Nr. 14), TE-13 (Sequenz ID Nr. 15), TE-14 (Sequenz ID Nr. 16), TE-15 (Sequenz ID Nr. 17), TE-16 (Sequenz ID Nr. 18), TE-17 (Sequenz ID Nr. 19), TE-18 (Sequenz ID Nr. 20), TE-21 (SEQ ID No. 21). Bevorzugt führt das Fragment bei stabiler chromosomaler Integration zu einer Steigerung der Transkription bzw. Expression eines funktionell verknüpften Gens von Interesse. Bevorzugte Teilstücke" bzw. "Fragmente" bzw. "Bereiche" der Sequenz ID Nr. 1, die zu einer Steigerung der Transkription bzw. Expression eines Gens von Interesse führen, sind z.B. TE-01 (Sequenz ID Nr. 3), TE-02 (Sequenz ID Nr. 4), TE-07 (Sequenz ID Nr. 9), TE-08 (Sequenz ID Nr. 10), TE-10 (Sequenz ID Nr. 12), TE-11 (Sequenz ID Nr. 13), TE-12 (Sequenz ID Nr. 14), TE-13 (SEQ ID Nr. 15), TE-15 (SEQ ID Nr. 17), TE-17 (SEQ ID Nr. 19), TE-18 (SEQ ID Nr. 20). Aber mit "Fragment" sind auch alle möglichen anderen Teilstücke der SEQ ID Nr. 1 in beliebiger Orientierung gemeint, welche zu einer Steigerung der Transkription bzw. Expression eines Gens von Interesse führen, insbesondere solche, die ganz oder zumindestens teilweise im Bereich 5' von TE-00 (SEQ ID Nr. 2) liegen. Dies entspricht dem Teilbereich von SEQ ID Nr. 1 zwischen 1 bp und 1578bp. Weiterhin bevorzugt ist das Fragment TE-08 (SEQ ID Nr. 10).

Unter einem "Derivat" wird in der vorliegenden Erfindung ein Nukleinsäuremolekül (Einzel- oder Doppelstrang) verstanden, welches mit SEQ ID Nr. 1 oder deren komplementärer Sequenz oder mit einem Teilstück bzw. Fragment bzw. Bereich der SEQ ID Nr. 1 bzw. dessen komplementärer Sequenz mindestens ca. 85% Sequenzidentität, bevorzugt mindestens ca. 90% Sequenzidentität und besonders bevorzug mindestens ca. 95% Sequenzidentität aufweist und welches bei chromosomaler Integration zu einer Steigerung der Transkription bzw. Expression eines Gens von Interesse führt. Sequenzunterschiede zur SEQ ID Nr. 1 können zum einen auf Unterschiede in homologen, endogenen Nukleotidsequenzen aus anderen Organismen beruhen. Zum anderen können sie auch auf gezielten Modifikationen der Nukleotidsäuresequenz basieren, z.B. auf Substitution, Insertion oder Deletion zumindest einer oder mehrerer Nukleotide. Deletions-, Insertions- und Substitutionsmutanten lassen sich mittels "ortspezifischer Mutagenese" und/oder "PCR-basierten Mutagenese-Techniken" erzeugen. Entsprechende Methoden sind beispielhaft von Lottspeich und Zorbas (1998; Kapitel 36.1 mit weiteren Verweisen) beschrieben. Die Sequenzidentität kann beispielsweise mit Hilfe sogenannter Standard "alignment" Algorithmen, wie beispielsweise "BLAST" (Altschul, S.F., Gish, W., Miller, W., Myers, E.W. & Lipman, D.J. (1990) "Basic local alignment search tool." J. Mol. Biol. 215:403-410; Madden, T.L., Tatusov, R.L. & Zhang, J. (1996) "Applications of network BLAST server" Meth. Enzymol. 266:131-141; Zhang, J. & Madden, T.L. (1997) "PowerBLAST: A new network BLAST application for interactive or automated sequence analysis and annotation." Genome Res. 7:649-656) mit einer Referenzsequenz, vorliegend mit der Sequenz ID Nr. 1, in Übereinstimmung gebracht werden kann. Sequenzen sind dann in Übereinstimmung gebracht, wenn sie sich in ihrer Sequenzabfolge entsprechen und mit Hilfe der Standard "alignment" Algorithmen identifizieren lassen.

Der Ausdruck "Variante" bezieht sich auf die im jeweiligen Transfektionsansatz eingesetzten Expressionsvektoren. Darunter fallen sowohl die Basisvektoren (pTE4/MCP-1 bzw. pTE5/MCP-1) bzw. die Basisvektorkombination (pBING-LC + pBID-HC) als auch die Basisvektoren, die jeweils ein oder mehrere TE-Elemente in unterschiedlicher Positionierung, Kombination und Orientierung enthalten.

Bei Primern bezieht sich der Ausdruck "Orientierung" auf die Anordnung der Primer im Verhältnis zur SEQ ID Nr. 1. Alle Primer, deren Sequenzabfolge der in der unter der SEQ ID Nr.1 geführten Sequenz in 5'-3'-Abfolge entspricht (= "forward primer"), befinden sich in gleicher Orientierung wie diese Sequenz, was auch als "direkte Orientierung" bezeichnet wird. Primer, deren Sequenzabfolge komplementär zu der unter der SEQ ID Nr.1 geführten Sequenz ist (= "reverse primer"), befinden sich entgegensetzter Orientierung zu dieser Sequenz, was auch als "reverse Orientierung" bezeichnet wird.
Im Zusammenhang mit TE-Elementen wird in der vorliegenden Erfindung unter "Orientierung" die Anordnung in Bezug auf das Gen von Interesse verstanden. Die unter der SEQ ID Nr. 1 geführte Sequenz repräsentiert eine Genomsequenz, die 5' von der für das Ubiquitin/S27a-Gen kodierenden Region positioniert ist, was auch als "stromaufwärts" bezeichnet wird. Die Fortführung dieser in der SEQ ID Nr.1 angegebenen Sequenz in Richtung Kodierregion des nachfolgenden Ubiquitin/S27a-Gens würde zum Startcodon dieses Gens führen. Diese Anordnung wird deshalb als "direkte Orientierung" bezeichnet. Analog liegt in der vorliegenden Erfindung das TE-Element in direkter Orientierung vor, wenn die unter der SEQ ID Nr.1 angegebene Sequenz, oder ein beliebiges Teilstück, Fragment, Bereich oder Derivat davon, im Expressionsvektor auf dem gleichen DNA-Strang wie das Start-Codon des Gens von Interesse vorliegt. Liegt hingegen die zur SEQ ID Nr.1 komplementäre Sequenz, oder ein beliebiges Teilstück, Fragment, Bereich oder Derivat davon, im Expressionsvektor auf dem gleichen DNA-Strang wie das Start-Codon des Gens von Interesse, dann liegt eine "reverse Orientierung" des jeweiligen TE-Elements vor. Ist nichts weiter angegeben, so sind bei der Nennung eines TE-Elementes immer beide Orientierungen umfasst / gemeint, also direkt und reverse.

Unter "chromosomaler Integration" wird die Integration einer beliebigen Nukleinsäuresequenz in das Genom, also in die Chromosomen, einer Zelle verstanden, wobei die Integration in ein oder mehrere Chromosomen in beliebiger Zahl, Position und Orientierung erfolgen kann. Des weiteren wird unter "chromosomaler Integration" auch die Integration einer beliebigen Nukleinsäuresequenz in künstliche, artifizielle oder Mini-Chromosomen verstanden.

### Gen von Interesse:

Das im erfindungsgemäßen Expressionsvektor enthaltene "Gen von Interesse" umfasst eine Nukleotidsequenz beliebiger Länge, die für ein Produkt von Interesse kodiert. Das Genprodukt oder auch "Produkt von Interesse" ist in der Regel ein Protein, Polypeptid, Peptid bzw. Fragment oder Derivat davon. Es kann aber auch RNA oder antisense RNA sein. Das Gen von Interesse kann in voller Länge, in verkürzter Form, als Fusionsgen oder markiertes Gen vorliegen. Es kann sich um genomische DNA oder vorzugsweise cDNA bzw. entsprechende Fragmente oder Fusionen handeln. Das Gen von Interesse kann die native Gensequenz darstellen, mutiert oder auf sonstige Weise modifiziert sein. Derartige Modifikationen schließen Codon-Optimierungen zur Anpassung an eine bestimmte Wirtszelle und eine Humanisierung ein. Das Gen von Interesse kann z.B. für ein sekretiertes, zytoplasmatisches, kernlokalisiertes, membrangebundenes oder zelloberflächengebundenes Polypeptid kodieren.

Der Ausdruck "Nukleinsäure", "Nukleotidsequenz" oder "Nukleinsäuresequenz" bezeichnet ein Oligonukleotid, Nukleotide, Polynukleotide und deren Fragmente sowie DNA oder RNA genomischen oder synthetischen Ursprungs, die als Einzel- oder Doppelstrang vorliegen und den kodierenden oder den nicht-kodierenden Strang eines Gens repräsentieren kann. Zur Modifikation von Nukleinsäuresequenzen können Standardtechniken, wie z.B. ortsspezifische Mutagenese oder PCR-vermittelte Mutagenese (z.B. in Sambrook et al., 1989 oder Ausubel et al., 1994 beschrieben), eingesetzt werden.

Unter "kodieren" versteht man die Eigenschaft oder Fähigkeit einer spezifischen Sequenz von Nukleotiden in einer Nukleinsäure, beispielsweise einem Gen in einem Chromosom oder einer mRNA, als Matrize für die Synthese von anderen Polymeren und Makromolekülen wie z.B. rRNA, tRNA, mRNA, anderen RNA-Molekülen, cDNA oder Polypeptiden in einem biologischen Prozess zu dienen. Demnach kodiert ein Gen für ein Protein, wenn durch Transkription und nachfolgende Translation der mRNA das gewünschte Protein in einer Zelle oder einem anderen biologischen System produziert wird. Sowohl der kodierende Strang, dessen Nukleotidsequenz identisch mit der mRNA-Sequenz ist und normalerweise auch in Sequenzdatenbanken, z.B. EMBL oder GenBank, angegeben wird, als auch der als Matrize für die Transkription dienende nicht-kodierende Strang eines Gens oder cDNA kann dabei als kodierend für ein Produkt oder Protein bezeichnet werden. Eine Nukleinsäure, die für ein Protein kodiert, schließt auch Nukleinsäuren mit ein, die auf Grund des degenerierten genetischen Codes eine andere Nukleotidsequenzabfolge aufweisen, aber in der gleichen Aminosäuresequenz des Proteins resultieren. Nukleinsäuresequenzen, die für Proteine kodieren, können auch Introns enthalten.

Mit dem Ausdruck "cDNA" werden Desoxyribonukleinsäuren bezeichnet, die durch reverse Transkription und Synthese des zweiten DNA-Strangs aus einer von einem Gen produzierten mRNA oder anderen RNA hergestellt werden. Liegt die cDNA als doppelstränges DNA-Molekül vor, dann enthält sie sowohl einen kodierenden als auch einen nicht-kodierenden Strang.

Mit dem Ausdruck "Intron" werden nicht-kodierende Nukleotidsequenzen beliebiger Länge bezeichnet. Sie kommen natürlicherweise in vielen eukaryontischen Genen vor und werden von einem zuvor transkribierten mRNA-Vorläufer durch einen als Spleissen bezeichneten Prozess entfernt. Hierfür ist ein exaktes Herausschneiden des Introns am 5'-und 3'-Ende und eine korrekte Verbindung der entstehenden mRNA-Enden erforderlich, damit eine reife prozessierte mRNA mit dem für die erfolgreiche Proteinsynthese richtigen Leseraster hergestellt wird. Viele der an diesem Spleissing-Prozess beteiligten Spleiss-Donor- und Spleiss-Akzeptor-Stellen, das sind die unmittelbar an den Exon-Intron- bzw. Intron-Exon-Grenzen vorliegenden Sequenzen, sind mittlerweile charakterisiert worden. Für einen Überblick siehe Ohshima et al., 1987.

### Protein/Produkt von Interesse:

Biopharmazeutisch bedeutsame Proteine/Polypeptide umfassen z.B. Antikörper, Enzyme, Cytokine, Lymphokine, Adhäsionsmoleküle, Rezeptoren sowie deren Derivate bzw. Fragmente, sind aber nicht auf diese beschränkt. Im Allgemeinen sind alle Polypeptide bedeutsam, die als Agonisten oder Antagonisten wirken und/oder therapeutische oder diagnostische Anwendung finden können. Andere Proteine von Interesse sind beispielsweise Proteine/Polypeptide, die zur Veränderung der Eigenschaften von Wirtszellen im Rahmen des sogenannten "Cell Engineering" verwendet werden, wie z.B. anti-apoptotische Proteine, Chaperone, Stoffwechselenzyme, Glykosylierungsenzyme sowie deren Derivate bzw. Fragmente, sind aber nicht auf diese beschränkt.

Der Ausdruck "Polypeptide" wird für Aminosäuresequenzen oder Proteine verwendet und bezeichnet Polymere von Aminosäuren beliebiger Länge. Dieser Ausdruck schließt auch Proteine ein, die posttranslational durch Reaktionen wie beispielsweise Glykosylierung, Phosphorylierung, Acetylierung oder Proteinprozessierung modifiziert werden. Die Struktur des Polypeptids kann z.B. durch Substitutionen, Deletionen oder Insertion von Aminosäuren. Fusion mit anderen Proteinen, unter Beibehaltung seiner biologischen Aktivität modifiziert werden. Zudem können die Polypeptide multimerisieren und Homo- und Heteromere bilden.

Beispiele für therapeutische Proteine sind Insulin, Insulin-ähnlicher Wachstumsfaktor, humanes Wachstumshormon (hGH) und andere Wachstumsfaktoren, Rezeptoren, Gewebeplasminogenaktivator (tPA), Erythropoetin (EPO), Cytokine, beispielsweise Interleukine (IL) wie IL-1, IL-2, IL-3, IL-4, IL-5, IL-6, IL-7, IL-8, IL-9, IL-10, IL-1 1, IL-12, IL-13, IL-14, 1L-15, IL-16, IL-17, IL-18, Interferon (IFN)-alpha, beta, gamma, omega oder tau, Tumornekrosefaktor (TNF) wie z.B. TNF-alpha, beta oder gamma, TRAIL, G-CSF, GM-CSF, M-CSF, MCP-1 und VEGF. Weitere Beispiele sind monoklonale, polyklonale, multispezifische und einzelkettige (*single chain*) Antikörper und Fragmente davon, wie z.B. Fab, Fab', F(ab')₂, Fc und Fc'-Fragmente, leichte. (L) und schwere (H) Immunglobulinketten und deren konstante, variable oder hypervariable Regionen sowie Fv- und Fd-Fragmente (Chamov et al., 1999). Die Antikörper können humanen oder nichthumanen Ursprungs sein. Auch humanisierte und chimäre Antikörper kommen in Frage.

Fab-Fragmente (Fragment antigen-binding = Fab) bestehen aus den variablen Regionen beider Ketten, die durch die angrenzenden konstanten Regionen zusammengehalten werden. Sie können z.B. durch Behandlung mit einer Protease, wie beispielsweise Papain, aus konventionellen Antikörpern erzeugt werden oder aber auch durch DNA-Klonierung. Weitere Antikörperfragmente sind F(ab')₂-Fragmente, die durch proteolytischen Verdau mit Pepsin hergestellt werden können.

Durch Genklonierung können auch verkürzte Antikörperfragmente hergestellt werden, die nur aus den variablen Region der schweren (VH) und der leichten Kette (VL) bestehen. Diese werden als Fv-Fragmente (Fragment variable = Fragment des variablen Teils) bezeichnet. Da bei diesen Fv-Fragmenten die kovalente Verbindung über die Cysteinreste der konstanten Ketten nicht möglich ist, werden diese Fv-Fragmente oft anderweitig stabilisiert. Dazu werden die variablen Region der schweren und leichten Kette häufig mittels eines kurzen Peptidfragments von ca. 10 - 30 Aminosäuren, besonders bevorzugt 15 Aminosäuren, miteinander verknüpft. Auf diese Weise entsteht eine einzelne Polypeptidkette, in der VH und VL durch einen Peptidlinker miteinander verbunden sind. Solche Antikörperfragmente werden auch als *single-chain* Fv-Fragment (scFv) bezeichnet. Beispiele von scFv-Antikörpern sind bekannt und beschrieben, siehe z.B. Huston et al. (1988).

In den vergangenen Jahren wurden verschiedene Strategien entwickelt um multimere scFv-Derivate herzustellen. Die Intention besteht in der Erzeugung von rekombinanten Antikörpern mit verbesserten pharmakokinetischen Eigenschaften und verstärkter Bindungsavidität. Zur Erreichung der Multimerisierung der scFv-Fragmente werden diese als Fusionsproteine mit Multimerisierungsdomänen hergestellt. Als Multimerisierungsdomänen können dabei z.B. die CH3-Region eines IgGs oder Helixstrukturen ("*coiled coil structure*") wie die Leucin-Zipper-Domänen fungieren. In anderen Strategien wird die Interaktion zwischen den VH- und VL-Regionen des scFv-Fragments für eine Multimerisierung genutzt (z.B. Dia-, Tri- und Pentabodies).

Als "Diabody" bezeichnet ein Fachmann ein bivalentes homodimeres scFv-Derivat. Die Verkürzung des Peptidlinkers im scFv-Moleküle auf 5 - 10 Aminosäuren resultiert in der Bildung von Homodimeren durch Überlagerung von VH/VL-Ketten. Die Diabodies können zusätzlich durch eingeführte Disulfidbrücke stabilisiert werden. Beispiele von Diabodies finden sich in der Literatur, z.B. bei Perisic et al. (1994).

Als "Minibody" bezeichnet der Fachmann ein bivalentes, homodimeres scFv-Derivat. Es besteht aus einem Fusionsprotein, das die CH3-Region eines Immunglobulins, vorzugsweise IgG, besonders bevorzugt IgGI, als Dimerisierungsregion enthält. Diese verbindet die scFv-Fragmente über eine Hinge-Region, ebenfalls von IgG, und eine Linker-Region. Beispiele solcher Minibodies sind bei Hu et al. (1996) beschrieben.

Mit "Triabody" bezeichnet der Fachmann ein trivalentes homotrimeres scFv-Derivat (Kortt et al., 1997). Die direkte Fusion von VH-VL ohne Verwendung einer Linkersequenz führt zur Ausbildung von Trimeren.

Bei den vom Fachmann als Mini-Antikörper bezeichneten Fragmenten, die eine bi-, tri- oder tetravalente Struktur haben, handelt es sich ebenfalls um Derivate von scFv-Fragmenten. Die Multimerisierung wird dabei über di-, tri- oder tetramere "coited coil"-Strukturen erzielt (Pack et al., 1993 und 1995; Lovejoy et al., 1993).

### Gen, das für ein fluoreszierendes Protein kodiert:

In einer weiteren Ausführungsform enthält der erfindungsgemäße Expressionsvektor ein für ein fluoreszierendes Protein kodierendes Gen in funktioneller Verknüpfung mit dem Gen von Interesse. Vorzugsweise erfolgt die Transkription beider Gene unter der Kontrolle eines einzigen heterologen Promotors, so dass das Protein/Produkt von Interesse und das fluoreszierende Protein durch eine bicistronische m-RNA kodiert werden. Dies ermöglicht eine Identifizierung von Zellen, die das Protein/Produkt von Interesse in hohen Menge produzieren, über die Expressionsrate des fluoreszierenden Proteins. Alternativ kann die Transkription des für das fluoreszierende Protein kodierenden Gens unter der Kontrolle eines eigenen Promotors erfolgen.

Bei dem fluoreszierenden Protein kann es sich z.B. um ein grün, blaugrün, blau, gelb oder andersfarben fluoreszierendes Protein handeln. Ein spezielles Beispiel ist das grün fluoreszierende Protein (GFP) aus *Aequorea victoria* oder *Renilla reniformis* und daraus entwickelte Mutanten; siehe z.B. Bennet et al. (1998); Chalfie et al. (1994); WO 01/04306 und die dort zitierte Literatur.

Weitere fluoreszierende Proteine und dafür kodierende Gene sind in WO 00/34318, WO 00/34326, WO 00/34526 und WO 01/27150 beschrieben, die durch Bezugnahme hierin inkorporiert werden. Bei diesen fluoreszierenden Proteinen handelt es sich um Fluorophore von nicht-biolumineszierenden Organismen der Spezies Anthozoa, beispielsweise von *Anemonia majano, Clavularia sp., Zoanthus sp.* I, *Zoanthus sp.* II, *Discosoma striata, Discosoma sp.* "red", *Discosoma sp.* "green", *Discosoma sp.* "Magenta", *Anemonia sulcata, Aequorea coerulescens.*

Die erfindungsgemäß eingesetzten Fluoreszenzproteine beinhalten neben den Wildtyp-Proteinen auch natürliche oder gentechnologisch hergestellte Mutanten und -varianten, deren Fragmente, Derivate oder z.B. mit anderen Proteinen oder Peptiden fusionierte Varianten. Die eingebrachten Mutationen können dabei beispielsweise das Exzitations- oder Emissionsspektrum, die Chromophorenbildung, den Extinktionskoeffizienten oder die Stabilität des Proteins verändern. Durch Codon-Optimierung kann zudem die Expression in Säugerzellen oder anderen Spezies verbessert werden. Erfindungsgemäß kann das fluoreszierende Protein auch in Fusion mit einem Selektionsmarker, bevorzugterweise mit einem amplifzierbaren Selektionsmarker wie beispielsweise der Dihydrofolat-Reduktase (DHFR), eingesetzt werden.

Die von den fluoreszierenden Proteinen emittierte Fluoreszenz ermöglicht die Detektion der Proteine z.B. durch Durchflusszytometrie mit einem Fluoreszenz-aktivierten Zellsortierer (FACS) oder durch Fluoreszenzmikroskopie.

### Weitere regulatorische Elemente:

Der Expressionsvektor enthält zumindest einen heterologen Promotor, der die Expression des Gens von Interesse und vorzugsweise auch die des fluoreszierenden Proteins ermöglicht.

Als "Promotor" wird eine Polynukleotidsequenz bezeichnet, die die Transkription der mit ihr funktionell verknüpften Gene oder Sequenzen ermöglicht und kontrolliert. Ein Promotor enthält Erkennungssequenzen für die Bindung der RNA-Polymerase und die Initiationsstelle der Transkription (Transkriptionsinitiationsstelle). Zur Expression einer gewünschten Sequenz in einem bestimmten Zelltyp oder einer Wirtszelle muss jeweils ein geeigneter, funktionaler Promotor gewählt werden. Der Fachmann kennt eine Vielzahl von Promotoren aus verschiedenen Quellen, einschließlich konstitutiver, induzierbarer und reprimierbarer Promotoren. Sie sind in Datenbanken, z.B. GenBank, hinterlegt und können als eigenständige oder innerhalb von Polynukleotidsequenzen klonierte Elemente von kommerziellen oder individuellen Quellen bezogen werden. In induzierbaren Promotoren kann die Aktivität des Promotors in Reaktion auf ein Signal reduziert oder verstärkt werden. Ein Beispiel für einen induzierbaren Promotor stellt der Tetracyclin (tet)-Promotor dar. Dieser enthält Tetracyclin-Operatorsequenzen (tetO), die durch ein Tetracyclinreguliertes Transaktivatorprotein (tTA) induziert werden können. In der Anwesenheit von Tetracyclin wird die Bindung von tTA an tetO inhibiert. Beispiele für weitere induzierbare Promotoren sind der jun-, fos-, Metallothionin- und Hitzeschockpromotor (siehe auch Sambrook et al., 1989; Gossen et al., 1994).

Unter den Promotoren, die für eine hohe Expression in Eukaryonten besonders gut geeignet sind, befinden sich beispielsweise der Ubiquitin/S27a-Promotor des Hamsters (WO 97/15664), der SV40 early Promotor, der Adenovirus major late Promotor, der Maus Metallothionin-I Promotor, die lange terminale Repeatregion des Rous Sarcoma Virus, der early Promotor des humanen Cytomegalie-Virus. Beispiele für andere heterologe Säugerpromotoren sind der/die Aktin-, Immunglobulin- oder Hitzeschockpromotor(en).

Ein entsprechender heterologer Promotor kann zur Steigerung/Regulierung der Transkriptionsaktivität in einer Expressionskassette in funktionellen Zusammenhang mit weiteren regulatorischen Sequenzen gebracht werden.

Beispielsweise kann der Promotor mit Enhancer-Sequenzen funktionell verknüpft werden, um die Transkriptionsaktivität zu steigern. Hierzu können ein oder mehrere Enhancer und/oder mehrere Kopien einer Enhancer-Sequenz verwendet werden, beispielsweise ein CMV- oder SV40-Enhancer. Dem entsprechend enthält ein erfindungsgemäßer Expressionsvektor in einer weiteren Ausführungsform einen oder mehrere Enhancer /Enhancer-Sequenzen, vorzugsweise einen CMV- oder SV40-Enhancer.

Mit dem Ausdruck "Enhancer" wird eine Polynukleotidsequenz bezeichnet, die in *cis-*Lokalisierung auf die Aktivität eines Promotors einwirkt und so die Transkription eines mit diesem Promotor funktionell verknüpften Gens stimuliert. Im Gegensatz zu Promotoren ist die Wirkung der Enhancer positions- und orientierungsunabhängig und können somit vor oder hinter eine Transkriptionseinheit, innerhalb eines Introns oder selbst innerhalb der Kodierregion positioniert werden. Der Enhancer kann dabei sowohl in unmittelbarer Nähe der Transkriptionseinheit als auch in beträchtlichem Abstand zum Promotor lokalisiert sein. Auch eine physikalische und funktionelle Überlappung mit dem Promotor ist möglich. Dem Fachmann sind eine Vielzahl von Enhancern aus verschiedenen Quellen bekannt (und in Datenbanken wie GenBank hinterlegt, z.B. SV40 Enhancer, CMV Enhancer, Polyoma Enhancer, Adenovirus Enhancer) und als eigenständige oder innerhalb von Polynukleotidsequenzen klonierte Elemente verfügbar (z.B. bei ATCC hinterlegt oder aus konunerziellen und individuellen Quellen). Eine Vielzahl von Promotorsequenzen beinhalten auch Enhancer-Sequenzen, wie z.B. der häufig verwendete CMV Promotor. Der humane CMV-Enhancer gehört dabei zu den stärksten bisher identifizierten Enhancern. Ein Beispiel für einen induzierbaren Enhancer ist der Metallothionein-Enhancer, der durch Glucocorticoide oder Schwermetalle stimuliert werden kann.

Eine weitere mögliche Modifikation ist z.B. die Einführung multipler Sp1-Bindungsstellen. Die Promotorsequenzen können ferner mit regulatorischen Sequenzen kombiniert werden, die eine Steuerung/Regulierung der Transkriptionsaktivität gestatten. So kann der Promotor reprimierbar/induzierbar gemacht werden. Dies kann beispielsweise durch die Verknüpfung mit Sequenzen geschehen, die Bindungsstellen für positiv oder negativ regulierende Transkriptionsfaktoren darstellen. Der oben genannte Transkriptionsfaktor SP-1 beispielsweise hat einen positiven Einfluss auf die Transkriptionsaktivität. Ein weiteres Beispiel ist die Bindungsstelle für das Aktivatorprotein AP-1, das sowohl in positiver als auch in negativer Weise auf die Transkription einwirken kann. Die Aktivität des AP-1 kann durch verschiedenste Faktoren, wie z.B. Wachstumsfaktoren, Zytokine und Serum, gesteuert werden (Faisst et al., 1992, und Referenzen darin). Die Transkriptionseffzienz kann auch dadurch gesteigert werden, dass die Promotorsequenz durch Mutation (Substitution, Insertion oder Deletion) von einer, zwei, drei oder mehr Basen verändert wird und dann in einem Reportergen-Assay bestimmt wird, ob sich dadurch die Promotoraktivität erhöht.

Grundsätzlich umfassen die zusätzlichen regulatorischen Elemente heterologe Promotoren, Enhancer, Terminations- und Polyadenylierungssignale und weitere Expressionskontrollelemente. Für die verschiedenen Zelltypen sind sowohl induzierbare als auch konstitutive regulatorische Sequenzen bekannt.

"Transkriptionsregulatorische Elemente" umfassen gewöhnlich zumindest einen Promotor stromaufwärts von der zu exprimierenden Gensequenz, Transkriptionsinitiations- und - terminationsstellen sowie ein Polyadenylierungssignal.

Der Ausdruck "Transkriptionsinitiationsstelle" bezieht sich auf eine Nukleinsäure in dem Konstrukt, die der ersten Nukleinsäure entspricht, welche in das primäre Transkript, d.h. den mRNA-Precursor, inkorporiert wird. Die Transkriptionsinitiationsstelle kann mit den Promotorsequenzen überlappen.

Der Ausdruck "Transkriptionsterminationsstelle" bezieht sich auf eine Nukleotidsequenz, die normalerweise am 3'-Ende des Gens von Interesse oder des zu transkribierenden Genabschnitts vorhanden ist und die den Abbruch der Transkription durch RNA-Polymerase bewirkt.

Das "Polyadenylierungssignal" ist eine Signalsequenz, welche die Spaltung an einer spezifischen Stelle am 3'-Ende der eukaryontischen mRNA und den posttranskriptionellen Einbau einer Sequenz von etwa 100-200 Adeninnukleotiden (polyA-Schwanz) am gespattenen 3'-Ende verursacht. Das Polyadenylierungssignal umfasst die Sequenz AATAAA etwa 10-30 Nukleotide stromaufwärts von der Spaltstelle sowie eine stromabwärts gelegene Sequenz. Es sind verschiedene Polyadenylierungselemente bekannt, z.B. tk polyA, SV40 late und early polyA oder BGH polyA (z.B. beschrieben in US 5,132,458).

"Translationsregulatorische Elemente" umfassen eine Translationsinitiationsstelle (AUG), ein Stoppcodon und ein polyA-Signal für jedes zu exprimierende Polypeptid. Für eine optimale Expression kann es günstig sein, 5'- und/oder 3'-nichttranslatierte Bereiche der zu exprimierenden Nukleinsäuresequenz zu entfernen, hinzuzufügen oder zu ändern, um potentielle zusätzliche ungeeignete Translationsinitiationscodons oder andere Sequenzen, welche die Expression auf dem Niveau der Transkription oder Expression beeinträchtigen können, zu climinieren. Um die Expression zu fördern, kann man alternativ ribosomale Konsensus-Bindungsstellen unmittelbar stromaufwärts vom Startcodon inserieren. Um ein sekretiertes Polypeptid zu produzieren, enthält das Gen von Interesse gewöhnlich eine Signalsequenz, welche für ein Signalvorläuferpeptid kodiert, die das synthetisierte Polypeptid zu und durch die ER-Membran transportiert. Die Signalsequenz befindet sich oft, jedoch nicht immer, am Aminoterminus des sekretierten Proteins und wird durch Signal-Peptidasen abgespalten, nachdem das Protein durch die ER-Membran geschleust wurde. Die Gensequenz wird gewöhnlich, jedoch nicht notwendigerweise, eine eigene Signalsequenz enthalten. Wenn die native Signalsequenz nicht vorhanden ist, kann in bekannter Weise eine heterologe Signal sequenz eingeführt werden. Dem Fachmann sind zahlreiche solcher Signalsequenzen bekannt und in Sequenzdatenbanken wie GenBank und EMBL hinterlegt.

Ein weiteres regulatorisches Element ist die interne Ribosomenbindungsstelle (IRES). Das "IRES-Element" umfasst eine Sequenz, welche die Translationsinitiation unabhängig von einer 5'-terminalen Methylguanosiniumkappe (CAP-Struktur) sowie dem stromaufwärts gelegenen Gen funktionell bewerkstelligt und in einer tierischen Zelle die Translation zweier Cistrone (offener Leseraster) von einem einzigen Transkript ermöglicht. Das IRES-Element stellt eine unabhängige Ribosomenbindungsstelle für die Translation des unmittelbar stromabwärts gelegenen offenen Leserasters zur Verfügung. Im Gegensatz zu bakterieller mRNA, die multicistronisch sein kann, d.h. für mehrere verschiedene Polypeptide oder Produkte kodieren kann, die nacheinander von der mRNA translatiert werden, sind die meisten mRNAs von Tierzellen monocistronisch und kodieren nur für ein einziges Protein oder Produkt. Bei einem multicistronischen Transkript in einer eukaryontischen Zelle würde die Translation von der stromaufwärts am nächsten gelegenen Translationsinitiationsstelle initiiert und vom ersten Stoppcodon beendet werden, worauf das Transkript aus dem Ribosom freigesetzt würde. Bei der Translation entstünde somit nur das erste von der mRNA kodierte Polypeptid oder Produkt. Dagegen ermöglicht ein multicistronisches Transkript mit einem IRES-Element, das mit dem zweiten oder weiteren offenen Leserastern in dem Transkript funktionell verknüpft ist, die anschließende Translation des stromabwärts gelegenen offenen Leserasters, so dass in der eukaryontischen Zelle zwei oder mehr, von demselben Transkript kodierte Polypeptide oder Produkte produziert werden.

Das IRES-Element kann von verschiedener Länge und von verschiedenem Ursprung sein und z.B. vom Encephalomyocarditisvirus (EMCV) oder anderen Picornaviren stammen. In der Literatur sind verschiedene IRES-Sequenzen und deren Anwendung bei der Konstruktion von Vektoren beschrieben worden; siehe z.B. Pelletier et al., 1988; Jang et al., 1989; Davies et al., 1992; Adam et al., 1991; Morgan et al., 1992; Sugimoto et al., 1994; Ramesh et al., 1996, Mosser et al., 1997.

Die stromabwärts gelegene Gensequenz ist mit dem 3'-Ende des IRES-Elements funktionell verknüpft, d.h. der Abstand wird so gewählt, dass die Expression des Gens nicht oder nur marginal beeinflusst wird bzw. eine für den Zweck ausreichende Expression aufweist. Der optimale und zulässige Abstand zwischen dem IRES-Element und dem Startcodon des stromabwärts gelegenen Gens für eine noch ausreichende Expression lässt sich in einfachen Versuchen durch Variation des Abstands und Bestimmung der Expressionsrate als Funktion des Abstandes mit Hilfe von Reportergen-Assays ermitteln.

Durch die geschilderten Maßnahmen kann eine optimierte Expressionskassette erhalten werden, die von hohem Nutzen für die Expression heterologer Genprodukte ist. Eine durch eine oder mehrere solcher Maßnahmen erhaltene Expressionskassette ist daher ebenfalls Gegenstand der Erfindung.

### Hamster-Ubiquitin/S27a-Promotor:

In einer weiteren Ausführungsform enthält der erfindungsgemäße Expressionsvektor den Ubiquitin/S27a-Promotor des Hamsters, vorzugsweise in funktioneller Verknüpfung mit dem Gen von Interesse und noch mehr bevorzugt in funktioneller Verknüpfung mit dem Gen von Interesse und dem Gen, das für ein fluoreszierendes Protein oder einen Selektionsmarker kodiert.

Der Ubiquitin/S27a-Promotor des Hamsters ist ein starker homologer Promotor, der in der WO 97/15664 beschrieben ist. Ein solcher Promotor weist vorzugsweise mindestens eines der folgenden Merkmale auf: GC-reicher Sequenzbereich, Sp1-Bindungsstelle, Polypyrimidinelement, Abwesenheit einer TATA-Box. Besonders bevorzugt ist ein Promotor, der eine Sp1-Bindungsstelle bei Abwesenheit einer TATA-Box aufweist. Ferner ist ein solcher Promotor bevorzugt, der konstitutiv aktiviert ist und insbesondere unter serumhaitigen, serumarmen und serumfreien Zellkulturbedingungen gleichermaßen aktiv ist. In einer anderen Ausführungsform handelt es sich um einen induzierbaren Promotor, insbesondere um einen Promotor, der durch Serumentzug aktiviert wird.

Eine besonders vorteilhafte Ausführungsform ist ein Promotor mit einer Nukleotidsequenz, die in Fig. 5 der WO 97/15664 enthalten ist. Besonders bevorzugt sind dabei Promotorsequenzen, in denen die Sequenz von Position -161 bis - 45 von Fig. 5 enthalten ist.

Die in den Beispielen der vorliegenden Patentbeschreibung verwendeten Promotoren beinhalten jeweils ein DNA-Molekül mit einer Sequenz, die dem Fragment - 372 bis + 111 aus Fig. 5 der WO 97/15664 entspricht und repräsentiert den bevorzugten Promotor, d.h. ein bevorzugter Promotor sollte diesen Sequenzbereich umfassen.

### Herstellung erfindungsgemäßer Expressionsvektoren:

Die Herstellung des erfindungsgemäßen Expressionsvektors kann grundsätzlich nach herkömmlichen, dem Fachmann geläufigen Methoden, wie z.B. bei Sambrock et al. (1989), beschrieben, erfolgen. Dort findet sich auch eine Beschreibung der funktionellen Komponenten eines Vektors, z.B. geeigneter Promotoren (zusätzlich zum Hamster-Ubiquitin/S27a-Promotor), Enhancer, Terminations- und Polyadenylierungssignale, Antibiotikaresistenzgene, Selektionsmarker, Replikationsstartpunkte und Spleisssignale. Zur Herstellung können herkömmliche Klonierungsvektoren verwendet werden, z.B. Plasmide, Bacteriophagen, Phagemide, Cosmide oder virale Vektoren wie Baculovirus, Retroviren, Adenoviren, Adeno-assoziierte Viren und Herpes Simplex-Virus, aber auch künstliche oder artifizielle oder Mini-Chromosomen.. Die eukaryontischen Expressionsvektoren enthalten typischerweise auch prokaryontische Sequenzen wie z.B. Replikationsursprung und Antibiotikaresistenzgene, die die Vermehrung und Selektion des Vektors in Bakterien ermöglichen. Eine Vielzahl von eukaryontischen Expressionsvektoren, die multiple Klonierungsstellen zur Einführung einer Polynukleotidsequenz enthalten, sind bekannt und einige sind kommerziell bei verschiedenen Firmen wie Stratagene, La Jolla, CA, USA; Invitrogen, Carlsbad, CA, USA; Promega, Madison, WI, USA oder BD Biosciences Clontech, Palo Alto, CA, USA erhältlich.

Auf eine dem Fachmann geläufige Weise werden der heterologe Promotor, das Gen (oder Gene) von Interesse, Selektionsmarker sowie gegebenenfalls das für ein fluoreszierendes Protein kodierende Gen, zusätzliche regulatorische Elemente wie beispielsweise die interne Ribosomenbindungsstelle (IRES), Enhancer, Polyadenylierungssignal und andere *cis-*aktive Elemente, wie beispielsweise TE-Elemente, in den Expressionsvektor eingeführt. Ein erfindungsgemäßer Expressionsvektor enthält minimal einen heterologen Promotor, das Gen von Interesse und ein TE-Element. Vorzugsweise enthält der Expressionsvektor noch ein für ein fluoreszierendes Protein kodierendes Gen. Erfindungsgemäß ist insbesondere die Verwendung eines Ubiquitin/S27a-Promotors als heterologen Promotor. Besonders bevorzugt ist ein Expressionsvektor, bei dem der heterologe Promotor, vorzugsweise ein Ubiquitin/S27a-Promotor, das Gen von Interesse und ein TE-Element funktionell miteinander verknüpft sind oder in funktioneller Verknüpfung stehen

Im Rahmen der vorliegenden Beschreibung bezieht sich der Ausdruck "funktionelle Verknüpfung" bzw. "funktionell verknüpft" auf zwei oder mehr Nukleinsäuresequenzen oder -teilsequenzen, die so positioniert sind, dass sie ihre beabsichtigte Funktion ausüben können. Beispielsweise sind ein Promotor/Enhancer, ein Promotor/TE-Element oder ein Promotor/Enhancer/TE-Element funktionell mit einer kodierenden Gensequenz verknüpft, wenn sie in cis-Stellung die Transkription der verknüpften Gensequenz kontrollieren oder modulieren können. Im Allgemeinen, jedoch nicht notwendigerweise, befinden sich funktionell verknüpfte DNA-Sequenzen in enger Nachbarschaft und, sofern zwei kodierende Gensequenzen verknüpft werden oder im Falle einer Sekretionssignalsequenz, im gleichen Leseraster. Obwohl sich ein funktionell verknüpfter Promotor im Allgemeinen stromaufwärts von der kodierenden Gensequenz befindet, muss er nicht notwendigerweise eng benachbart sein. Enhancer oder TE-Elemente müssen ebenfalls nicht in enger Nachbarschaft vorliegen, solange sie die Transkription bzw. Expression der Gensequenz begünstigen. Zu diesem Zweck können sie sowohl stromaufwärts als auch stromabwärts von der Gensequenz vorliegen, gegebenenfalls in einigem Abstand. Eine Polyadenylierungsstelle ist funktionell mit einer Gensequenz verknüpft, wenn sie am 3'-Ende der Gensequenz derart positioniert ist, dass die Transkription über die kodierende Sequenz bis hin zum Polyadenylierungssignal fortschreitet. Die Verknüpfung kann nach üblichen rekombinanten Methoden erfolgen, z.B. mittels der PCR-Technik, durch Ligation an geeigneten Restriktionsschnittstellen oder durch Spleissen. Wenn keine geeigneten Restriktionsschnittstetlen vorhanden sind, können in an sich bekannter Weise synthetische Oligonukleotid-Linker oder Adaptoren verwendet werden.

In einer der beschriebenen Ausführungsformen sind der heterologe Promotor, vorzugsweise ein Ubiquitin/S27a-Promotor oder CMV-Promotor, das Gen von Interesse und das für ein fluoreszierendes Protein kodierende Gen funktionell miteinander verknüpft. Dies meint z.B., dass sowohl das Gen von Interesse als auch das für ein fluoreszierendes Protein kodierende Gen ausgehend von dem selben heterologen Promotor exprimiert werden. In einer besonders bevorzugten Ausführungsform erfolgt die funktionelle Verknüpfung über ein IRES-Element, so dass von beiden Genen eine bicistronische mRNA synthetisiert wird. Der erfindungsgemäße Expressionsvektor kann zusätzlich Enhancer-Elemente und/oder TE-Elemente enthalten, die funktionell auf einen oder mehrere Promotoren wirken. Besonders bevorzugt ist ein Expressionsvektor, bei dem der heterologe Promotor, vorzugsweise der Ubiquitin/S27a-Promotor bzw. eine modifizierte Form hiervon oder der CMV-Promotor, mit einem Enhancer-Element, z.B. einen SV40-Enhancer oder einem CMV-Enhancer-Element, sowie einem TE-Element verknüpft ist.

Grundsätzlich kann die Expression der Gene innerhalb eines Expressionsvektors von einer oder mehreren Transkriptionseinheiten aus erfolgen. Als "Transkriptionseinheit" wird Dabei eine Region definiert, die ein oder mehr zu transkribierende Gene enthält. Dabei sind die Gene innerhalb einer Transkriptionseinheit funktionell derart miteinander verknüpft, dass alle Gene innerhalb einer solchen Einheit unter der transkriptionellen Kontrolle desselben Promotors, Promotors/Enhancers oder Promotors/Enhancers/TE-Elements stehen. Als Resultat dieser transkriptionellen Verknüpfung von Genen kann mehr als ein Protein oder Produkt von einer Transkriptionseinheit aus transkribiert und somit exprimiert werden. Jede Transkriptionseinheit enthält dabei die regulatorischen Elemente, die für die Transkription und die Translation der in ihr enthaltenen Gensequenzen erforderlich sind. Jede Transkriptionseinheit kann dabei die gleichen oder verschiedene regulatorische Elemente enthalten. Für die funktionelle Verknüpfung der Gene innerhalb einer Transkriptionseinheit können IRES-Elemente oder Introns verwendet werden.

Der Expressionsvektor kann eine einzige Transkriptionseinheit zur Expression des Gens (oder auch Gene) von Interesse, des Selektionsmarkers und gegebenenfalls des Gens, das für das Fluoreszenzprotein kodiert, enthalten. Alternativ können diese Gene auch in zwei oder mehr Transkriptionseinheiten angeordnet sein. Dabei sind verschiedene Kombinationen der Gene innerhalb einer Transkriptionseinheit möglich. In einer weiteren Ausführungsform der vorliegenden Erfindung kann mehr als ein Expressionsvektor, bestehend aus ein, zwei oder mehr Transkriptionseinheiten, in eine Wirtszelle durch Co-Transfektion oder in aufeinanderfolgenden Transfektion in beliebiger Reihenfolge eingeführt werden. Jede Kombination von regulatorischen Elementen und Genen auf jedem Vektor kann gewählt werden, so lange eine ausreichende Expression der Transkriptionseinheiten gewährleistet ist. Falls erforderlich können weitere regulatorische Elemente, wie beispielsweise TE-Elemente, und Gene, wie z.B. zusätzliche Gene von Interesse oder Selektionsmarker, auf den Expressionsvektoren positioniert werden.

Erfindungsgemäß sind auch solche Expressionsvektoren, die ein oder mehrere TE-Elemente enthalten, und die anstelle des Gens von Interesse lediglich eine multiple Klonierungsstelle aufweisen, die die Klonierung des Gens von Interesse über Erkennungssequenzen für Restriktionsendonukleasen ermöglicht. Im Stand der Technik sind zahlreiche Erkennungssequenzen für die verschiedensten Restriktionsendonukleasen, sowie die hierzu gehörigen Restriktionsendonukleasen bekannt. Bevorzugt werden Sequenzen verwendet, die aus zumindest 6 Nukleotiden als Erkennungssequenz bestehen. Eine Auflistung geeigneter Erkennungssequenzen finden sich beispielsweise in Sambrook et al., (1989).

Weiterhin erfindungsgemäß sind auch solche Expressionsvektoren, die anstelle des Gens von Interesse lediglich eine multiple Klonierungsstelle, die die Klonierung des Gens von Interesse über Erkennungssequenzen für Restriktionsendonukleasen ermöglicht und die zudem eine oder mehrere, vorzugsweise multiple, Klonierungsstellen an verschiedenen Positionen des Expressionsvektors aufweisen, die zusätzlich die Klonierung von TE-Elemente über Erkennungssequenzen für Restriktionsendonukleasen ermöglicht. Im Stand der Technik sind zahlreiche Erkennungssequenzen für die verschiedensten Restriktionsendonukleasen, sowie die hierzu gehörigen Restriktionsendonukleasen bekannt. Bevorzugt werden Sequenzen verwendet, die aus zumindest 6 Nukleotiden als Erkennungssequenz bestehen. Eine Auflistung geeigneter Erkennungssequenzen finden sich beispielsweise in Sambrook et al., (1989).

### Wirtszellen:

Zur Transfektion mit dem erfindungsgemäßen Expressionsvektor werden eukaryontische Wirtszellen verwendet, vorzugsweise Säugerzellen und insbesondere Nagerzellen wie z.B. Mäuse-, Ratten- und Hamster-Zelllinien. Die erfolgreiche Transfektion der entsprechenden Zellen mit einem erfindungsgemäßen Expressionsvektor resultiert in transformierten, genetisch modifizierten, rekombinanten oder transgenen Zellen, die ebenfalls Gegenstand der vorliegenden Erfindung sind.

Im Rahmen der Erfindung bevorzugte Wirtszellen sind Hamsterzellen wie z.B. BHK21, BHK TK⁻, CHO, CHO-K1, CHO-DUKX, CHO-DUKX B1 und CHO-DG44 Zellen oder Derivate/Abkömmlinge dieser Zelllinien. Besonders bevorzugt sind CHO-DG44, CHO-DUKX, CHO-K1 und BHK21 Zellen, insbesondere CHO-DG44 und CHO-DUKX Zellen. Ebenfalls geeignet sind Myelomzellen der Maus, vorzugsweise NS0 und Sp2/0 Zellen sowie Derivate/Abkömmlinge dieser Zelllinien.

Beispiele für Hamster- und Mäusezellen, die erfindungsgemäß angewandt werden können, sind in der folgenden Tabelle 1 angegeben. Aber auch Derivate und Abkömmlinge dieser Zellen, andere Säugerzellen, einschließlich aber nicht beschränkt auf Zelllinien von Mensch. Maus, Ratte, Affen, Nagetieren, oder eukaryontische Zellen, einschließlich aber nicht beschränkt auf Hefe-, Insekten- , Vogel- und Pflanzenzellen, können ebenfalls als Wirtszellen zur Produktion von biopharmazeutischen Proteinen verwendet werden.

**Tabelle 1: Hamster- and Mäuse-Produktionszelllinien**

| Zelllinie | Hinterlegungsnummer |
|---|---|
| NS0 | ECACC No. 85110503 |
| Sp2/0-Ag14 | ATCC CRL-1581 |
| BHK21 | ATCC CCL-10 |
| BHK TK⁻ | ECACC No. 85011423 |
| HaK | ATCCCCL-15 |
| 2254-62.2 (BHK-21-Derivat) | ATCC CRL-8544 |
| CHO | ECACC No. 8505302 |
| CHO-K1 | ATCC CCL-61 |
| CHO-DUKX (= CHO duk⁻, CHO/dhfr⁻) | ATCC CRL-9096 |
| CHO-DUKX B 1 | ATCC CRL-9010 |
| CHO-DG44 | Urlaub et al., Cell 33[2], 405-412, 1983 |
| CHO Pro-5 | ATCC CRL-1781 |
| V79 | ATCC CCC-93 |
| B14AF28-G3 | ATCC CCL-14 |
| CHL | ECACC No. 87111906 |

Die Transfektion der eukaryontischen Wirtszellen mit einem Polynukleotid oder einem der erfindungsgemäßen Expressionsvektor, erfolgt nach üblichen Methoden (Sambrook et al., 1989; Ausubel et al., 1994). Geeignete Transfektionsmethoden sind z.B. die Liposomenvermittelte Transfektion, Calciumphosphat-Copräzipitation, Elektroporation, Polykationen (z.B. DEAE-Dextran)-vermittelte Transfektion, Protoplastenfusion, Mikroinjektion und virale Infektionen. Erfindungsgemäß wird vorzugsweise eine stabile Transfektion durchgeführt, wobei die Konstrukte entweder in das Genom der Wirtszelle oder ein artifizielles Chromosom/Minichromosom integriert werden oder in stabiler Weise episomal in der Wirtszelle enthalten sind. Die Transfektionsmethode, die die optimale Transfektionsfrequenz und Expression des heterologen Gens in der jeweiligen Wirtszelle ermöglicht, ist dabei bevorzugt. Per Definition wird jede Sequenz oder jedes Gen, das in eine Wirtszelle eingebracht wird, in Bezug auf die Wirtszelle als "heterologe Sequenz" oder "heterologes Gen" bezeichnet. Selbst dann, wenn die einzubringende Sequenz oder das einzubringende Gen identisch zu einer endogenen Sequenz oder einem endogenen Gen der Wirtszelle ist. Beispielsweise ist ein Hamster Aktingen, das in eine Hamster-Wirtszelle eingebracht wird, per Definition ein heterologes Gen.

Erfindungsgemäß sind insbesondere rekombinante Säugerzellen, vorzugsweise Nagerzellen, besonders bevorzugt Hamsterzellen, wie z.B. CHO- oder BHK-Zellen, die mit einem der hier beschriebenen erfindungsgemäßen Expressionsvektoren transfiziert wurden.

Bei der rekombinanten Herstellung heteromerer Proteine, wie z.B. monoklonaler Antikörper (mAk), kann die Transfektion geeigneter Wirtszellen prinzipiell auf zwei verschiedenen Wegen erfolgen. Derartige mAk sind aus mehreren Untereinheiten, den schweren und leichten Ketten, aufgebaut. Für diese Untereinheiten kodierende Gene können in unabhängigen oder in multicistronischen Transkriptionseinheiten auf einem einzigen Plasmid untergebracht werden, mit dem dann die Wirtszelle transfiziert wird. Dies soll die stöchiometrische Repräsentanz der Gene nach Integration in das Genom der Wirtszelle sichern. Allerdings muss hierbei im Falle unabhängiger Transkriptionseinheiten sichergestellt werden, dass die mRNAs, die für die verschiedenen Proteine kodieren, die gleiche Stabilität, Transkriptions- und Translationseffizienz aufweisen. Im zweiten Fall erfolgt die Expression der Gene innerhalb einer multicistronischen Transkriptionseinheit durch einen einzigen Promotor und es entsteht nur ein Transkript. Durch Verwendung von IRES-Elementen wird eine recht effiziente interne Translationsinitiation der Gene in dem zweiten und den nachfolgenden Cistrons ermöglicht. Dennoch sind die Expressionsraten für diese Cistrons geringer als die des ersten Cistrons, dessen Translationsinitiation über einen sogenannten "cap"-abhängigen Prä-Initiationskomplex wesentlich effizienter ist als die IRES-abhängige Translationsinitiation. Um eine tatsächlich äquimolare Expression der Cistrons zu erreichen, können beispielsweise noch zusätzliche intercistronische Elemente eingeführt werden, die im Zusammenwirken mit den IRES-Elementen für einheitliche Expressionsraten sorgen (WO 94/05785).

Eine weitere erfindungsgemäße Möglichkeit der Herstellung mehrerer heterologer Proteine ist die sequenzielle Transfektion, bei der die Gene zeitlich getrennt in verschiedenen Expressionsvektoren integriert transfiziert werden. Zum Beispiel können die schwere Kette und die leichte Kette eines Antikörpers hintereinander in zwei Transfektionsschritten in eine Zelle eingebracht werden.

Eine andere und erfindungsgemäß bevorzugte Möglichkeit der simultanen Herstellung mehrerer heterologer Proteine ist die Co-Transfektion, bei der die Gene getrennt in verschiedene Expressionsvektoren integriert werden. Dies hat den Vorteil, dass bestimmte Verhältnisse der Gene und Genprodukte zueinander eingestellt werden können, wodurch Unterschiede in der mRNA-Stabilität sowie in der Transkriptions- und Translationseffizienz ausgeglichen werden können. Außerdem sind die Expressionsvektoren wegen ihrer geringeren Größe stabiler und sowohl bei der Klonierung als auch bei der Transfektion einfacher handhabbar.

In einer besonderen Ausführungsform der Erfindung werden daher die Wirtszellen zusätzlich mit einem oder mehreren Vektoren mit Genen, die für ein oder mehrere andere Proteine von Interesse kodieren, transfiziert, bevorzugt co-transfiziert. Der oder die zur Co-Transfektion verwendeten weiteren Vektoren kodieren z.B. für das oder die anderen Proteine von Interesse unter der Kontrolle des gleichen Promotors, bevorzugt unter der Kontrolle der gleichen Promotor/Enhancer-Kombination oder besonders bevorzugt unter der Kontrolle der gleichen Promotor/Enhancer/TE-Element Kombination oder auch unter der Kontrolle der gleichen Promotor/Enhancer-Kombination mit unterschiedlichen TE-Elementen sowie für zumindest einen Selektionsmarker, z.B. die Dihydrofolat-Reduktase. In einer weiteren Ausführungsform der Erfindung können die zur Transfektion verwendeten Vektoren ein oder mehrere TE-Elemente in beliebiger Kombination, Position und Orientierung enthalten.

In einer weiteren besonderen Ausführungsform der Erfindung werden die Wirtszellen mit zumindest zwei eukaryontischen Expressionsvektoren co-transfiziert, wobei zumindest der eine der beiden Vektoren zumindest ein Gen), das zumindest Protein von Interesse kodiert, enthält und der andere Vektor ein oder mehrere erfindungsgemäße Nukleinsäuren in beliebiger Kombination, Position und Orientierung enthält, und optional auch für zumindest ein Gen (von Interesse kodiert, und diese erfindungsgemäßen Nukleinsäuren ihre transkriptions- bzw. expressionssteigernde Wirkung auf die Gene von Interesse, die auf dem anderen co-transfizierten Vektor lokalisiert sind, via Co-Integration mit dem anderen Vektor vermitteln.
Erfindungsgemäß werden die Wirtszellen vorzugsweise unter serumfreien Bedingungen etabliert, adaptiert und kultiviert, gegebenenfalls in Medien, die frei von tierischen Proteinen /Peptiden sind. Beispiele für im Handel erhältliche Medien sind Ham's F12 (Sigma, Deisenhofen, DE), RPMI-1640 (Sigma), Dulbecco's Modified Eagle's Medium (DMEM; Sigma), Minimal Essential Medium (MEM; Sigma), Iscove's Modified Dulbecco's Medium (IMDM; Sigma), CD-CHO (Invitrogen, Carlsbad, CA, USA), CHO-S-SFMII (Invitrogen), serumfreies CHO-Medium (Sigma) und proteinfreies CHO-Medium (Sigma). Jedes dieser Medien kann gegebenenfalls mit verschiedenen Verbindungen ergänzt werden, z.B. Hormonen und/oder anderen Wachstumsfaktoren (z.B. Insulin, Transferrin, epidermalem Wachstumsfaktor, Insulin-ähnlichem Wachstumsfaktor), Salzen (z.B. Natriumchlorid, Calcium, Magnesium, Phosphat), Puffern (z.B. HEPES), Nukleosiden (z.B. Adenosin, Thymidin), Glutamin, Glukose oder anderen äquivalenten Nährstoffen, Antibiotika und/oder Spurenelementen. Obwohl erfindungsgemäß serumfreic Medien bevorzugt sind, können zur Züchtung der Wirtszellen auch Medien verwendet werden, die mit einer geeigneten Menge an Serum versetzt wurden. Zur Selektion von genetisch modifizierten Zellen, die ein oder mehrere Selektionsmarkergene exprimieren, wird dem Medium ein oder mehrere geeignete Selektionsmittel zugefügt.

Als "Selektionsmittel" wird eine Substanz bezeichnet, die das Wachstum oder das Überleben von Wirtszellen mit einer Defzienz für das jeweilige Selektionsmarkergen beeinträchtigt. Im Rahmen der vorliegenden Erfindung wird vorzugsweise Geneticin (G418) als Mediumzusatz zur Selektion heterologer Wirtszellen, die ein Wildtyp- oder vorzugsweise ein modifiziertes Neomycin-Phosphotransferase-Gen tragen, verwendet. Vorzugsweise werden G418-Konzentrationen zwischen 100 und 800 µg/mL Medium verwendet, besonders bevorzugt sind 200 bis 400 µg G418/mL, Medium. Sollten die Wirtzellen mit mehreren Expressionsvektoren transfiziert werden, z.B. wenn separat mehrere Gene von Interesse in die Wirtszelle eingebracht werden sollen, so verfügen diese meist über verschiedene Selektionsmarkergene.

Ein "Selektionsmarkergen" ist ein Gen, das die spezifische Selektion von Zellen, die dieses Gen erhalten, durch Zugabe eines entsprechenden Selektionsmittels in das Kultivierungsmedium ermöglicht. Zur Verdeutlichung, ein Antibiotika-Resistenzgen kann als positiver Selektionsmarker verwendet werden. Nur Zellen, die mit diesem Gen transformiert wurden, können in der Gegenwart des entsprechenden Antibiotikums wachsen und somit selektioniert werden. Nichttransfomierte Zellen hingegen können unter diesen Selektionsbedingungen nicht wachsen oder überleben. Es gibt positive, negative und bifunktionale Selektionsmarker. Positive Selektionsmarker ermöglichen die Selektion und damit Anreicherung von transformierten Zellen durch Vermittlung einer Resistenz gegenüber dem Selektionsmittel oder durch Kompensierung eines metabolischen oder katabolischen Defekts der Wirtszelle. Im Gegensatz dazu können durch negative Selektionsmarker Zellen, die das Gen für den Selektionsmarker erhalten haben, selektiv eliminiert werden. Ein Beispiel hierfür ist das Thymidinkinasegen des Herpes Simplex Virus, dessen Expression -in Zellen bei gleichzeitiger Gabe von Acyclovir oder Gancyclovir zu deren Elimination führt. Die in dieser Erfindung verwendeten Selektionsmarker, einschließlich der amplifzierbaren Selektionsmarker, schließt gentechnologisch veränderte Mutanten und Varianten, Fragmente, funktionelle Äquivalente, Derivate, Homologe und Fusionen mit anderen Proteinen oder Peptiden ein, solange der Selektionsmarker seine selektiven Eigenschaften beibehält. Solche Derivate weisen eine beträchtliche Homologie in der Aminosäuresequenz in den Bereichen oder Domänen auf, denen die selektive Eigenschaft zugeschrieben wird. In der Literatur sind eine Vielzahl von Selektionsmarkergenen, einschließlich bifunktionaler (positiv/negativ) Marker, beschrieben (siehe z.B. WO 92/08796 und WO 94/28143). Beispiele für Selektionsmarker, die für gewöhnlich in eukaryontischen Zellen verwendet werden, beinhalten die Gene für Aminoglykosid-Phosphotransferase (APH), Hygromycin-Phosphotransferase (HYG), Dihydrofolat-Reduktase (DHFR), Thymidinkinase (TK), Glutamin-Synthetase, Asparagin-Synthetase und Gene, die Resistenz gegenüber Neomycin (G418), Puromycin, Histidinol D, Bleomycin, Phleomycin und Zeocin vermitteln.

Unter "modifizierte Neomycin-Phosphotransferase (NPT)" fallen alle in WO2004/050884 beschriebenen Mutanten, insbesondere die Mutante D227G (Asp227Gly), die sich durch den Austausch von Asparaginsäure (Asp, D) gegen Glycin (Gly, G) an Aminosäureposition 227 auszeichnet und besonders bevorzugt die Mutante F240I (Phe240Ile), die sich durch den Austausch von Phenylalanin (Phe, F) gegen Isoleucin (Ile, I) an Aminosäureposition 240 auszeichnet.

Die vorliegende Erfindung schließt deshalb eine Methode zur Herstellung und Selektion von rekombinanten Säugerzellen ein, die folgende Schritte enthält: (i) Transfektion der Wirtszellen mit Genen, die zumindest für ein Protein/Produkt von Interesse und eine Neomycin-Phosphotransferase, vorzugsweise modifiziert, kodieren, wobei zumindest das Gen (bzw. Gene) von Interesse zur Transkriptions- bzw. Expressionssteigerung funktionell mit zumindest einem TE-Element verknüpft ist; (ii) Kultivierung der Zellen unter Bedingungen, die eine Expression der verschiedenen Gene ermöglichen; und (iii) die selektion dieser co-integrierten Gene durch Kultivierung der Zellen in Gegenwart eines Selektionsmittels, wie z.B. G418. Bevorzugt werden die transfizierten Zellen hierbei in Medium in der Abwesenheit von Serum kultiviert. Verzugsweise beträgt die Konzentration an G418 zumindest 200 µg/mL. Die Konzentration kann aber auch zumindest 400 µg/mL betragen.

### Amplifizierbares Selektionsmarkergen:

Ferner können die erfindungsgemäßen Zellen optional auch einem oder mehreren Genamplifikationsschritten unterzogen werden, in dem sie in Gegenwart eines Selektionsmittels kultiviert werden, dass zu einer Amplifikation eines amplifzierbaren Selektionsmarkergens führt.
Voraussetzung ist, dass die Wirtszellen zusätzlich mit einem Gen transfiziert werden, das für einen amplifizierbaren Selektionsmarker kodiert. Denkbar ist, dass das Gen, welches für einen amplifizierbaren Selektionsmarker kodiert, auf einem der erfindungsgemäßen Expressionsvektoren vorhanden ist oder aber mit Hilfe eines weiteren Vektors in die Wirtszelle eingebracht wird.

Das amplifizierbare Selektionsmarkergen kodiert gewöhnlich für ein Enzym, das für das Wachstum von eukaryontischen Zellen unter bestimmten Kultivierungsbedingungen erforderlich ist. Beispielsweise kann das amplifizierbare Selektionsmarkergen für Dihydrofolat-Reduktase (DHFR) kodieren. In diesem Fall wird das Gen amplifiziert, wenn man eine damit transfizierte Wirtszelle in Gegenwart des Selektionsmittels Methotrexat (MTX) kultiviert.

In der folgenden Tabelle 2 sind Beispiele für weitere erfindungsgemäß verwendbare amplitizierbare Selektionsmarkergene und die dazugehörigen Selektionsmittel angegeben, dic in einem Überblick bei Kaufman, Methods in Enzymology, 185:537-566 (1990) beschrieben sind.

**Tabelle 2: Amplifizierbare Selektionsmarkergene**

| Amplifizierbares Selektionsmarkergen | Hinterlegungsnummer | Selektionsmittel |
|---|---|---|
| DHFR (Dihydrololatreduktase) | M 19869 (Hamster) | Methotrexat (MTX) |
| | E00236 (Maus) | |
| Metallothioncin | D10551 (Hamster) | Cadmium |
| | M13003 (Human) | |
| | M11794 (Ratte) | |
| CAD (Carbamoylphosphat-Synthetase : Aspartat-Transcarbamylase: Dihydroorotase) | M23652 (Hamster) | N-Phosphoacetyl-L-aspartat |
| | D78586 (Human) | |
| Adenosin-Deaminase | K02567 (Human) | Xyl-A- oder Adenosin, 2'Deoxycoformycin |
| | M 10319 (Maus) | |
| AMP (Adenylat)-Deaminase | D 12775 (Human) | Adenin, Azaserin, |
| | J02811 (Ratte) | Coformycin |
| UMP-Synthase | J03626 (Human) | 6-Azauridin, Pyrazofuran |
| IMP 5'-Dehydrogenase | J04209 (Hamster) | Mycophenolsäure |
| | J04208 (Human) | |
| | M33934 (Maus) | |
| Xanthin-Guanin-Phosphoribosyltransferase | X00221 (E. coli) | Mycophenolsäure mit limitierendem Xanthin |
| Mutanten-HGPRTase oder Mutanten-Thymidin-Kinase | J00060 (Hamster) | Hypoxanthin, Aminopterin, und Thymidin (HAT) |
| | M13542, K02581 (Human) | |
| | J00423, M68489(Maus) | |
| | M63983 (Ratte) | |
| | M36160 (Herpes virus) | |
| Thymidylat-Synthetase | D00596 (Human) | 5-Fluordeoxyuridin |
| | M13019 (Maus) | |
| | L12138 (Ratte) | |
| P-Glycoprotein 170 (MDR1) | AF016535 (Human) | mehrere Arzneistoffe, z.B. |
| | J03398 (Maus) | Adriamycin, Vincristin, Colchicin |
| Ribonukleotid-Reduktase | M 124223, K02927 (Maus) | Aphidicolin |
| Glutamin-Synthetase | AF 150961 (Hamster) | Methioninsulfoximin (MSX) |
| | U09114, M60803 (Maus) | |
| | M29579 (Ratte) | |
| Asparagin-Synthetase | M27838 (Hamster) | β-Aspartylhydroxamat, |
| | M27396 (Human) | Albizziin, 5'Azacytidin |
| | U38940 (Maus) | |
| | U07202 (Ratte) | |
| Argininosuccinat- Synthetase | X01630 (Human) | Canavanin |
| | M31690 (Maus) | |
| | M26198 (Rind) | |
| Ornithin-Decarboxylase | M34158 (Human) | α-Difluormethylomithin |
| | J03733 (Maus) | |
| | M 16982 (Ratte) | |
| HMG-CoA-Reduktase | L00183,M12705 (Hamster) | Compactin |
| | M11058 (Human) | |
| N-Acetylglucosaminyl-Transferase | M55621 (Human) | Tunicamycin |
| Threonyl-tRNA-Synthetase | M63180 (Human) | Borrelidin |
| Na⁺K⁺-ATPase | J05096 (Human) | Ouabain |
| | M14511 (Ratte) | |

Als amplifrizierbares Selektionsmarkergen wird erfindungsgemäß ein Gen bevorzugt, das für ein Polypeptid mit der Funktion von DHFR kodiert, z.B. für DHFR oder ein Fusionsprotein aus dem fluoreszierenden Protein und DHFR. DHFR ist für die Biosynthese von Purinen erforderlich. Zellen, denen das DHFR-Gen fehlt, können in Purin-defizientem Medium nicht wachsen. Das DHFR-Gen ist deshalb ein nützlicher Selektionsmarker zur Selektion und Amplifikation von Genen in Zellen, die in Purin-freiem Medium kultiviert werden. Das Selektionsmittel, das in Verbindung mit dem DHFR-Gen verwendet wird, ist Methotrexal (MTX).

Säugerzellen, vorzugsweise Mausmyeloma- und Hamsterzellen, sind bevorzugte Wirtszellen für den Einsatz von DHFR als amplifizierbaren Selektionsmarker. Besonders bevorzugt sind die Zelllinien CHO-DUKX (ATCC CRL-9096) und CHO-DG44 (Urlaub et al., 1983), da sie bedingt durch Mutation keine eigene DHFR-Aktivität aufweisen. Um die DHFR-bedingte Amplifikation auch in anderen Zelltypen anwenden zu können, die über eine eigene endogene DHFR-Aktivität verfügen, kann bei der Transfektion ein mutiertes DHFR-Gen verwendet werden, das für ein Protein mit einer reduzierten Sensitivität gegenüber Methotrexat kodiert (Simonson et al., 1983; Wigler et al., 1980; Haber et al., 1982).

Der DHFR-Marker ist bei der Verwendung von DHFR-negativen Grundzellen wie CHO-DG44 oder CHO-DUKX besonders gut zur Selektion und nachfolgenden Amplifikation geeignet, da diese Zellen kein endogenes DHFR exprimieren und somit nicht in Purin-freiem Medium wachsen. Deshalb kann hier das DHFR-Gen als dominanter Selektionsmarker eingesetzt werden und die transformierten Zellen werden in Hypoxanthin/Thymidin-freiem Medium selektioniert.

Die vorliegende Erfindung schließt deshalb Methode zur Herstellung und Selektion von rekombinanten Säugerzellen ein, die folgende Schritte enthält: (a) Transfektion der Wirtszellen mit Genen, die zumindest für ein Protein/Produkt von Interesse, eine Neomycin-Phosphotransferase, vorzugsweise modifiziert, und den amplifzierbaren Selektionsmarker DHFR kodieren, wobei zumindest das Gen (bzw. Gene) von Interesse zur Transkriptions- bzw. Expressionssteigerung funktionell mit zumindest einem erfindungsgemäßen TE-Element verknüpft ist; (b) Kultivierung der Zellen unter Bedingungen, die eine Expression der verschiedenen Gene ermöglichen; (c) die Selektion dieser co-integrierten Gene durch Kultivierung der Zellen in Gegenwart eines Selektionsmittels, wie z.B. G418, in

Hypoxanthin/Thymidin-freiem Medium; und (d) die Amplifikation dieser co-integrierten Gene durch Kultivierung der Zellen in Gegenwart eines Selektionsmittels, das die Amplifikation zumindest des amplifizierbaren Selektionsmarkergens erlaubt, wie z.B. Methotrexat. Bevorzugt werden die transfizierten Zellen hierbei in Hypoxanthin/Thymidin-freiem Medium, supplementiert mit zumindest 200 µg/mL G418, vorzugsweise 400 µg/mL oder auch mehr G418, in der Abwesenheit von Serum und unter Zugabe steigender Konzentrationen an MTX kultiviert. Verzugsweise beträgt die Konzentration an MTX bei dem ersten Amplifikationsschritt zumindest 100 nM. Die Konzentration an MTX kann aber auch zumindest 250 nM betragen und stufenweise auf bis zu 1 µM gesteigert werden. Im Einzelfall können auch Konzentration von über 1 µM verwendet werden, z.B. 2 µM.

Eine Selektion von transfizierten Zellen ist auch durch fluoreszenzaktivierte Zellsortierung (FACS) möglich. Hierzu werden beispielsweise bakterielle β-Galaktosidase, Zelloberflächenmarker oder fluoreszierende Proteine (z.B. grünes fluoreszierendes Protein (GFP) und dessen Varianten von *Aequorea victoria* und *Renilla reniformis* oder anderer Spezies; rote fluoreszierende Protein und in anderen Farben fluoreszierende Proteine und deren Varianten von nicht-biolumineszierenden Organismen wie z.B. *Discosoma sp., Anemonia sp., Clavularia sp., Zoanthus sp., Aequorea coerulescens* zur Selektion von transformierten Zellen eingesetzt.

### Genexpression und Selektion hoch produzierender Wirtszellen:

Der Ausdruck "Genexpression" bezieht sich auf die Transkription und/oder Translation einer heterologen Gensequenz in einer Wirtszelle. Die Expressionsrate kann hierbei allgemein bestimmt werden, entweder auf Basis der Menge der entsprechenden mRNA, die in der Wirtszelle vorhanden ist, oder auf Basis der produzierten Menge an Genprodukt, das von dem Gen von Interesse kodiert wird. Die Menge der durch Transkription einer ausgewählten Nukleotidsequenz erzeugten mRNA kann beispielsweise durch Northern Blot Hybridisierung, Ribonuklease-RNA-Protektion, *in situ* Hybridisierung von zellulärer RNA oder durch PCR-Methoden (z.B. quantitativer PCR) bestimmt werden (Sambrook et al., 1989; Ausubel et al., 1994). Proteine, die von einer ausgewählten Nukleotidsequenz kodiert werden, können ebenfalls durch verschiedene Methoden, wie z.B. durch ELISA, Protein A HPLC, Western Blot, Radioimmunassay, Immunpräzipitation, Nachweis der biologischen Aktivität des Proteins, Immunfärbung des Proteins mit nachfolgender FACS-Analyse oder Fluoreszenzmikroskopie, direkte Detektion eines fluoreszierenden Proteins mittel FACS-Analyse oder durch Fluoreszenzmikroskopie, bestimmt werden (Sambrook et al., 1989; Ausubel et al., 1994). Diese Methoden ermöglichen beispielsweise die Untersuchung, ob das erfindungsgemäße TE-Element der SEQ ID Nr.1 oder ein beliebiges Teilstück, Fragment oder Bereich davon oder deren Derivate oder Kombinationen aus diesen, zu einer Steigerung der Transkription bzw. Expression eines Gens von Interesse führen.

Mit "Expressions,- Transkriptions- oder Produktivitätssteigerung" wird die Steigerung der Expression oder Synthese einer in eine Wirtszelle eingebrachten heterologcn Sequenz, beispielsweise eines für ein therapeutisches Protein kodierenden Gens, im Vergleich zu einer Kontrolle bezeichnet. Eine Expressions-, Transkriptions- oder Produktivitätssteigerung liegt vor, wenn eine erfindungsgemäße Zelle nach einem hier beschriebenen erfindungsgemäßen Verfahren kultiviert wird, und wenn diese Zelle mindestens eine Steigerung der spezifischen Produktivität um das 1,3-fache bzw. 1,5-fache oder bevorzugt eine Verdopplung der spezifischen Produktivität aufweist. Eine Expressions-, Transkriptions- oder Produktivitätssteigerung liegt auch dann vor, wenn die erfindungsgemäße Zelle mindestens eine Verdreifachung der spezifischen Produktivität aufweist. Eine Expressions-, Transkriptions- oder Produktivitätssteigerung liegt insbesondere auch vor, wenn die erfindungsgemäße Zelle mindestens eine Vervierfachung der spezifischen Produktivität aufweist. Eine Expressions-, Transkriptions- oder Produktivitätssteigerung liegt insbesondere dann vor, wenn die erfindungsgemäße Zelle mindestens eine Verfünffachung der spezifischen Produktivität aufweist. Eine Expressions-, Transkriptions- oder Produktivitätssteigerung liegt insbesondere dann vor, wenn die erfindungsgemäße Zelle mindestens eine Versechsfachung der spezifischen Produktivität aufweist. Eine besondere Expressions-, Transkriptions- oder Produktivitätssteigerung liegt dann vor, wenn die erfindungsgemäße Zelle mindestens eine Versiebenfachung der spezifischen Produktivität aufweist.

Eine Expressions-, Transkriptions- oder Produktivitätssteigerung kann sowohl durch Verwendung einer der erfindungsgemäßen Expressionsvektoren als auch durch Anwendung eines der erfindungsgemäßen Verfahren erreicht werden.

Die entsprechenden Verfahren können mit einer durch FACS gestützten Selektion von rekombinanten Wirtszellen, die als weiteren Selektionsmarker beispielsweise ein (oder mehrere) Fluoreszenzprotein(e) (z.B. GFP) oder einen Zelloberflächenmarker enthalten, kombiniert werden. Andere Methoden zur Erzielung einer verstärkten Expression, wobei auch eine Kombination verschiedener Methoden möglich ist, beruhen beispielsweise auf der Verwendung von cis-aktiven Elementen zur Manipulation der Chromatinstruktur (z.B. LCR, UCOE, EASE, Isolatoren, S/MARs, STAR-Elemente), Verwendung von (artifiziellen) Transkriptionsfaktoren, Behandlung der Zellen mit natürlichen oder synthetischen Agenzien zur Hochregulation endogener oder heterologer Genexpression, Verbesserung der Stabilität (Halbwertszeit) der mRNA oder des Proteins, Verbesserung der mRNA-Translationsinitiation, Erhöhung der Gendosis durch Verwendung von episomalen Plasmiden (basierend auf der Verwendung von viralen Sequenzen als Replikationsursprung, z.B. von SV40, Polyoma, Adenovirus, EBV oder BPV), Einsatz von amplifikationsfördernden Sequenzen (Hemann et al., 1994) oder auf DNA-Konkatemeren basierenden *in vitro* Amplifikationssystemen (Monaco et al., 1996).

In einer weiteren Ausführung betrifft die vorliegende Erfindung somit auch Verfahren zur Gewinnung und Selektion von rekombinanten Säugerzellen, die mindestens ein heterologes Gen von Interesse exprimieren und dadurch gekennzeichnet sind, dass (i) rekombinante Säugerzellen mit einem erfindungsgemäßen Expressionsvektor sowie dem Gen für ein amplifizierbares Selektionsmarkergen transfiziert werden; (ii) die Säugerzellen unter Bedingungen kultiviert werden, die eine Expression des (der) Gens (e) von Interesse, des modifizierten Neomycin-Phosphotransferase-Gens und des Gens, das für ein fluoreszierendes Protein kodiert, ermöglicht; (iii) die Säugerzellen in Anwesenheit zumindest eines Selektionsmittels kultiviert werden, der selektiv auf das Wachstum von Säugerzellen wirkt, und solche Zellen im Wachstum bevorzugt, die das Neomycin-Phosphotransferase-Gen exprimieren; (iv) die Säugerzellen mittels flowzytometrischer Analyse aussortiert werden, die eine hohe Expression des fluoreszierenden Proteins zeigen; (v) die aussortierten Zellen unter Bedingungen kultiviert werden, unter denen das amplifizierbare Selektionsmarkergens exprimiert wird; und (vi) dem Kulturmedium ein Selektionsmittel zugegeben wird, das die Amplifikation des amplifizierbaren Selektionsmarkergens zur Folge hat.
Weiterhin erfindungsgemäß ist ein Verfahren; zur Herstellung eines biopharmazeutischen Produktes gekennzeichnet durch folgende Schritte:
(a) Integration einer erfindungsgemäßen Nukleinsäure in einen eukaryontischen Expressionsvektor enthaltend ein Gen von Interesse.
(b) Transfektion einer eukaryontischen Wirtszelle mit diesem Expressionsvektor.
(c) Selektion einer hochproduzierenden transtizierten Wirtszelle und
(d) Kultivierung der erhaltenen hochproduzierenden transfizierten Wirtszelle unter Bedingungen, die eine Expression des (der) Gens (Gene) von Interesse erlauben. Hierzu werden die selektionierten hoch produzierenden Zellen vorzugsweise in einem serumfreien Kulturmedium und vorzugsweise in Suspensionskultur unter Bedingungen gezüchtet, die eine Expression des Gens von Interesse erlauben. Das Protein/Produkt von Interesse wird dabei vorzugsweise als sekretiertes Genprodukt aus dem Zellkulturmedium gewonnen. Bei Expression des Proteins ohne Sekretionssignal kann das Genprodukt aber auch aus Zelllysaten isoliert werden. Um ein reines, homogenes Produkt zu erhalten, das im Wesentlichen frei ist von anderen rekombinanten Proteinen und Wirtszellproteinen, werden übliche Reinigungsschritte durchgeführt. Hierzu entfernt man häufig zunächst Zellen und Zelltrümmer aus dem Kulturmedium oder Lysat. Das gewünschte Genprodukt kann dann von kontaminierenden löslichen Proteinen, Polypeptiden und Nukleinsäuren befreit werden, z.B. durch Fraktionierung an Immunaffinitäts- und Ionenaustauschsäulen, Ethanolfällung, Umkehrphasen-HPLC oder Chromatographie an Sephadex, Silica oder Kationenaustauscherharzen wie DEAE. Methoden, die zur Aufreinigung eines von rekombinanten Wirtszellen exprimierten heterologen Proteins führen, sind dem Fachmann bekannt und sind in der Literatur beschrieben, z.B. bei Harris et al. (1995) und Scopes (1988).

### ERFINDUNGSGEMÄSSE ZUSAMMENSETZUNGEN

Die vorliegende Erfindung betrifft eine Nukleinsäure, welche TE-13 (SEQ ID Nr. 15) enthält oder ein Fragment von TE-13 (SEQ ID Nr. 15) oder deren komplementäre Nukleotidsequenzen oder ein Derivat von TE-13 (SEQ ID Nr. 15) oder deren komplementäre Nukleotidsequenzen, wobei die Nukleinsäure, das Fragment. das Derivat oder deren komplementäre Nukleotidsequenzen bei chromosomaler Integration zu einer Steigerung der Transkription bzw. Expression eines Gens von Interesse in einem Expressionssystem führt. und wobei das Derivat mindestens 85% Sequenzidentität aufweist.

Die vorliegende Erfindung betrifft eine Nukleinsäure, welche TE-13 (SEQ ID Nr. 15) enthält oder ein Fragment von TE-13 (SEQ ID Nr. 15) oder deren komplementäre Nukleotidsequenzen oder ein Derivat von TE-13 (SEQ ID Nr. 15) oder deren komplementäre Nukleotidsequenzen, wobei die Nukleinsäure, das Fragment. das Derivat oder deren komplementäre Nukleotidsepenzen bei chromosomaler Integration zu einer Steigerung der Transkription bzw. Expression eines Gens von Interesse in einem Expressionssystem führt. und wobei das Derivat mindestens 85% Sequenzidentität aufweist, mit der Maßgabe, dass das Fragment bzw. Derivat mindestens einen Sequenzbereich aus dem Nukleinsäurebereich zwischen 1bp und 1578bp (in Bezug auf SEQ ID Nr. 01) mit umfasst. Hierbei ist mit einem Sequenzbereich ein Nukleinsäurebereich von mindestens 10bp, 15bp, 20bp, 50bp, 100bp gemeint.
Die vorliegende Erfindung betrifft eine Nukleinsäure, welche TE-13 (SEQ ID Nr. 15) enthält oder ein Fragment von TE-13 (SEQ ID Nr. 15) oder deren komplementäre Nukleotidsequenzen oder ein Derivat von TE-13 (SEQ ID Nr. 15) oder deren komplementäre Nukleotidsequenzen, wobei die Nukleinsäure. das Fragment, das Derivat oder deren komplementäre Nukleotidsequenzen bei chromosomaler Integration zu einer Steigerung der Transkription bzw. Expression eines Gens von Interesse in einem Expressionssystem führt, und wobei das Derivat mindestens 85% Sequenzidentität aufweist, mit der Maßgabe, dass das Fragment bzw. Derivat mindestens einen Sequenzbereich von TE-08 (SEQ ID Nr. 10) oder TE-13 (SEQ ID Nr. 15) mit umfasst. Auch hierbei ist mit einem Sequenzbereich ein Nukleinsäurebereich von mindestens 10bp, 15bp, 20bp, 50bp, 100bp gemeint.

Die Expressionssteigerung des Gens von Interessse ist beispielsweise durch Messung des Produkttiters mittels ELISA messbar.
Die Erfindung betrifft in einer bevorzugten Ausführungsform eine Nukleinsäure, welche TE-08 (SEQ ID Nr. 10) enthält oder ein Fragment von TE-08 (SEQ ID Nr. 10) oder deren komplementäre Nukleotidsequenzen oder ein Derivat von TE-08 (SEQ ID Nr. 10) oder deren komplementäre Nukleotidsequenzen, wobei die Nukleinsäure, das Fragment, das Derivat oder deren komplementäre Nukleotidsequenzen bei chromosomaler Integration zu einer Steigerung der Transkription bzw. Expression eines Gens von Interesse in einem Expressionssystem führt, und wobei das Derivat mindestens 85% Sequenzidentität aufweist.
In einer bevorzugten Ausführungsform der obigen erfindungsgemäßen Nukleinsäure besteht die Maßgabe, dass das Fragment bzw. Derivat mindestens einen Sequenzbereich aus dem Nukleinsäurebereich zwischen 1bp und 1578bp (in Bezug auf SEQ ID Nr. 01) mit umfasst.
In einer besonders bevorzugten Ausführungsform der obigen erfindungsgemäßen Nukleinsäure besteht die Maßgabe, dass das Fragment bzw. Derivat mindestens einen Sequenzbereich von TE-08 (SEQ ID Nr. 10) oder TE-13 (SEQ ID Nr. 15) mit umfasst. Mit einem Sequenzbereich ist hierbei ein Nukleinsäurebereich von mindestens 10bp, 15bp, 20bp, 50bp, 100bp gemeint.

Die Erfindung betrifft in einer weiteren Ausführungsform eine Nukleinsäure, welche die SEQ ID Nr. 1 enthält oder ein Fragment von SEQ ID Nr. 1 oder deren komplementäre Nukleotidsequenzen oder ein Derivat von SEQ ID Nr. 1 oder deren komplementäre Nukleotidsequenzen, wobei die Nukleinsäure, das Fragment, das Derivat oder deren komplementäre Nukleotidsequenzen bei chromosomaler Integration zu einer Steigerung der Transkription bzw. Expression eines Gens von Interesse in einem Expressionssystem führt, wobei das Fragment bzw. Derivat mindestens einen Sequenzbereich aus dem Nukleinsäurebereich zwischen 1bp und 1578bp (in Bezug auf SEQ ID Nr. 01) mit umfasst, und wobei das Derivat mindestens 85% Sequenzidentität aufweist.
In einer besonders bevorzugten Ausführungsform der genannten erfindungsgemäßen Nukleinsäure besteht die Maßgabe, dass das Fragment bzw. Derivat mindestens einen Sequenzbereich von TE-08 (SEQ ID Nr. 10) oder TE-13 (SEQ ID Nr. 15) mit umfasst. Mit einem Sequenzbereich ist hierbei ein Nukleinsäurebereich von mindestens 10bp, 15bp, 20bp, 50bp, 100bp gemeint.

Die Expressionssteigerung des Gens von Interessse ist beispielsweise durch Messung des Produkttiters mittels ELISA messbar.

In einer speziellen Ausführungsform weist die erfindungsgemäße Nukleinsäure eine Länge von mindestens 511 bp (=Länge TE-13, SEQ ID Nr. 15) bzw. mindestens 1015 bp (=Länge TE-08, SEQ ID Nr. 10) auf.

Die vorliegende Erfindung betrifft in einer bevorzugten Ausführungsform das Fragment 5' des TE-Elements TE-00 (SEQ ID Nr. 2). Dies entspricht dem Teilbereich von SEQ ID Nr. 1 zwischen 1 bp und 1578bp oder dessen komplementärer Nukleotidsequenz.

Die vorliegende Erfindung betrifft, insbesondere eine Nukleinsäure bzw. ein transkriptionssteigerndes bzw. expressionssteigerndes Nukleinsäure Element (TE-Element), welches TE-13 (SEQ ID Nr. 15) bzw. TE-08 (SEQ ID Nr. 10) enthält oder ein Derivat davon oder deren komplementäre Nukleotidsequenzen, wobei die Nukleinsäure, das Derivat oder deren komplementäre Nukleotidsequenzen bei chromosomaler Integration zu einer Steigerung der Transkription bzw. Expression eines Gens von Interesse führt, und wobei das Derivat mindestens 85% Sequenzidentität aufweist.

Die vorliegende Erfindung betrifft bevorzugt eine isolierte Nukleinsäure bzw. ein isoliertes Nukleinsäuremolekül bzw. eine isolierte Nukleinsäuresequenz bzw. ein isoliertes transskriptionssteigerndes Nukleinsäure Element bzw. ein isoliertes TE-Element.

Die vorliegende Erfindung betrifft insbesondere eine isolierte Nukleinsäure welche TE-08 (SEQ ID Nr. 10) oder dessen komplementäre Nukleotidsequenz enthält und die bei chromsomaler Integration zu einer Steigerung der Transkription bzw. Expression eines Gens von Interesse in einem Expressionssystem führt.

In einer Ausführungsform enthält die Nukleinsäure bzw. das transkriptionssteigernde Nukleinsäure Element bzw. die isolierte Nukleinsäure ein Derivat eines TE-Elements bzw. von SEQ ID Nr. 1, welches mindestens ca. 85% Sequenzidentität, besonders bevorzugt mindestens ca. 90% Sequenzidentität und am bevorzugtesten mindestens ca. 95% Sequenzidentität mit dem korrespondierenden Teilbereich der TE-Element Sequenz bzw. dessen komplementärer Sequenz aufweist, insbesondere mit dem Sequenzbereich zwischen Nukleotidposition 1bp und 1578bp bezüglich SEQ ID Nr. 1, was dem Sequenzbereich 5' der TE-00 Sequenz entspricht, und besonders bevorzugt mit TE-13 (SEQ ID Nr. 15) bzw. TE-08 (SEQ ID Nr. 10).

In einer bevorzugten Ausführungsform enthält die Nukleinsäure bzw. das transkriptionssteigernde Nukleinsäure Element bzw. die isolierte Nukleinsäure ein Derivat einer TE-08 Nukleinsäure (SEQ ID Nr. 10) oder bevorzugt einer TE-13 Nukleinsäure (SEQ ID Nr. 15), welches mindestens ca. 85% Sequenzidentität, besonders bevorzugt mindestens ca. 90% Sequenzidentität und am bevorzugtesten mindestens ca. 95% Sequenzidentität mit dem korrespondierenden Teilbereich der TE-Element Sequenz bzw. dessen komplementärer Sequenz aufweist.

In einer weiteren Ausführungsform betrifft die Erfindung eine erfindungsgemäße Nukleinsäure bzw. ein transkriptionssteigerndes ertindungsgemäßes Nukleinsäure Element bzw. eine isolierte erfindungsgemäße Nukleinsäure bzw. ein erfindungsgemäßes Derivat eines TE-Elementes, welche(s) mit der Sequenz eines TE-Elementes oder mit der komplementären Sequenz eines TE-Elementes hybridisiert, insbesondere mit dem Sequenzbereich zwischen Nukleotidposition 1bp und 1578bp bezüglich SEQ ID Nr. 1, was dem Sequenzbereich 5' der TE-00 Sequenz (SEQ ID Nr. 2) entspricht, oder insbesondere mit dem TE-08 Element (SEQ ID Nr. 10). Vorzugsweise erfolgt die Hybridisierung unter stringenten Hybridisierungs- und Waschbedingungen.

In einer weiteren bevorzugten Ausführungsform ist die erfindungsgemäße Nukleinsäure bzw. das transkriptionssteigernde Element (TE-Element) ausgewählt aus der Gruppe bestehend aus: TE-01 (SEQ ID Nr. 3), TE-02 (SEQ ID Nr. 4), Te-07 (SEQ ID Nr. 9), TE-08 (SEQ ID Nr. 10), TE-10 (SEQ ID Nr. 12), TE-11 (SEQ ID Nr. 13), TE-12 (SEQ ID Nr. 14), TE-13 (SEQ ID Nr. 15), TE-15 (SEQ ID Nr. 17), TE-17(SEQ ID Nr. 19), TE-18 (SEQ ID Nr. 20) .

In einer besonders bevorzugten Ausführungsform ist die Nukleinsäure bzw. das transkriptionssteigernde Element (TE-Element) TE-08 (SEQ ID Nr. 10).

In einer bevorzugten Ausführungsform ist die Nukleinsäure bzw. das transkriptionssteigernde Element (TE-Element) TE-18 (SEQ ID Nr. 20).

In einer bevorzugten Ausführungsform ist die erfindungsgemäße Nukleinsäure TE-13 (SEQ ID Nr. 15).

In einer weiteren Ausführungsform ist die erfindungsgemäße Nukleinsäure oder das Fragment oder das Derivat eine isolierte Nukleinsäure.

Die vorliegende Erfindung betrifft außerdem eine Nukleinsäure, die dadurch gekennzeichnet ist, dass eine Nukleinsäure enthaltend eine erfindungsgemäße Nukleinsäure bzw. ein erfindungsgemäßes TE-Element mit einer heterologen Sequenz verknüpft ist.

Die mit einer heterologen Gensequenz verknüpfte Nukleinsäure kann in einer bevorzugten Ausführungsform ein Expressionvektor sein. Beispiele sind Plasmide, Bakteriophagen, Phagemide, Cosmide, virale Vektoren oder auch speziell ein Targeting-Vektor.

Die mit einer heterologen Gensequenz verknüpfte Nukleinsäure kann aber auch jedes andere künstliche Nukleinsäuremolekül sein wie z.B. künstliche, artifizielle oder Mini-Chromosomen.

Die vorliegende Erfindung betrifft weiterhin einen eukaryontischen Expressionsvektor dadurch gekennzeichnet, dass dieser Expressionsvektor eine oder mehrere erfindungsgemäße Nukleinsäure bzw. ein oder mehrere erfindungsgemäße transkriptionssteigernde Elemente (TE-Element) enthält.

In einer speziellen Ausfühmngsform ist der eukaryontische Expressionsvektor dadurch gekennzeichnet, dass er einen Promotor und / oder ein heterologes Gen von Interesse und /oder einen Selektionsmarker und / oder optional einen Enhancer enthält.

Die vorliegende Erfindung betrifft weiterhin einen eukaryontischen Expressionsvektor dadurch gekennzeichnet, dass dieser Expressionsvektor eine oder mehrere erfindungsgemäße Nukleinsäure bzw. ein oder mehrere erfindungsgemäße transkriplionssteigernde Elemente (TE-Element) und einen Promotor und / oder ein heterologes Gen von Interesse und / oder einen Selektionsmarker und / oder optional einen Enhancer enthält.

In einer weiteren Ausführungsform ist der eukaryontische Expressionsvektor dadurch gekennzeichnet, dass es sich um einen Targeting Vektor zur gezielten Integration des Gens von Interesse handelt.

In einer bevorzugten Ausführungsform ist der eukaryontische Expressionsvektor dadurch gekennzeichnet, dass der Promotor ein heterologer Promotor ist wie der early Promotor des humanen Cytomegalie-Virus (CMV-Promotor), SV40 early Promotor, der Adenovirus major late Promotor, der Maus Metallothionin-I Promotor, die lange terminale Repeatregion des Rous Sarcoma Virus, Aktin-, Immunglobulin- oder Hitzeschockpromotor(en), bevorzugt der CMV-Promotor.

In einer weiteren bevorzugten Ausführungsform ist der eukaryontische Expressionsvektor dadurch gekennzeichnet, dass der Promotor ein heterologer Promotor ist, bevorzugt der Ubiquitin/S27a-Promotor, besonders bevorzugt der Hamster Ubiquitin/S27a-Promotor.

In einer speziellen Ausführungsform ist der eukaryontische Expressionsvektor dadurch gekennzeichnet, dass er eine Kombination mehrerer gleicher oder verschiedener erfindungsgemäßer Nukleinsäuren bzw. TE-Elemente in beliebiger Orientierung zueinander enthält, wobei ein oder mehrere Nukleinsäuren bzw. TE-Elemente vor (d.h. 5' von) und / oder ein oder mehrere Nukleinsäuren bzw. TE-Elemente nach (d.h. 3' von) dem Gen von Interesse positioniert sind.

In einer bevorzugten Ausführungsform ist der eukaryontische Expressionsvektor dadurch gekennzeichnet, dass es sich bei den kombinierten Nukleinsäuren bzw. TE-Elementen um eine Kombination mit TE-08 (SEQ ID Nr. 10) handelt.

In einer besonders bevorzugten Ausführungsform ist der eukaryontische Expressionsvektor dadurch gekennzeichnet, dass ein oder mehrere TE-08-Nukleinsäure(n) bzw. Element(e) (SEQ ID Nr. 10) vor (d.h. 5' von) und eines oder mehrere nach (d.h. 3' von) dem Gen von Interesse positioniert sind, bevorzugt ein TE-08-Element (SEQ ID Nr. 10) davor und eines danach (siehe hierzu auch Abbildung 13).
Weitere bevorzugte Kombinationen von TE-Nukleinsäuren bzw. Elementen sind 2 TE-08 Nukleinsäuren /Elemente (SEQ ID Nr. 10) vor und / oder nach dem Gen von Interesse.

In einer bevorzugten Ausführungsform ist der eukaryontische Expressionsvektor dadurch gekennzeichnet, dass eine Kombination von einem oder mehreren TE-08-Nukleinsäure(n) bzw. Element(en) (SEQ ID Nr. 10) mit einem oder mehreren TE-06 Nukleinsäuren bzw. Element(en) (SEQ ID Nr. 8) vor (d.h. 5' von) und / oder nach (d.h. 3' von) dem Gen von Interesse positioniert sind, bevorzugt ist eine Kombination von einem TE-08 Nukleinsäure bzw. Element (SEQ ID Nr. 10) gefolgt von einem TE-06-Nukleinsäure bzw. Element (SEQ ID Nr. 8) vor (d.h. 5' von) dem Gen von Interesse. (siehe hierzu auch Abbildung 13).

In einer weiteren Ausführungsform ist der eukaryontische Expressionsvektor dadurch gekennzeichnet, dass ein oder mehrere TE-13-Nukleinsäuren bzw. Elemente (SEQ ID Nr. 15) vor (5' von) und / oder nach (3' von) dem Gen von Interesse positioniert sind.

In einer bevorzugten Ausführungsform ist der eukaryontische Expressionsvektor dadurch gekennzeichnet, dass er zusätzlich eine Integrase enthält.

In einer weiteren bevorzugten Ausführungsform werden die Wirtszellen mit zumindest zwei eukaryontischen Expressionsvektoren co-transfiziert, wobei zumindest der eine der beiden Vektoren zumindest ein Gen), das zumindest Protein von Interesse kodiert, enthält und der andere Vektor ein oder mehrere erfindungsgemäße Nukleinsäuren in beliebiger Kombination, Position und Orientierung enthält, und optional auch für zumindest ein Gen (von Interesse kodiert, und diese erfindungsgemäßen Nukleinsäuren ihre transkriptions- bzw. expressionssteigemde Wirkung auf die Gene von Interesse, die auf dem anderen co-transfizierten Vektor lokalisiert sind, via Co-Integration mit dem anderen Vektor vermitteln.

In einer speziellen Ausführungsform ist der eukaryontische Expressionsvektor dadurch gekennzeichnet, dass der Selektionsmarker DHFR oder Neo, beispielsweise Neo F240I, ist.

Die Erfindung betrifft weiterhin ein Verfahren zur Herstellung eines eukaryontischen Expressionsvektors gekennzeichnet durch die Integration einer erfindungsgemäßen Nukleinsäure in einen Expressionsvektor.

Die Erfindung betrifft weiterhin eine eukaryontische Wirtszelle dadurch gekennzeichnet, dass sie einen erfindungsgemäßen eukaryontischen Expressionsvektor enthält.

In einer speziellen Ausführungsform ist die eukaryontische Wirtszelle dadurch gekennzeichnet, dass sie hochproduzierend ist, d.h. eine höhere spezifische Produktivität besitzt als eine vergleichbare eukaryontische Wirtszelle ohne TE-Element bzw. erfindungsgemäße Nukleinsäure, wobei diese Wirtszelle einen bis zu zweifach, dreifach, vierfach, fünffach, sechsfach, siebenfach oder zehnfach gesteigerten oder einen mehr als zweifach, mehr als dreifach, mehr als vierfach, mehr als fünffach, mehr als siebenfach, mehr als zehnfach gesteigerten Expressionslevel hat, bevorzugt bis zu fünffach oder mehr als dreifach.

In einer besonders bevorzugten Ausführungsform ist die eukaryontische Wirtszelle dadurch gekennzeichnet, dass der Expressionsvektor stabil in das Genom integriert ist.

In einer weiteren Ausführungsform ist die eukaryontische Wirtszelle eine Hamster- oder Mauszelle wie beispielsweise eine CHO-, NS0-, Sp2/0-Ag14-, BHK21-, BHK TK⁻' HaK-, 2254-62.2 (BHK-21-Derivat)-, CHO-K1-, CHO-DUKX (= CHO duk⁻, CHO/dhfr⁻), CHO-DUKX B1-, CHO-DG44-, CHO Pro-5-, V79-, B14AF28-G3-, CHL-Zelle, bevorzugt eine CHO-Zelle und besonders bevorzugt eine CHO-DG44 Zelle.

In einer weiteren Ausführungsform ist die eukaryontische Wirtszelle eine Säugerzelle, einschließlich aber nicht beschränkt auf Zelllinien von Mensch, Maus, Ratte Affen, Nagetieren.

In einer weiteren Ausführungsform ist die Wirtszelle eine eukaryontische Zelle einschließlich aber nicht beschränkt auf Hefe-, Insekten-, Vogel- und Pflanzenzellen.

In einer weiteren speziellen Ausführungsform ist die eukaryontische Wirtszelle dadurch gekennzeichnet, dass sie zusätzlich ein anti-Apoptose-Gen wie BCL-xL, BCL-2, BCL-w, BFL-1, A1, MCL-1, BOO, BRAG-1, NR-13, CDN-1, CDN-2, CDN-3, BHRF-1, LMW5-HL or CED-9 enthält., bevorzugt Bcl-xL oder BCL-2, besonders bevorzugt BCL-xL.

Die vorliegende Erfindung betrifft weiterhin ein Verfahren zur Entwicklung einer hoch produzierenden stabil transfizierten eukaryontischen Wirtszelllinie gekennzeichnet durch folgende Schritte:
(a) Integration zumindest einer erfindungsgemäßen Nukleinsäure bzw. eines erfindungsgemäßen TE-Elementes in einen eukaryontischen Expressionsvektor enthaltend ein Gen von Interesse,
(b) Transfektion einer eukaryontischen Wirtszelle mit diesem Expressionsvektor,
(c) Selektion einer hochproduzierenden transfizierten Wirtszelle.

Die vorliegende Erfindung betrifft weiterhin ein Verfahren zur Entwicklung einer hoch produzierenden stabil transfizierten eukaryontischen Wirtszelllinie gekennzeichnet durch folgende Schritte:
(a) Integration eines Gens (Gene) von Interesse in einen eukaryontischen Expressionsvektor enthaltend zumindest eine erfindungsgemäße Nukleinsäure bzw. ein erfindungsgemäßes TE-Element
(b) Transfektion einer eukaryontischen Wirtszelle mit diesem Expressionsvektor,
(c) Selektion einer hochproduzierenden transfizierten Wirtszelle.

In einer speziellen Ausführungsform ist das Verfahren gekennzeichnet durch zumindest einen zusätzlichen Amplifikationsschritt.

Die vorliegende Erfindung betrifft zudem ein Verfahren zur Herstellung und Selektion von rekombinanten Säugerzellen gekennzeichnet durch folgende Schritte:
(a) Transfektion der Wirtszellen mit Genen, die zumindest für ein Protein/Produkt von Interesse, eine Neomycin-Phosphotransferase, vorzugsweise modifiziert, und den amplifzierbaren Selektionsmarker DHFR kodieren, wobei zumindest das Gen (bzw. Gene) von Interesse zur Transkriptions- bzw. Expressionssteigerung funktionell mit zumindest einer erfindungsgemäßen Nukleinsäure verknüpft ist,
(b) Kultivierung der Zellen unter Bedingungen, die eine Expression der verschiedenen Gene ermöglichen,
(c) die Selektion dieser co-integrierten Gene durch Kultivierung der Zellen in Gegenwart eines Selektionsmittels, wie z.B. G418, in Hypoxanthin/Thymidin-freiem Medium und
(d) die Amplifikation dieser co-integrierten Gene durch Kultivierung der Zellen in Gegenwart eines Selektionsmittels, das die Amplifikation zumindest des amplifizierbaren Selektionsmarkergens erlaubt, wie z.B. Methotrexat.

In einer besonderen Ausführungsform ist dieses Verfahren dadurch gekennzeichnet, dass die transfizierten Zellen in Hypoxanthin/Thymidin-freiem Medium, supplementiert mit zumindest 200 µg/mL G418, vorzugsweise 400 µg/mL oder auch mehr G418, in der Abwesenheit von Serum und unter Zugabe steigender Konzentrationen an MTX kultiviert werden.

In einer weiteren besonderen Ausführungsform ist dieses Verfahren dadurch gekennzeichnet, dass die Konzentration an MTX bei dem ersten Amplifikationsschritt zumindest 100 nM oder zumindest 250 nM beträgt und stufenweise auf bis zu 1 µM oder darüber gesteigert wird. Im Einzellfall kann die MTX Konzentration 2µM betragen.

In einer weiteren speziellen Ausführungsform ist das Verfahren gekennzeichnet durch einen zusätzlichen Klonierungsschritt.

In einer weiteren Ausführungsform des erfindungsgemäßen Verfahrens ist die Wirtszelle eine Nagetier-/Hamsterzelle wie beispielsweise eine CHO-, NS0-, Sp2/0-Ag14-, BHK21-, BHK TK⁻' HaK-, 2254-62.2 (BHK-21-Derivat)-, CHO-K1-, CHO-DUKX (= CHO duk⁻', CHO/dhfr⁻), CHO-DUKX B1-, CHO-DG44-, CHO Pro-5-, V79-, B14AF28-G3-, CHL-Zelle, bevorzugt eine CHO-Zelle und besonders bevorzugt eine CHO-DG44 Zelle.

In einem bevorzugten erfindungsgemäßen Verfahren enthält der Expressionsvektor einen Selektionsmarker wie DHFR oder NPT, beispielsweise NPT F240I oder NPT D227G.

In einer besonders bevorzugten Ausführungsform des erfindungsgemäßen Verfahrens ist der Anteil an Hochproduzenten bis zu zweifach, dreifach, vierfach, fünffach, sechsfach, siebenfach oder zehnfach gesteigerte oder mehr als zweifach, mehr als dreifach, mehr als vierfach, mehr als fünffach, mehr als siebenfach, mehr als zehnfach gesteigert, bevorzugt bis zu fünffach oder mehr als dreifach.

Die vorliegende Erfindung betrifft weiterhin ein Verfahren zur Herstellung eines biopharmazeutischen Produktes gekennzeichnet durch folgende Schritte:
(a) Integration zumindest einer erfindungsgemäßen Nukleinsäure bzw. eines erfindungsgemäßen TE-Elementes in einen eukaryontischen Expressionsvektor enthaltend ein Gen von Interesse,
(b) Transfektion einer eukaryontischen Wirtszelle mit diesem Expressionsvektor,
(c) Selektion einer hochproduzierenden transfizierten Wirtszelle und
(d) Kultivierung der erhaltenen hochproduzierenden transfizierten Wirtszelle unter Bedingungen, die eine Expression des (der) Gens (Gene) von Interesse erlauben.

Die vorliegende Erfindung betrifft weiterhin ein Verfahren zur Entwicklung einer hoch produzierenden stabil transfizierten eukaryontischen Wirtszelllinie gekennzeichnet durch folgende Schritte:
(a) Integration eines Gens (Gene) von Interesse in einen eukaryontischen Expressionsvektor enthaltend zumindest eine erfindungsgemäße Nukleinsäure bzw. ein erfindungsgemäßes TE-Element
(b) Transfektion einer eukaryontischen Wirtszelle mit diesem Expressionsvektor,
(c) Selektion einer hochproduzierenden transfizierten Wirtszelle und
(d) Kultivierung der erhaltenen hochproduzierenden transfizierten Wirtszelle unter Bedingungen, die eine Expression des (der) Gens (Gene) von Interesse erlauben.

In einer speziellen Ausführungsform ist das erfindungsgemäße Verfahren gekennzeichnet durch zumindest einen zusätzlichen Amplifikationsschritt.

In einer speziellen Ausführungsform ist das erfindungsgemäße Verfahren gekennzeichnet durch folgenden zusätzlichen Schritt:
(e) Ernte und Aufreinigung des Proteins von Interesse.

Die vorliegende Erfindung betrifft weiterhin die Verwendung einer erfindungsgemäßen Nukleinsäure bzw. eines erfindungsgemäßen transkriptionssteigernden Elements (TE-Element) in einem eukaryontischen Expressionsvektor, zur Steigerung der Transkription bzw. Expression eines Gens von Interesse in einem Expressionssystem in einer eukaryontischen Wirtszelle oder zur Herstellung eines biopharmazeutischen Produktes.

Die vorliegende Erfindung betrifft ausserdem die Verwendung einer erfindungsgemäßen Nukleinsäure bzw. eines erfindungsgemäßen transkriptionssteigernden Elements (TE-Element) zur Erzeugung von transgenen Tieren oder Pflanzen.

Die vorliegende Erfindung betrifft weiterhin die Verwendung einer erfindungsgemäßen Nukleinsäure bzw. eines erfindungsgemäßen transkriptionssteigernden Elements (TE-Element) in der Gentherapie.

Die vorliegende Erfindung betrifft insbesondere die Verwendung einer erfindungsgemäßen Nukleinsäure bzw. eines erfindungsgemäßen transkriptionssteigernden Elements (TE-Element) als Arzneimittel oder in einer pharmazeutischen Zusammensetzung.

Die vorliegende Erfindung betrifft weiterhin einen Kit bestehend aus einer erfindungsgemäßen Nukleinsäure bzw. (einem) erfindungsgemäßen TE-Element(en), optional Expressionsvektor(en), optional Wirtszelle(n) und optional Transfektionsreagenz(ien).

Eine weitere Ausführungsform der vorliegenden Erfindung bezieht sich auf ein erfindungsgemäßes TE-Element, Fragment oder Derivat, welches über 160 bp, bevorzugt über 170 bp lang ist. In einer speziellen Ausführungsforin ist das TE-Element Fragment zwischen 160bp und 1,200 bp bzw. zwischen 170 bp und 1000 bp, bevorzugt über 200 bp und zwischen 200 bp und 1000 bp lang.

In einer bevorzugten Ausführungsform liegt das erfindungsgemäße TE-Element Fragment im Teilbereich von SEQ ID Nr. 1 zwischen 1 bp und 1578bp (dies entspricht einem Fragment 5' des Elements TE-00 (SEQ ID Nr. 2) und ist über 113 bp lang bzw. über 132 bp und bevorzugt über 160 bp bzw. über 170 bp lang. In einer speziellen Ausführungsform ist das erfindunsgsgermäße TE-Element Fragment zwischen 113 bp und 1,200 bp bzw. zwischen 132 bp und 1,200 bp bzw. zwischen 160 bp und 1,200 bp, bevorzugt über 200 bp und zwischen 200 bp und 1000 b lang.

In einer weiteren Ausführungsform liegt das erfindungsgemäße TE-Element Fragment ohne benachbarte Sequenzen vor. Damit ist gemeint, dass das Fragment nicht Bestandteil einer grösseren Sequenz oder eines Sequenzbereiches ist, dass beispielsweise keine anderen Sequenzen vor (5') oder nach (3') angehängt sind.

In einer weiteren speziellen Ausführungsform betrifft die vorliegende Erfindung eine erfindungsgemäße Nukleinsäure, welche keine CpG-Inseln enthält.

Aus den nachfolgenden Experimenten wird ersichtlich, dass bei einem Vergleich von eukaryontischen Wirtszellen mit und ohne TE-Element eine bis zu siebenfache Steigerung der relativen Änderung der spezifischen Produktivität des Gens von Interesse gezeigt werden konnte.

Die Erhebung von Daten zum Produkttiter und zur spezifischer Produktivität ergab, dass fast alle Zellpools mit erfindungsgemäßen TE-Elementen, die Fragmenten bzw. Derivaten der SEQ ID Nr.1 darstellten, im Durchschnitt mehr Gen von Interesse exprimierten als Zellpools ohne TE-Element. Die TE-Elemente 01 (SEQ ID Nr. 3), 02 (SEQ ID Nr. 4) und 08 (SEQ ID Nr. 10) ergaben in zwei unabhängige Transfektionsserien, in denen jeweils ein anderer Selektionsmarker verwendet wurde (NPT bzw. DHFR) die höchste Produktivität. Sie sind in der Lage, die Produktivität um den Faktor 4 - 7 zu steigern. Allerdings sind die TE-Elemente 01 (SEQ ID Nr. 3) und 02 (SEQ ID Nr. 4) mit 3 kb und 2,5 kb sehr groß zum zusätzlichen Bestücken eines Expressionsvektors. Interessanter ist dagegen das nur 1 kb große TE-Element 08 (SEQ ID Nr. 10), das in der Lage ist, die Expression um den Faktor 5 - 6 zu steigern. Es ist von großem Vorteil, die Expressionsvektoren so klein wie möglich zu belassen, denn kleinere Vektoren sind in der Regel stabiler und sind sowohl bei der Klonierung als auch bei der Transfektion einfacher handhabbar. Daher sind die TE-Elemente 8 (SEQ ID Nr. 10) und 13 (SEQ ID Nr. 15) besonders interessant für den Einsatz als transkriptionsförderndes Element.

Aus den nachfolgenden Beispielen wird weiterhin ersichtlich, dass die Zellpools, die das TE-Element 07 (SEQ ID Nr. 9) enthielten, eine etwa 3- bis 3,5-fache Expression des Gens von Interesse und Zellpools mit den TE-Elementen 10 (SEQ ID Nr. 12), 11 (SEQ ID Nr. 13) bzw. 12 (SEQ ID Nr. 14) eine etwa doppelte Expression des Gens von Interesse zeigten

Das TE-Element 08 (SEQ ID Nr. 10) zeigte in Verbindung mit NPT F240I als Selektionsmarker mit Faktor 5 die größte Steigerung der spezifischen Produktivität des Gens von Intereesse gegenüber den Kontrollpools ohne TE-Element. In Verbindung mit DHFR als Selektionsmarker zeigt das TE-Element 08 (SEQ ID Nr. 10) eine noch bessere Steigerung um ca. Faktor 6,0.

Weiterhin verursacht das TE-Element 02 (SEQ ID Nr. 4) in der Versuchsreihe mit DHFR als Selektionsmarker ein Steigerung der Produktivität des Gens von Interesse um den Faktor 6,8.

Die größte Steigerung (15-fach) verursacht das Element 13.

Aus den nachfolgenden Beispielen wird weiterhin ersichtlich, dass Pools mit den TE-Elementen 05 (SEQ ID Nr. 7) und 09 (SEQ ID Nr. 11) in einer Versuchsreihe keine Steigerung der Expression des Gens von Interesse und in einer Versuchsreihe sogar eine geringere Expression des Gens von Interesse als die Kontrollpools aufwiesen. Diese beiden Elemente sowie möglicherweise Teilfragmente in diesen Sequenzbereichen können also unter Umständen eine reprimierende Wirkung ausüben, müssen dies aber nicht.

Ausserdem konnten in den nachfolgenden Beispielen die relativen Änderungen der spezifischen Produktivität für die verschiedenen getesteten TE-Elemente weitgehend unabhängig vom Vektorsystem erzielt werden, d.h. unabhängig vom eingesetzten Selektionsmarker. oder unabhängig vom jeweiligen Gen von Interesse.

Aus den nachfolgenden Beispielen wird weiterhin ersichtlich, dass die Änderung der Expression des Markergens korreliert mit der Änderungen der Expression des Gens von Interesse.

Aus den nachfolgenden Beispielen wird darüber hinaus ersichtlich, dass keines der getesteten TE-Elemente eine Enhancerwirkung ausübt. Damit ist klar, dass die TE-Elemente nur auf chromosomaler Ebene eine Steigerung der Expression des Gens von Interesse hervorrufen.

Die nachfolgenden Beispiele machen zudem deutlich, dass die Kombination bzw. Hintereinanderschaltung mehrerer entweder gleicher oder verschiedener kurzer TE-Elemente wie z.B.TE-Element 06, zu einem zusätzlichen expressionssteigemden Effekt führen kann.

### BEISPIELE

### ABKÜRZUNGEN

- AP:: alkalische Phosphatase
- Asp (=D):: Asparaginsäure
- bp:: Basenpaar
- CHO:: Chinese Hamster Ovary
- DHFR:: Dihydrofolat-Reduktase
- ELISA:: enzyme-linked immunosorbant assay
- FACS:: fluorescence-activated cell sorter
- GFP:: grünes fluoreszierendes Protein
- Gly (=G):: Glycin
- HT:: Hypoxanthin/Thymidin
- IgG:: Immunglobulin G
- Ile (=I):: Isoleucin
- IRES:: internal ribosomal entry site
- kb:: Kilobase
- mAk:: monoklonaler Antikörper
- MCP-1:: monocyte chemoattractant protein-1
- MTX:: Methotrexat
- NPT:: Neomycin-Phosphotransferase
- PCR:: polymerase chain reaction
- Phe (=F):: Phenylalanin
- SEAP:: secreted alkaline phosphatase
- Ub:: Ubiquitin
- UTR:: untranslatierte Region

### METHODEN

### Zellkultur ud Transfektion

Die Zellen *CHO-DG44*/*dhfr^{-l-}* (Urlaub et al., 1983) wurden permanent als Suspensionszellen in serum-freiem und mit Hypoxanthin und Thymidin (HT) supplementiertem CHO-S-SFMII Medium (Invitrogen GmbH, Karlsruhe, DE) in Zellkulturflaschen bei 37°C in feuchter Atmosphäre und 5% CO₂ kultiviert. Die Zellzahlen sowie die Viabilität wurden mit einem Coulter Counter Z2 (Beckmann Coulter) oder mit einem Cedex (Innovatis) bestimmt und die Zellen dann in einer Konzentration von 1 - 3 x10⁵/mL, eingesät und alle 2 - 3 Tage passagiert.
Zur Transfektion von CHO-DG44 wurde Lipofectamine Plus Reagenz (Invitrogen) eingesetzt. Pro Transfektionsansatz wurden dabei insgesamt 1,0 - 1,3 µ Plasmid-DNA, 4 µ Lipofectamine und 6 µ Plus-Reagenz nach den Angaben des Herstellers gemischt und in einem Volumen von 200 µ zu 6x10⁵ Zellen in 0,8 ml HT-supplementiertem CHO-S-SFMII Medium gegeben. Nach dreistündiger Inkubation bei 37°C in einem Zellinkubator erfolgte eine Zugabe von 2 mL HT-supplementiertem CHO-S-SFMII Medium. Nach einer Kultivierungszeit von 48 Stunden wurden die Transfektionsansätze entweder geerntet (transiente Transfektion) oder einer Selektion unterworfen. Zur NPT-baiserten Selektion wurden die Zellen 2 Tage nach Transfektion in HT-supplementiertes CHO-S-SFMII Medium mit 400 µ/mL G418 (Invitrogen) transfieriert. Zur DHFR-basierten Selektion wurden die Zellen 2 Tage nach Transfektion in HT-freies CHO-S-SFMII Medium überführt. Bei einer DHFR- und NPT-basierten Selektion im Falle einer Go-Transfektion, in der der eine Expressionsvektor einen DHFR- und der andere einen NPT-Selektionsmarker enthielt, wurden die Zellen 2 Tage nach Transfektion in CHO-S-SFMII Medium ohne Hypoxanthin- und Thymidinzusatz transferiert und dem Medium außerdem noch G418 (Invitrogen) in einer Konzentration von 400 µg/mL zugesetzt.
Eine DHFR-basierte Genamplifikation der integrierten heterologen Gene kann durch Zugabe des Selektionsagens MTX (Sigma, Deisenhofen, DE) in einer Konzentration von 5 - 2000 nM zu einem HT-freien CHO-S-SFMII Medium erreicht werden.

### Expressionsvektoren

Zur Expressionsanalyse wurden eukaryontische Expressionsvektoren eingesetzt, die auf dem pAD-CMV Vektor (Werner et al., 1998) basieren und die Expression eines heterologen Gens über die Kombination CMV Enhancer/Hamster Ubiquitin/S27a Promotor (WO 97/15664) oder CMV Enhancer/CMV Promotor vermitteln. Während der Basisvektor pBID das dhfr-Minigen enthält, das als amplifzierbarer Selektionsmarker dient (siehe z.B. EP-0-393-438), ist im Vektor pBING das dhfr-Minigen durch ein modifiziertes NPT-Gen ersetzt worden. Es handelt sich dabei um die NPT-Variante D227G (Asp227Gly). Die Klonierung von pBING mit der NPT-Variante D227G sowie der IRES-GFP-Genregion erfolgte wie in (WO2004/050884) beschrieben. Das Basisplasmid pTE4 enthält die NPT-Variante F240I (Phe240Ile) als Selektionsmarker und ist ein Derivat des Plasmids pBING. Neben dem Austausch der NPT-Variante D227G durch die NPT-Variante F240I wurde zudem auch noch das GFP durch das rot fluoreszierende Protein DsRed2 aus dem Vektor pDsRed2 (Clontech, Palo Alto, CA, USA) ersetzt. Das Basisplasmid pTE5 enthält DHFR als Selektionsmarker und ist ein Derivat des Vektors pBIDG (WO2004/050884), in dem ebenfalls das GFP durch das rot fluoreszierende Protein DsRed2 aus dem Vektor pDsRed2 (Clontech, Palo Alto, CA, USA) ersetzt wurde.

Zur Expression eines monoklonalen humanisierten IgG1 Antikörpers wurde die schwere Kette als 1,4 kb Sall/SpeI-Fragment in das mit XbaI- und SalI-verdaute Plasmid pBID kloniert, wobei das Plasmid pBID-HC resultierte (Abb. 1A). Die leichte Kette hingegen wurde als 0,7 kb BamHI/HindIII-Fragment in die Schnittstellen BgIII/HindIII des Plasmids pBING kloniert, wodurch das Plasmid pBING-LC (Abb. 1A) entstand.

Die humane MCP-1 cDNA (Yoshimura et al., 1989) wurde als 0,3 kb HindIII/EcoRI-Fragment in die entsprechenden Schnittstellen des Vektors pTE4 bzw. pTE5 kloniert, wobei die Plasmide pTE4/MCP-1 bzw. pTES/MCP-1 resultierten (Abb. 1B bzw. Abb. 2).

### FACS (fluorescerace-activated cell sorter)

Die flowcytometrischen Analysen wurden mit einem BD FACScalibur (BD Bioscience) durchgeführt. Das FACS ist mit einem Helium-Argon-Laser mit einer Anrcgungswellenlänge von 488 nm ausgestattet. Die Fluoreszenzintensität wird bei einer dem jeweiligen Fluoreszenzprotein adäquaten Wellenlänge aufgenommen und mittels der angeschlossenen Software Cell Quest Pro prozessiert.

### ELISA (enzyme-linked immunosorbant assay)

Die MCP-1-Titer in Überständen von stabil oder transient transfizierten CHO-DG44 Zellen wurden mittels ELISA mit dem Kit OptEIA Human MCP-1 Set nach dem Protokoll des Herstellers quantifiziert (BD Biosciences Pharmingen, Heidelberg, DE).
Die Quantifizierung des IgG1 mAks in den Überständen von stabil transfizierten CHO-DG44 Zellen erfolgte mittels ELISA nach Standardprotokollen (Current Protocols in Molecular Biology, Ausubel et al., 1994, updated), wobei zum einen ein Ziege anti Human IgG Fc-Fragment (Dianova, Hamburg, DE) und zum anderen ein AP-konjugierter Ziege anti Human Kappa light chain Antikörper (Sigma) eingesetzt wurde. Als Standard diente gereinigter IgG1 Antikörper.
Produktivitäten (pg/Zelle/Tag) wurden dabei mit der Formel pg/((Ct-Co) t / ln (Ct-Co)) berechnet, wobei Co und Ct die Zellzahl bei Aussaat bzw. Ernte und t die Kultivierungsdauer angibt.

### SEAP-Assay

Der SEAP-Titer in Kulturüberständen von transient transfizierten CHO-DG44 Zellen wurde unter Verwendung des SEAP Reporter Gene Assays nach den Protokollvorgaben des Hersteller quantifiziert (Roche Diagnostics GmbH, Mannheim, DE).

### BEISPIEL 1: ISOLIERUNG UND KLONIERUNG DES TE-ELEMENTS TE-A

Ausgehend von der in der WO97/15664 beschriebenen Sequenz aus dem Hamstergenom, die neben der kodierenden Region für das Ubiquitin/S27a-Gen auch angrenzende 5'UTR-Bereiche inklusive des Ub/S27a-Promotors umfasst, wurden weiter stromaufwärts gelegene, bisher unbekannte Sequenzbereiche isoliert. Hierzu wurde Adapter-ligerte genomische CHO-DG44 DNA als Matrize für "nested PCRs" eingesetzt. Die erste PCR wurde mit einer Kombination von Primern mit Komplementarität zum Adaptor bzw. zu einer Hamstersequenz im 5' Bereich der in der WO97/15664 unter SEQ ID Nr. 5 geführten Sequenz (Primer Ub20: 5'- CTCCACACATTTACACATGGACAC-3' (SEQ ID Nr. 39)); entspricht Nukleotiden 62 bis 85 (komplentäre Sequenz) der SEQ ID Nr. 5 aus WO 97/15664) durchgeführt. Nachfolgend wurde eine zweite PCR mit einer zweiten Primerkombination, bestehend aus einem inneren Adaptorprimer und einem inneren ("nested") Hamster-spezifischen Primer (Primer Ub21: 5'-GGGTTTCTCTGTGTAATAGCCATG-3'(SEQ ID Nr. 40); enspricht Nukleotiden 16 bis 39 (komplementäre Sequenz) der SEQ ID Nr.5 aus WO97/15664), durchgeführt. Die resultierenden überlappenden DNA-Fragmente, die am Hamster-spezifischen Primerende mit einer bekannten Sequenz starteten und dann in neue, unbekannte stromaufwärts gelegene Sequenzbereiche übergingen, wurden in pCR2.1 TOPO-Vektoren (Invitrogen) subkloniert und durch Sequenzierung analysiert. Insgesamt wurden 348 bp einer neuen, bisher unbekannten Sequenz stromaufwärts vom Hamster Ub/S27a-Gen erhalten.
Auf der Basis dieser neuen Sequenzinformation wurde ein noch weiter stromaufwärts liegender DNA-Bereich mittels der oben beschriebenen "nested PCR" isoliert. Diesmal wurde die erste PCR mit einem Adapterprimer und dem Primer Ub33 (5'-ATCTCACTGTGTCTACCAACTTAG-3' (SEQ ID Nr. 41); im 5' Bereich der neu isolierten 384 bp-Sequenz gelegen; entspricht Nukleotid 1268 - 1291 (komplementäre Sequenz) der SEQ ID Nr. 1) und die zweite PCR mit einem inneren Adaptorprimer und dem weiter innen gelegenen hamsterspezifischen Primer Ub32a (5'-TCTGCACCACCACTACCTGACT -3' (SEQ ID Nr. 42); stromaufwärts vom Primer Ub33 innerhalb der neu isolierten 384 bp-Sequenz gelegen; entspricht Nukleotid 1243 - 1264 (komplementäre Sequenz) der SEQ ID Nr. 1) durchgerührt. Das erhaltene Amplifikat wurde in den pCR2.1 TOPO-Vektor (Invitrogen) subkloniert und sequenziert. Es enthielt weitere 1239 bp einer neuen, bisher unbekannten Sequenz stromaufwärts vom Hamster Ub/S27a-Gen.
Die aus den überlappenden PCR-Fragmenten erhaltene Sequenzinformation wurde genutzt, um unter Verwendung der Primer
a) Ub34 (5'-CTAAGAGTACTTGCCATGAGAGCCTGAA-3' (SEQ ID Nr. 43);am äußersten 5' Ende der neu isolierten 1239 bp-Sequenz lokalisiert; in SEQ ID Nr.1 nur noch partiell (Nukleotide 1 bis 14) enthalten )
   und
b) Ub35 (5'-CATTGATACACCACCAAAGAACTTG-3' (SEQ ID Nr. 44); enspricht Nukleotiden 1941 bis 1965 (komplementäre Sequenz) der SEQ ID Nr. 1)
einen zusammenhängenden Sequenzbereich aus der genomischen DNA von CHO-DG44 via PCR zu amplifizieren, der all bisher isolierten Teilfragmente umfasste und 383 bp in den 5' Sequenzbereich der SEQ ID Nr. 5 aus WO 97/15664 hineinreichte. Das resultierende 2 kb DNA-Fragment wurde mit dem 5' UTR-Bereich der in WO 97/15664 beschriebenen Sequenz ID Nr. 5 über die endogene EcoRI-Schnittstelle (Position 353 - 358) ligiert, wobei diese Schnittstelle jedoch durch eine Auffüllreaktion mit Klenow DNA-Polymerase elimieniert wurde. Eine zweite endogene EcoRI-Schnittstelle im neu isolierten Genombereich wurde auf die gleiche Weise eliminiert, woraus die gegenüber der ursprünglichen Genomsequenz zusätzlichen Nukleotide 326 bis 329 in der SEQ ID Nr. 1 resultierten. Insgesamt wurden in die SEQ ID Nr. 1 auf diese Weise 8 zusätzliche Nukleotide im Vergleich zur endogen Hamster-Sequenz eingeführt. Das resultierende 3788 bp große DNA-Fragment aus einem stromaufwärts vom Hamster Ub/S27a-Gens gelegenen Sequenzbereichs wurde als TE-Element A mit der Sequenz ID Nr.1 bezeichnet und in den Vektor pBluescript SKM subkloniert (Stratagene, La Jolla, CA).

### BEISPIEL 2: Generierung von diversen TE-Expressionsvektoren

Ausgehend vom 3,8 kb großen TE-Element TE-A (Abb. 3, Sequenz ID Nr. 1) aus dem CHO-Genom wurden mittels PCR diverse Fragmente erzeugt; die im Vergleich zum TE-Element TE-A entweder Deletionen am 5'- oder am 3'-Ende aufwiesen (Abb. 4 und Abb. 5). Zur Synthese dieser Fragmente wurden jeweils Kombinationen aus direkten und einem reversen Primern eingesetzt (Abb. 5 und Abb.6). Zu Klonierungszwecken wurde durch die Primer jeweils am 5'-Ende des Teilstücks eine BamHI-Schnittstelle und am 3'-Ende eine BsrGI-Schnittstelle angefügt. Auf diese Art und Weise wurden 12 TE-Elemente unterschiedlicher Länge mit der Bezeichnung TE-01 bis TE-12 generiert (Abb. 4 und 5). Nach Verdau mit BsrGI und BamHI wurden diese jeweils in direkter Orientierung in die Adapterregion der Basisplasmide pTE4/MCP-1 (Abb. 1B) bzw. pTE5/MCP-1 (Abb. 2) kloniert.
Das Fragment TE-00 (SEQ ID Nr. 2) wurde via SacII-Restriktionsenzymverdau aus einem Subklon von TE-A isoliert und sowohl in direkter als auch in reverser Orientierung über die 5' vom Promotor/Enhancer-Element gelegene SpeI-Schnittstelle in die Basisvektoren pBING-LC (Abb. IA) bzw. pBID-HC (Abb. 1A) kloniert.

### Sequenzen der TE-Elemente

TE-A (SEQUENZ ID NR. 1)
TE-ELEMENT 00 (SEQUENZ ID NR. 2)
TE-ELEMENT 01 (SEQUENZ ID NR. 3)
TE-ELEMENT 02 (SEQUENZ ID NR. 4)
TE-ELEMENT 03 (SEQUENZ ID NR. 5)
TE-ELEMENT 04 (SEQUENZ ID NR. 6)
TE-ELEMENT 05 (SEQUENZ ID NR. 7)
TE-ELEMENT 06 (SEQUENZ ID NR. 8)
TE-ELEMENT 07 (SEQUENZ ID NR. 9)
TE-ELEMENT 08 (SEQUENZ ID NR. 10)
TE-ELEMENT 09 (SEQUENZ ID NR. 11)
TE-ELEMENT 10 (SEQUENZ ID NR. 12)
TE-ELEMENT 11 (SEQUENZ ID NR. 13)
TE-ELEMENT 12 (SEQUENZ ID NR. 14)

### BEISPIEL 3: Einfluss der TE-Elementvariante TE-00 auf die Expression von GFP und Immunglobulin G1 (IgG1)

Die Wirkung des TE-Elements TE-00 auf die Expression des zytoplasmatisch lokalisierten GFPs (grün fluoreszierendes Protein) und eines sezernierten monoklonalen IgG1-Antikörpcrs wurde in zwei unabhängigen stabilen Transfektionsserien mit CHO-DG44 Zellen untersucht. Hierzu wurden CHO-DG44 Zellen mit folgenden Plasmidkombinationen bzw. Plasmidvarianten co-transfiziert:
a) Kontrollplasmide pBING-LC (Abb. 1A) und pBID-HC (Abb. 1A) ohne TE-Element
b) pBING-LC und pBID-HC mit jeweils stromaufwärts vom Promoter/Enhancer integriertem TE-Element TE-00 in direkter Orientierung
c) pBING-LC und pBID-HC mit jeweils stromaufwärts vom Promoter/Enhancer integriertem TE-Element TE-00 in reverser Orientierung

In der Transfektionsserie A wurden jeweils vier Pools, in Transfektionsserie B jeweils zehn Pools pro Variante erzeugt. Es wurden dabei jeweils äquimolare Mengen beider Plasmide eingesetzt. Um auf die gleiche Gesamtmolekülzahl zu kommen, betrug die in Serie A zur Transfektion eingesetzte Gesamt-DNA-Menge bei den Kontrollansätzen 1 µg, in den Ansätzen mit TE-Element 1,3 µg. Dieser Unterschied resultierte aus den unterschiedlichen Plasmidgrößen, denn die Plasmide mit TE-Element waren um den Faktor 1,3 größer als die Kontrollplasmide. Da die eingesetzte DNA-Menge im Transfektionsmix einen Einfluss auf die Transfektionseffizienz haben kann, wurde in der Serie B die Gesamt-DNA-Menge mit 300 ng "Mock-DNA" (= Vektor ohne Produktgen, TE-Element und eukaryontischen Selektionsmarker) ausgeglichen, so dass im Ansatz mit den Kontrollplasmiden in Summe ebenfalls 1,3 µg DNA enthalten waren. Als Negativkontrolle wurde bei jeder Transfektionsserie ein Mock-transfizierter Pool mitgeführt, d.h. gleich behandelt, aber ohne DNA-Zugabe im Transfektionsansatz. Die Selektion stabil transfizierter Zellen erfolgte zwei Tage nach der Transfektion mit - HT/+G418 (400 µg/mL).
Nach der Selektion wurde im FACS der Anteil GFP-exprimierender Zellen bestimmt. Der Vergleich der Varianten im Plot-Overlay ergab in beiden Transfektionsserien für Pools mit TE-Element 00 einen größeren Anteil GFP-exprimierender Zellen als in Pools mit Kontrollplasmiden (Abb. 7). Zwischen den Pools, in denen das TE-Element entweder in direkter oder in reverser Orientierung im Plasmid vorlag, zeigten sich keinerlei Unterschiede. Die Wirkung des TE-Elements 00, nämlich den Anteil an Zellen mit höherer Produktivität in einer gemischten Population zu erhöhen, war folglich unabhängig von dessen Orientierung.
Zusätzlich wurden auch die IgG1-Titer und die spezifische Produktivität der Pools über einen Zeitraum von sechs bis acht Passagen (Passagierungsrhythmus 2-2-3 Tage) ermittelt. Auch hierbei bestätigte sich, dass die Zellpools mit dem TE-Element 00 im Durchschnitt mehr exprimierten als die Zellpools ohne TE-Element (Abb. 9, Serie A und B). In beiden Serien konnte eine Verdopplung der Pool-Produktivität durch das Vorhandensein des TE-Elements nachgewiesen werden, wobei es keine Rolle spielte, in welcher Orientierung das Element im Expressionsplasmid einkloniert wurde.

### BEISPIEL 4: Einfluss der TE-Elemente TE-01 bis TE-12 auf die Expression von MCP-1

Die Wirkung der TE-Elemente TE-01 bis TE-12 auf die Expression des sezernierten MCP-1 wurde in 3 stabilen Transfektionsserien (Serie C, D und E) von CHO-DG44-Zellen im Vergleich zur Expression ohne das TE-Element untersucht. In allen drei Serien wurden jeweils sechs Pools pro Plasmidvariante erzeugt. Das Basisplasmid war in Serie C und D pTE4/MCP-1 (Abb. 1B; Selektionsmarker NPT= Neomycin-Phosphotransferase F240I), in Serie E pTE5/MCP-1 (Abb. 2; Selektionsmarker DHFR = Dihydrofolat-Reduktase). Diese enthielten entweder kein TE-Element (= Kontrollansätze) oder jeweils eines der TE-Elemente TE-01 bis TE-12 in direkter Orientierung stromaufwärts vom Promotor/Enhancer. Um eine Beeinflussung der Transfektionseffizienz durch unterschiedliche DNA-Mengen im Transfektionsansatz zu minimieren, wurde jeweils in der Summe 1,2 µg Plasmid-DNA eingesetzt. Abhängig von der Größe des eingebrachten TE-Elements variierte die Plasmidgröße zwischen 6,7 kb und 10,7 kb. Damit in allen Ansätzen jedoch die Gesamtmolekülzahl der Testplasmide konstant gehalten werden konnte, wurde in den Ansätzen mit kleineren Plasmidmolekülen die Gesamt-DNA-Menge mit einem sogenannten Mockplasmid ausgeglichen, das weder Produktgen und TE-Element noch einen eukaryontischen Selektionsmarker enthielt. Als Negativkontrolle wurde bei jeder Transfektionsserie ein Mock-transfizierter Pool mitgeführt, d. h. gleich behandelt, aber ohne DNA-Zugabe im Transfektionsansatz. Die Selektion stabil transfizierter Zellen erfolgte zwei Tage nach der Transfektion, in den Serien C und D mit HT-supplementiertem CHO-S-SFMII +G418 (400 µg/mL), in Serie E mit HT-freiem CHO-S-SFMII.
Nach der Selektion wurde im FACS der Anteil dsRed2-exprimierender Zellen bestimmt. Abbildung 8 stellt die relative, prozentuale Fluoreszenz der lebenden transfizierten Zellen aus Serie C dar. Im Vergleich zu den Kontrollpools enthielten die Pools, die die TE-Elemente TE-01, TE-02 oder TE-08 enthielten, etwa 3 bis 3,5mal mehr dsRed2-exprimierende Zellen und Pools mit dem Element TE-06 eine etwa doppelt so hohe Zahl an dsRed2-exprimierenden Zellen. Bei Pools mit den Fragmenten TE-05 und TE-09 konnte hingegen keine Steigerung des Anteils von DsRed2-exprimierenden Zellen im Vergleich zur Kontrolle beobachtet werden.

Zusätzlich wurden auch MCP-1-Produkttiter und die spezifische Produktivität über einen Zeitraum von 6 Passagen (Passagierungsrhythmus 2-2-3 Tage) erhoben. In Abbildung 9 (Serie C und D) und Abbildung 10 (Serie E) sind die relativen spezifischen MCP-1-Produktivitäten dargestellt. Das Element TE-08 zeigte hierbei in Verbindung mit NPT F240I als Selektionsmarker mit Faktor 5,3 die größte Steigerung der spezifischen MCP-1-Produktivität gegenüber den Kontrollpools ohne TE-Element (Abb.9). In Verbindung mit DHFR als Selektionsmarker wurde mit dieser Variante eine 6fache Steigerung erzielt (Abb. 10). Die TE-Elemente 01, 02 und 03 ergaben bei den NPT-selektionierten Pools eine 4- bzw. 4,5-fache (Abb. 9) und bei den DHFR-selektionierten Pools eine 2,6- bis 6,8-fache Steigerung der Produktivität (Abb. 10) gegenüber den Kontrollpools. Auch das nur 300 bp große TE-Element 06 konnte die Produktivität in allen Serien um den Faktor 2,5 bis 3,2 steigern (Abb. 9 und 10). Die mit den Fragmenten TE-04 und TE-07 erzielten Steigerungen lagen ebenfalls in diesem Größenbereich (Abb. 10). Pools, in denen die etwas längeren Fragmente TE-10, TE-11 und TE-12 verwendet wurden, wiesen eine Verdoppelung der MCP-1 Expression auf (Abb. 9 und 10). Offensichtlich war in all diesen Pools die Zahl der Zellen, die kein oder wenig Produkt exprimieren, reduziert und somit stieg insgesamt der Anteil an Hochproduzenten in der Zellpopulation. Das ist ein Hinweis, dass die TE-Elemente negative chromosomale Positionseffekte unterdrücken, abschirmen oder aufheben können.
Im Gegensatz dazu konnte durch die Verwendung der Fragmente TE-05 und TE-09 die Expression, wie auch schon bei der DsRed2-Expression gesehen (Abb. 8), im Vergleich zur Kontrolle nicht gesteigert werden und war in einigen Fällen sogar geringer (Abb. 9 und 10). Diese Elemente, bzw. Teilfragmente in diesen Sequenzbereichen, könnten somit möglicherweise sogar eher eine reprimierende Wirkung ausüben.
Insgesamt gesehen korrelierte die beobachte Änderung in der MCP-1 Expression mit dem Anteil von DsRed2-exprimierenden Zellen in den stabilen Zellpools.

### BEISPIEL 5: Test der TE-Elemente TE-01 bis TE-12 auf Enhanceraktivität

Durch transiente Transfektion von CHO-DG44 Zellen wurde überprüft, ob die beobachtete Steigerung der Produktexpression tatsächlich auf einem chromatinöffnenden Effekt der TE-Elemente beruht oder ob sie auf einer Enhanceraktivität basiert. Da in einer transienten Transfektion das Plasmid nicht in das Genom integriert, wird die genetische Information direkt vom Plasmid abgelesen. Somit können keine chromosomalen Positionseffekte entstehen. Treten dennoch positive Effekte auf die Genexpression auf, so könnten diese auf im TE-Element vorliegende Enhancer zurückgegeführt werden. Solche Enhancer können in *cis*-Lokalisierung positions- und orientierungsunabhängig auf die Aktivität eines Promotors einwirken und die Transkription eines funktionell verknüpften Gens stimulieren.
In der in Abbildung 11 gezeigten transienten Expressionsstudie wurden jeweils 6 Pools mit dem Basisplasmid pTE4/MCP-1 (= Kontrolle; Abb. 1B) bzw. Derivaten davon, die jeweils zusätzlich eines der TE-Elemente TE-01 bis TE-12 stromaufwärts vom Promotor/Enhancer enthielten, transfiziert. Nach 48 h Kultivierung in einem Gesamtvolumen von 3 mL erfolgte die Ernte und die Bestimmung des MCP-1-Titers im Zellkulturüberstand mittels ELISA. Unterschiede in der Transfektionseffizienz wurden durch Go-Transfektion mit einem SEAP-Expressionsplasmid (Zugabe von jweils 100 ng Plasmid-DNA pro Transfektionsansatz) und nachfolgende Messung der SEAP-Aktivitität korrigiert. In Abbildung 11 ist der Mittelwert aus den jeweils 6 Parallelpools dargestellt. Die Daten zeigen, dass die MCP-1-Titer im Zellkulturüberstand in allen Pools sehr ähnlich waren und es keine signifikanten Expressionsunterschiede zum Kontrollplasmid pTE4/MCP-1 ohne TE-Element gab. Somit beruht die durch einige TE-Elemente bewirkte Produktivitätssteigerung um mehr als den Faktor 2 bei stabil transfizierten Zellpools nicht auf dem Vorhandensein eines Enhancers in der TE-Sequenz. Für die durch TE-Elemente bedingte Expressionssteigerung ist eine chromosomale Integration also zwingend erforderlich.

### BEISPIEL 6: Erzeugung weiterer TE-Elemente und Testung unterschiedlicher TE-Element-Positionen und -Kombinationen

Analog der in Beispiel 2 beschriebenen Vorgehensweise können weitere Teilfragmente der Sequenz ID Nr. 1 oder auch Derivate davon generiert werden und, wie in Beispiel 3 und 4 geschildert, hinsichtlich ihres positiven Einflusses auf die Produktivität getestet werden.

Einige Ausführungsbeispiele für mögliche Fragmente sind in Abbildung 12 dargestellt. Die bisherigen Ergebnisse deuten beispielsweise darauf hin, dass die in Abbildung 12 gezeigten Bereiche der Sequenz ID Nr. 1 ebenfalls eine Steigerung der Genexpression hervorrufen könnten. In stabilen Transfektionsserien sollen diese neuen TE-Elemente hinsichtlich ihres Einflusses auf die spezifische Produktivitätnäher näher charakterisiert werden, um die für die Funktion wichtigen Sequenzbereiche exakter zu lokalisieren und weiter einzugrenzen. Eine Eingrenzung der Funktion auf bestimmte Sequenzbereiche und der damit auch verbundenen möglichen Reduktion der Fragmentlänge ist für einen effizienten Einsatz in Expressionsvektoren vorteilhaft, da kleinere Expressionsplasmide stabiler und sowohl bei der Klonierung als auch bei der Tranfektion einfacher handhabbar sind.
Des Weiteren ist eine beliebige Anordnung gleichartiger oder verschiedener Fragmentbereiche in beliebiger Orientierung zueinander und auch in beliebiger Position innerhalb des Plasmids möglich. Die Untersuchung, welche der Kombinationen in einer bestmöglichen Steigerung der Expression resultiert, kann wiederum in stabilen Transfektionsserien untersucht werden. Einige Ausführungsbeispiele, die in keiner Weise limitierend sind, sind in Abbildung 13 dargestellt. So soll beispielsweise untersucht werden, ob die TE-Elemente TE-06 und TE-08 bei beidseitiger Flankierung des Produktgens oder bei Hintereinanderschaltung eine zusätzliche Steigerung der Expression bewirken können. Außerdem ist denkbar, dass ein Hintereinanderschalten kurzer TE-Elemente, ob gleiche oder verschiedene, wie z.B. TE-Element 06 und 08, ebenfalls zu einem zusätzlichen expressionssteigernden Effekt führen.

### Weitere TE-Elemente

TE-ELEMENT 13 (SEQUENZ ID NR. 15)
TE-ELEMENT 14 (SEQUENZ ID NR. 16)
TE-ELEMENT 15 (SEQUENZ ID NR. 17)
TE-ELEMENT 16 (SEQUENZ ID NR. 18)
TE-ELEMENT 17 (SEQUENZ ID NR. 19)
TE-ELEMENT 18 (SEQUENZ ID NR. 20)
TE-ELEMENT 21 (SEQUENZ ID NR. 21)

### BEISPIEL 7: Einfluss der TE-Elemente TE-13 bis TE-18 auf die Expression von MCP-1

Die Wirkung der TE-Elemente TE-13 bis TE-18 auf die Expression des sezernierten MCP-1 wurde in einer stabilen Transfektionsserien (Serie F) von CHO-DG44-Zellen im Vergleich zur Expression ohne das TE-Element untersucht. Es wurden jeweils vier Pools pro Plasmidvariante erzeugt. Das Basisplasmid war in allen Serien pTE4/MCP-1 (Abb. 1B; Selektionsmarker NPT= Neomycin-Phosphotransferase F240I). Die verschiedenen Plasmidvarianten enthielten entweder kein TE-Element (= Kontrollansätze) oder jeweils eines der TE-Elemente TE-13 bis TE-18 in direkter Orientierung stromaufwärts vom Promotor/Enhancer (Abbildung 12). Um eine Beeinflussung der Transfektionseffizienz durch unterschiedliche DNA-Mengen im Transfektionsansatz zu minimieren, wurde jeweils in der Summe 1,2 µg Plasmid-DNA eingesetzt. Abhängig von der Größe des eingebrachten TE-Elements variierte die Plasmidgröße zwischen 6,7 kb und 8,2 kb. Als Negativkontrolle wurde bei jeder Transfektionsserie ein Mock-transfizierter Pool mitgeführt, d. h. gleich behandelt, aber ohne DNA-Zugabe im Transfektionsansatz. Die Selektion stabil transfizierter Zellen erfolgte zwei Tage nach der Transfektion, mit HT-supplementiertem CHO-S-SFMII +G418 (400 µg/mL).
MCP-1-Produkttiter und die spezifische Produktivität wurden über einen Zeitraum von 5 bis 6 Passagen (Passagierungsrhythmus 2-2-3 Tage) erhoben. In Abbildung 14 (Serie F) sind die relativen spezifischen MCP-1-Produktivitäten dargestellt. Jedes der Elemente führt zu einer Steigerung der durchschnittlichen MCP-1-Expression. Die größte Steigerung (15-fach) verursachte das Element 13, das damit sogar die 10-fache Steigerung durch das Element 08 übertrifft.

### BEISPIEL 8: Einfluss der TE-Elemente an verschiedenen Positionen und in verschiedenen Kombinationen auf die Expression von MCP-1

Die Wirkung der TE-Elemente TE-06 und TE-08 in verschiedenen Kombinationen und an verschiedenen Positionen im Expressionsplasmid auf die Expression des sezernierten MCP-1 wird in 2 stabilen Transfektionsserien (Serie G und H) von CHO-DG44-Zellen im Vergleich zur Expression ohne das TE-Element untersucht. In beiden Serien werden jeweils sechs Pools pro Plasmidvariante erzeugt. Das Basisplasmid ist pTE-4/MCP-1 (Abb. 1B; Selektionsmarker NPT= Neomycin-Phosphotransferase F240I). Die verschiedenen Plasmidvarianten enthalten entweder kein TE-Element (= Kontrollansätze) oder TE-08 bzw. TE-A vor dem Enhancer/Promotor-Element oder die Kombination von TE-06 und TE-08 vor dem Enhancer/Promotor-Element oder TE-08 bzw. TE-09 in reverser Orientierung vor dem Enhancer/Promotor-Element (Serie G). In Serie H werden die Elemente TE-06 und TE-21 bzw. TE-08 vor dem Enhancer/Promotor-Element (E/P) und zusätzlich nach dem Terminationssignal (T) (Abbildung 13) eingesetzt. Um eine Beeinflussung der Transfektionseffizienz durch unterschiedliche DNA-Mengen im Transfektionsansatz zu minimieren, werden jeweils in der Summe 1,2 µg Plasmid-DNA eingesetzt. Abhängig von der Größe des eingebrachten TE-Elements variiert die Plasmidgröße zwischen 6,7 kb und 10,2 kb. Als Negativkontrolle wird bei jeder Transfektionsserie ein Mock-transfizierter Pool mitgeführt, d. h. gleich behandelt, aber ohne DNA-Zugabe im Transfektionsansatz. Die Selektion stabil transfizierter Zellen erfolgt zwei Tage nach der Transfektion, mit HT-supplementiertem CHO-S-SFMII +G418 (300 µg/mL).
MCP-1-Produkttiter und die spezifische Produktivität werden über einen Zeitraum von 6 Passagen (Passagierungsrhythmus 2-2-3 Tage) erhoben. In Abbildung 15 sind die relativen spezifischen MCP-1-Produktivitäten der Serie G dargestellt. Alle Elemente führen zu einer Steigerung der durchschnittlichen MCP-1-Expression. Die größte Steigerung (4-fach) verursacht das Element TE-A. Der Einsatz der Elemente TE-06 und TE-21 bzw. TE-08 vor und nach der Expressionskassette verursacht ebenfalls eine Steigerung.

### BEISPIEL 9: Einfluss des TE-Elements TE-08 auf die Expression zweier Imnunglobuline G 4 (IgG4)

Die Wirkung des TE-Elements TE-08 auf die Expression zweier IgG4-Antikörper wird in einer stabilen Transfektionsserien (Serie J) von CHO-DG44-Zellen im Vergleich zur Expression ohne das TE-Element untersucht. Es werden jeweils 24 Pools mit den Basisplasmiden pBIN-LC2 bzw. pBIN-LC3 und pBID-HC2 bzw. pBID-HC3 und 24 Pools mit pBIN-LC2/TE08 bzw. pBIN-LC3/TE08 und pBID-HC2/TE08 bzw. pBID-HC3/TE08 erzeugt (Abb. 16; Selektionsmarker NPT= Neomycin-Phosphotransferase F240I und dhfr = Dihydrofolatreduktase). Um eine Beeinflussung der Transfektionseffizienz durch unterschiedliche DNA-Mengen im Transfektionsansatz zu minimieren, wird jeweils in der Summe 1,2 µg Plasmid-DNA eingesetzt. Abhängig von der Größe des eingebrachten TE-Elements variiert die Plasmidgröße zwischen 6,1 kb und 7,5 kb. Als Negativkontrolle wird bei jeder Transfektionsserie ein Mock-transfizierter Pool mitgeführt, d. h. gleich behandelt, aber ohne DNA-Zugabe im Transfektionsansatz. Die Selektion stabil transfizierter Zellen erfolgt zwei Tage nach der Transfektion, mit HT-freiem CHO-S-SFMII +G418 (400 µg/mL).
IgG4-Produkttiter und die spezifische Produktivität werden über einen Zeitraum von 4 Passagen (Passagierungsrhythmus 2-2-3 Tage) erhoben. Das Element 08 führt bei der Expression von IgG4-Antikörpern zu einer Steigerung der durchschnittlichen Expressionsrate. Des Weiteren kann durch das Vorhandensein des Elements TE-08 die Chance, einen hochproduzierenden Zellpool zu finden, gesteigert werden.

### BEISPIEL 10: Einfluss von TE-Elementen auf die Proteinexpression in 293F-Zellen

Die Wirkung verschiedener TE-Elemente auf die Expression des sezemierten MCP-1 wird in einer stabilen Transfektionsserie (Serie K) von HEK293Freestyle-Zellen im Vergleich zur MCP-1-Expression ohne ein TE-Element untersucht. Das Basisplasmid ist pTE-4/MCP-1 (Abb. 1B; Selektionsmarker NPT= Neomycin-Phosphotransferase F240I). Es werden die Elemente TE-08, TE-13 und TE-A in direkter Orientierung stromaufwärts vom Enhancer/Promotor eingesetzt und jeweils 7-10 Pools pro Plasmidvariante erzeugt. Um eine Beeinflussung der Transfektionseffizienz durch unterschiedliche DNA-Mengen im Transfektionsansatz zu minimieren, werden jeweils in der Summe 1,2 µg Plasmid-DNA eingesetzt. Abhängig von der Größe des eingebrachten TE-Elements variiert die Plasmidgröße zwischen 6,7 kb und 10,2 kb. Als Negativkontrolle wird bei jeder Transfektionsserie ein Mock-transfizierter Pool mitgefiihrt, d. h. gleich behandelt, aber ohne DNA-Zugabe im Transfektionsansatz. Die Selektion stabil transfizierter Zellen erfolgt zwei Tage nach der Transfektion mit 293 SFM II-Medium + 4 mM Glutamin + G418 (100 µg/ml).
MCP-1-Produkttiter und die spezifische Produktivität werden über einen Zeitraum von 5 bis 6 Passagen (Passagierungsrhythmus 2-2-3 Tage) erhoben.

### BEISPIEL 11: EINFLUSS DES TE-ELEMENTS TE-08 AUF DIE EXPRESSION EINES ENZYMS (SEAP)

Die Wirkung des TE-Elements TE-08 auf die Expression eines Enzyms (SEAP) wird in einer stabilen Transfektionsserie (Serie L) von CHO-DG44-Zellen im Vergleich zur SEAP-Expression ohne das TE-Element untersucht. Es werden jeweils sechs Pools pro Plasmidvariante erzeugt. Das Basisplasmid ist pTE-4/SEAP. Es wird durch einen Austausch der MCP-1 - IRES - DsRed2-Expressionskassette gegen SEAP generiert. Das Element TE-08 wird in den Adaptor A einkloniert (Abb. 1B; Selektionsmarker NPT= Neomycin-Phosphotransferase F240I). Um eine Beeinflussung der Transfektionseffizienz durch unterschiedliche DNA-Mengen im Transfektionsansatz zu minimieren, wird jeweils in der Summe 1,2 µg Plasmid-DNA eingesetzt. Abhängig von der Größe des eingebrachten TE-Elements variiert die Plasmidgröße zwischen 6,6 kb und 7,6 kb. Als Negativkontrolle wird bei jeder Transfektionsserie ein Mock-transfizierter Pool mitgeführt, d. h. gleich behandelt, aber ohne DNA-Zugabe im Transfektionsansatz. Die Selektion stabil transfizierter Zellen erfolgt zwei Tage nach der Transfektion, mit HT-supplementiertem CHO-S-SFMII +G418 (400 µg/mL).

Die relative SEAP-Expression wird anhand des kommerziell erhältlichen SEAP-Assays (Clontech) ermittelt und über einen Zeitraum von 6 Passagen (Passagierungsrhythmus 2-2-3 Tage) erhoben.

### REFERENZLISTE

Adam, M.A. et al., J Virol 1991, 65, 4985 - 4990
Altschul, S.F. et al., Nucleic Acids Res. 1997, 25, 3389 - 3402
Altschul, S.F. et al., J Mol Biol 1990, 215, 403 - 410
Aronow, B.J. et al., Mol. Cell. Biol. 1995, 15, 1123-1135.
Ausubel, F.M. et al., Current Protocols in molecular biology. New York : Greene Publishing Associates and Wiley-Interscience 1994 (updated)
Baker,J.E., Journal of Experimental Medicine 1999, 190, 669-679.
Bell,A.C. and Felsenfeld,G., Current Opinion in Genetics & Development 1999, 9, 191-198.
Bennett, R.P. et al., BioTechniques 1998, 24, 478 - 482
Chalfie, M. et al., Science 1994, 263, 802 - 805
Chamov, S.M. et al., Antibody Fusion Proteins, Wiley-Liss Inc., 1999
Davies, M.V. et al., J Virol 1992, 66, 1924 - 1932
Delgado,S. et al., EMBO Journal 1998, 17, 2426-2435.
Faisst, S. et al., Nucleic Acids Research 1992, 20, 3 - 26
Gossen, M. et al., Curr Opi Biotech 1994, 5, 516 - 520
Haber, D.A. et al., Somatic Cell Genetics 1982, 8, 499 - 508
Harris et al., Protein Purification : A Practical Approach, Pickwood and Hames, eds., IRL Press, 1995
Hemann, C. et al., DNA Cell Biol 1994, 13 (4), 437 - 445
Hu, S. et al., Cancer Res. 1996, 56 (13), 3055 - 3061
Huston, C. et al., Proc Natl Acad Sci USA 1988, 85 (16), 5879 - 5883
Jang, S.K. et al., J Virol 1989, 63, 1651 - 1660
Jenuwein,T. et al., Nature 1997, 385, 269-272.
Kaufman, R.J., Methods in Enzymology 1990, 185, 537 - 566
Klehr,D. et al., Biochemistry 1991, 30, 1264-1270.
Kortt, A.A. et al., Protein Engineering 1997, 10 (4), 423 - 433
Kwaks,T.H.J. et al., Nature Biotechnology 2003, 21, 553-558.
Li,Q. et al., Blood 2002, 100, 3077-3086.
Lottspeich F. and Zorbas H. eds., Bioanalytic, Spektrum Akad. Verl., 1998
Lovejoy, B. et al., Science 1993, 259, 1288 - 1293
McKnight,R.A. et al., PNAS 1992, 89, 6943-6947.
Monaco, L. et al., Gene 1996, 180, 145 - 15
Morgan, R.A. et al., Nucleic Acids Research 1992, 20, 1293 - 1299
Mosser, D.D. et al., BioTechniques 1997, 22, 150 - 161
Ortiz,B.D. et al., Molecular & Cellular Biology 1999, 19, 1901-1909.
Ortiz,B.D. et al., EMBO J 1997, 16, 5037-5045.
Ohshima, Y. et al., J Mol Biol 1987, 195, 247 - 259
Pack, P. et al., Biotechnology 1993, 11, 1271 - 1277
Pack, P. et al., J Mol Biol 1995, 246 (11), 28 - 34
Pelletier, J. et al., Nature 1988, 334, 320 - 325
Perisic, O. et al., Structure 1994, 2, 1217 - 1226
Pikaart,M.J. et al., Genes Dev 1998, 12, 2852-2862.
Poljak,L. et al., Nucl. Acids. Res 1994, 22, 4386-4394.
Ramesh, N. et al., Nucleic Acids Research 1996, 24, 2697 - 2700
Sambrook, J. et al.,Molecular Cloning: A Laboratory Manual Cold Spring Harbor Laboratory, Cold Spring Harbor, New York, 1989
Sautter,K. and Enenkel,B., Biotechnology and Bioengineering 2005, 89, 530-538.
Scopes, R., Protein Purification, Springer Verlag, 1988
Simonson, C.C. et al., Proc Natl Acad Sei USA 1983, 80, 2495 - 2499
Stief,A. et al., Nature 1989, 341, 343-345.
Sugimoto et al., Biotechnology 1994, 12, 694 - 698
Udvardy,A. et al., Journal of Molecular Biology 1985, 185, 341-358.
Udvardy,A., EMBO J 1999, 18, 1-8.
Urlaub, G. et al., Cell 1983, 33, 405 - 412
Urlaub.G. et al., Somatic Cell & Molecular Genetics 1986, 12, 555-566.
Werner,R.G. et al., Arzneimittel-Forschung 1998, 48, 870-880.
Wigler. M. et al., Proc Natl Acad Sci USA 1980, 77, 3567 - 3570
Yoshimura,T., FEBS Letters 1989, 244, 487-493.
Zahn-Zabal,M. et al., Journal of Biotechnology 2001, 87, 29-42.
WO97/15664
EP-0-393-438
WO2004/050884
WO02/081677
US6,027,915
US6,309,851
WO01/04306
WO00/34318
WO00/34326
WO00/34526
WO01/271
US5,122,458
WO94/05785
WO92/08796
WO94/28143
WO03/004704

## Patentansprüche

1. Nukleinsäure, welche TE-13 (SEQ ID Nr. 15) enthält oder ein Fragment von TE-13 (SEQ ID Nr. 15) oder deren komplementäre Nukleotidsequenzen oder ein Derivat von TE-13 (SEQ ID Nr. 15) oder deren komplementäre Nukleotidsequenzen, wobei die Nukleinsäure, das Fragment, das Derivat oder deren komplementäre Nukleotidsequenzen bei chromosomaler Integration zu einer Steigerung der Transkription bzw. Expression eines Gens von Interesse in einem Expressionssystem führt, und wobei das Derivat mindestens 85% Sequenzidentität aufweist.

2. Nukleinsäure, welche TE-08 (SEQ ID Nr. 10) enthält oder ein Fragment von TE-08 (SEQ ID Nr. 10) oder deren komplementäre Nukleotidsequenzen oder ein Derivat von TE-08 (SEQ ID Nr. 10) oder deren komplementäre Nukleotidsequenzen, wobei die Nukleinsäure, das Fragment, das Derivat oder deren komplementäre Nukleotidsequenzen bei chromosomaler Integration zu einer Steigerung der Transkription bzw. Expression eines Gens von Interesse in einem Expressionssystem führt, und wobei das Derivat mindestens 85% Sequenzidentität aufweist.

3. Nukleinsäure gemäß Anspruch 1 oder 2, wobei die besagte Nukleinsäure die SEQ ID Nr. 1 enthält oder ein Fragment von SEQ ID Nr. 1, welches eine Nukleinsäure gemäß Anspruch 1 oder 2 umfasst, oder deren komplementäre Nukleotidsequenzen oder ein Derivat von SEQ ID Nr. 1 oder deren komplementäre Nukleotidsequenzen, wobei die Nukleinsäure, das Fragment, das Derivat oder deren komplementäre Nukleotidsequenzen bei chromosomaler Integration zu einer Steigerung der Transkription bzw. Expression eines Gens von Interesse in einem Expressionssystem führt, wobei das Fragment bzw. Derivat mindestens einen Sequenzbereich aus dem Nukleinsäurebereich zwischen 1 bp und 1578 bp mit umfasst und wobei das Derivat mindestens 85% Sequenzidentität aufweist.

4. Nukleinsäure gemäß einem der Ansprüche 1 bis 3 **dadurch gekennzeichnet, dass** die Steigerung der Transkription bzw. Expression eines Gens von Interesse in einem Expressionssystem im Vergleich zu einer Kontrolle, die kein TE-Element enthält, durch Messung des Produkttiters bestimmt wird.

5. Nukleinsäure gemäß einem der Ansprüche 1 bis 4 **dadurch gekennzeichnet, dass** diese Nukleinsäure eine Länge von mindestens 511 bp (=Länge TE-13, SEQ ID Nr. 15) bzw. mindestens 1015 bp (=Länge TE-08, SEQ ID Nr. 10) aufweist.

6. Nukleinsäure ausgewählt aus der Gruppe bestehend aus: TE-01 (SEQ ID Nr. 3), TE-02 (SEQ ID Nr. 4), TE-07 (SEQ ID Nr. 9), TE-08 (SEQ ID Nr. 10), TE-10 (SEQ ID Nr. 12), TE-11 (SEQ ID Nr. 13), TE-12 (SEQ ID Nr. 14), TE-13 (SEQ ID Nr. 15), TE-15 (SEQ ID Nr. 17), TE-17 (SEQ ID Nr. 19), TE-18 (SEQ ID Nr. 20).

7. Nukleinsäure gemäß Anspruch 6, wobei die Nukleinsäure TE-08 (SEQ ID Nr. 10) ist.

8. Nukleinsäure gemäß Anspruch 6, wobei die Nukleinsäure TE-13 (SEQ ID Nr. 15) ist.

9. Eukaryontischer Expressionsvektor **dadurch gekennzeichnet, dass** dieser Expressionsvektor eine Nukleinsäure gemäß einem der Ansprüche 1 bis 8 enthält.

10. Eukaryontischer Expressionsvektor gemäß Anspruch 9 **dadurch gekennzeichnet, dass** er eine Kombination mehrerer gleicher oder verschiedener Nukleinsäuren gemäß einem der Ansprüche 1 bis 8 enthält, wobei ein oder mehrere Nukleinsäuren vor (d.h. 5' von) und / oder ein oder mehrere Nukleinsäuren nach (d.h. 3' von) dem Gen von Interesse positioniert sind.

11. Eukaryontischer Expressionsvektor gemäß Anspruch 10 **dadurch gekennzeichnet, dass** eine oder mehrere TE-08-Nukleinsäure(n) (SEQ ID Nr. 10) vor (d.h. 5' von) und eine oder mehrere nach (d.h. 3' von) dem Gen von Interesse positioniert sind, bevorzugt eine TE-08-Nukleinsäure davor und eine danach.

12. Verfahren zur Herstellung eines eukaryontischen Expressionsvektors **gekennzeichnet durch** die Integration einer Nukleinsäure gemäß einem der Ansprüche 1 bis 8 in einen Expressionsvektor.

13. Eukaryontische Wirtszelle **dadurch gekennzeichnet, dass** sie einen eukaryontischen Expressionsvektor gemäß Anspruch 9 bis 11 enthält.

14. Eukaryontische Wirtszelle gemäß Anspruch 13 **dadurch gekennzeichnet, dass** sie hochproduzierend ist, d.h. eine höhere spezifische Produktivität besitzt als eine vergleichbare eukaryontische Wirtszelle ohne TE-Element, wobei diese Wirtszelle einen bis zu zweifach, dreifach, vierfach, fünffach, sechsfach, siebenfach oder zehnfach gesteigerten oder einen mehr als zweifach, mehr als dreifach, mehr als vierfach, mehr als fünffach, mehr als siebenfach, mehr als zehnfach gesteigerten Expressionslevel hat.

15. Verfahren zur Entwicklung einer hochproduzierenden stabil transfizierten eukaryontischen Wirtszelllinie **gekennzeichnet durch** folgende Schritte:
(a) Integration einer Nukleinsäure gemäß einer der Ansprüche 1 bis 8 in einen eukaryontischen Expressionsvektor enthaltend ein Gen von Interesse,
(b) Transfektion einer eukaryontischen Wirtszelle mit diesem Expressionsvektor,
(c) Selektion einer hochproduzierenden transfizierten Wirtszelle.

16. Verfahren zur Herstellung und Selektion von rekombinanten Säugerzellen **gekennzeichnet durch** folgende Schritte:
(a) Transfektion der Wirtszellen mit Genen, die zumindest für ein Protein/Produkt von Interesse, eine Neomycin-Phosphotransferase, vorzugsweise modifiziert, und den amplifzierbaren Selektionsmarker DHFR kodieren, wobei zumindest das Gen (bzw. die Gene) von Interesse zur Transkriptions- bzw. Expressionssteigerung funktionell mit zumindest einer Nukleinsäure gemäß einem der Ansprüche 1 bis 8 verknüpft ist,
(b) Kultivierung der Zellen unter Bedingungen, die eine Expression der verschiedenen Gene ermöglichen,
(c) die Selektion dieser co-integrierten Gene **durch** Kultivierung der Zellen in Gegenwart eines Selektionsmittels, wie z.B. G418, in Hypoxanthin/Thymidinfreiem Medium und
(d) die Amplifikation dieser co-integrierten Gene **durch** Kultivierung der Zellen in Gegenwart eines Selektionsmittels, das die Amplifikation zumindest des amplifizierbaren Selektionsmarkergens erlaubt, wie z.B. Methotrexat.

17. Verfahren gemäß einem der Ansprüche 15 oder 16, wobei der Anteil an Hochproduzenten bis zu zweifach, dreifach, vierfach, fünffach, sechsfach, siebenfach oder zehnfach gesteigerte oder mehr als zweifach, mehr als dreifach, mehr als vierfach, mehr als fünffach, mehr als siebenfach, mehr als zehnfach gesteigert ist.

18. Verfahren zur Herstellung eines biopharmazeutischen Produktes **gekennzeichnet durch** folgende Schritte:
(a) Integration einer Nukleinsäure gemäß einem der Ansprüche 1 bis 8 in einen eukaryontischen Expressionsvektor enthaltend ein Gen von Interesse,
(b) Transfektion einer eukaryontischen Wirtszelle mit diesem Expressionsvektor,
(c) Selektion einer hochproduzierenden transfizierten Wirtszelle und
(d) Kultivierung der erhaltenen hochproduzierenden transfizierten Wirtszelle unter Bedingungen, die eine Expression des (der) Gens (Gene) von Interesse erlauben.

19. Verfahren gemäß Anspruch 18 **gekennzeichnet durch** folgenden zusätzlichen Schritt:
(e) Ernte und Aufreinigung des Proteins von Interesse.

20. Verwendung einer Nukleinsäure gemäß einem der Ansprüche 1 bis 8 zur Herstellung eines Arzneimittels zur medizinischen Verwendung, wobei die Nukleinsäure als transkriptionssteigerndes Element zur Herstellung eines biopharmazeutischen Produktes dient.

21. Verwendung gemäß Anspruch 20, wobei das biopharmazeutische Produkt ein monoklonaler Antikörper ist.

22. Verwendung einer Nukleinsäure gemäß einem der Ansprüche 1 bis 8 zur Erzeugung von nicht humanen transgenen Tieren oder Pflanzen.

23. Kit bestehend aus Nukleinsäure(n) gemäß einem der Ansprüche 1 bis 8, Expressionsvektor(en), Wirtszelle(n) und optional Transfektionsreagenz(ien).

## Claims

1. A nucleic acid comprising TE-13 (SEQ ID No. 15) or a fragment of TE-13 (SEQ ID No. 15) or the complementary nucleotide sequences thereof or a derivative of TE-13 (SEQ ID No. 15) or the complementary nucleotide sequences thereof, wherein the nucleic acid, the fragment, the derivative or the complementary nucleotide sequences thereof on chromosomal integration leads to an increase in the transcription or expression of a gene of interest in an expression system, and wherein the derivative has at least 85% sequence identity.

2. A nucleic acid comprising TE-08 (SEQ ID No. 10) or a fragment of TE-08 (SEQ ID No. 10) or the complementary nucleotide sequences thereof or a derivative of TE-08 (SEQ ID No. 10) or the complementary nucleotide sequences thereof, wherein the nucleic acid, the fragment, the derivative or the complementary nucleotide sequences thereof on chromosomal integration leads to an increase in the transcription or expression of a gene of interest in an expression system, and wherein the derivative has at least 85% sequence identity.

3. A nucleic acid according to claim 1 or 2, wherein the said nucleic acid contains SEQ ID No. 1 or a fragment of SEQ ID No. 1 which comprises a nucleic acid according to claim 1 or 2, or the complementary nucleotide sequences thereof or a derivative of SEQ ID No. 1 or the complementary nucleotide sequences thereof, wherein the nucleic acid, the fragment, the derivative or the complementary nucleotide sequences thereof on chromosomal integration leads to an increase in the transcription or expression of a gene of interest in an expression system, wherein the fragment or derivative also includes at least one sequence region consisting of the nucleic acid region between 1 bp and 1578 bp and wherein the derivative has at least 85% sequence identity.

4. Nucleic acid according to one of claims 1 to 3, **characterised in that** the increase in the transcription or expression of a gene of interest in an expression system in comparison to a control which does not comprise a TE element is determined by measuring the product titre.

5. Nucleic acid according to one of claims 1 to 4, **characterised in that** this nucleic acid has a length of at least 511 bp (= length TE-13, SEQ ID No. 15) or at least 1015 bp (= length TE-08, SEQ ID No. 10).

6. Nucleic acid selected from among : TE-01 (SEQ ID No. 3), TE-02 (SEQ ID No. 4), TE-07 (SEQ ID No. 9), TE-08 (SEQ ID No. 10), TE-10 (SEQ ID No. 12), TE-11 (SEQ ID No. 13), TE-12 (SEQ ID No. 14), TE-13 (SEQ ID No. 15), TE-15 (SEQ ID No. 17), TE-17 (SEQ ID No. 19), TE-18 (SEQ ID No. 20).

7. Nucleic acid according to claim 6, wherein the nucleic acid is TE-08 (SEQ ID No. 10).

8. Nucleic acid according to Claim 6, wherein the nucleic acid is TE-13 (SEQ ID No. 15).

9. Eukaryotic expression vector, **characterised in that** this expression vector comprises a nucleic acid according to one of claims 1 to 8.

10. Eukaryotic expression vector according to Claim 9, **characterised in that** it comprises a combination of several identical or different nucleic acids according to one of claims 1 to 8, wherein one or more nucleic acids are positioned in front of (i.e. 5' of) and / or one or more nucleic acids are positioned behind (i.e. 3' of) the gene of interest.

11. Eukaryotic expression vector according to Claim 10, **characterised in that** one or more TE-08-nucleic acid(s) (SEQ ID No. 10) are positioned in front of (i.e. 5' of) and one or more are positioned behind (i.e. 3' of) the gene of interest, preferably one TE-08-nucleic acid in front of it and one behind.

12. Method of producing a eukaryotic expression vector, **characterised by** the integration of a nucleic acid according to one of claims 1 to 8 in an expression vector.

13. Eukaryotic host cell, **characterised in that** it comprises a eukaryotic expression vector according to Claim 9 to 11.

14. Eukaryotic host cell according to Claim 13, **characterised in that** it is a high producer, i.e. it has a higher specific productivity than a comparable eukaryotic host cell without a TE element, this host cell having an expression level which is increased up to two-fold, three-fold, four-fold, five-fold, six-fold, seven-fold or ten-fold or one which is increased more than two-fold, more than three-fold, more than four-fold, more than five-fold, more than seven-fold or more than ten-fold.

15. Method of developing a high-producing stably transfected eukaryotic host cell line, **characterised by** the following steps:
(a) integrating a nucleic acid according to one of Claims 1 to 8 in a eukaryotic expression vector comprising a gene of interest,
(b) transfecting a eukaryotic host cell with this expression vector,
(c) selecting a high-producing transfected host cell.

16. Method of preparing and selecting recombinant mammalian cells, **characterised by** the following steps:
(a) transfecting the host cells with genes that code at least for a protein/product of interest, a neomycin-phosphotransferase, preferably modified, and the amplifiable selectable marker DHFR, wherein in order to enhance the transcription or expression at least the gene (or genes) of interest is or are functionally linked to at least one nucleic acid according to one of claims 1 to 8,
(b) cultivating the cells under conditions which enable expression of the different genes,
(c) selecting these co-integrated genes by cultivating the cells in the presence of a selecting agent, such as e.g. G418, in a hypoxanthine/thymidine-free medium and
(d) amplifying these co-integrated genes by cultivating the cells in the presence of a selecting agent which allows the amplification of at least the amplifiable selectable marker gene, such as e.g. methotrexate.

17. Method according to one of claims 15 or 16, wherein the proportion of high producers is increased up to two-fold, three-fold, four-fold, five-fold, six-fold, seven-fold or ten-fold or more than two-fold, more than three-fold, more than four-fold, more than five-fold, more than seven-fold or more than ten-fold.

18. Method of preparing a biopharmaceutical product, **characterised by** the following steps:
(a) integrating a nucleic acid according to one of claims 1 to 8 in a eukaryotic expression vector comprising a gene of interest,
(b) transfecting a eukaryotic host cell with this expression vector,
(c) selecting a highly-productive transfected host cell and
(d) cultivating the highly-productive transfected host cell obtained under conditions which allow expression of the gene(s) of interest.

19. Method according to Claim 18, **characterised by** the following additional step:
(e) harvesting and purifying the protein of interest.

20. Use of a nucleic acid according to one of claims 1 to 8 for preparing a medicament for medicinal use, wherein the nucleic acid is used as a transcription-promoting element for the preparation of a biopharmaceutical product.

21. Use according to claim 20, wherein the biopharmaceutical product is a monoclonal antibody.

22. Use of a nucleic acid according to one of claims 1 to 8 for the production of non-human transgenic animals or plants.

23. Kit consisting of nucleic acid(s) according to one of claims 1 to 8, expression vector(s), host cell(s) and optionally transfection reagent(s).

## Revendications

1. Acide nucléique qui contient TE-13 (SEQ ID N° _{:} 15) ou un fragment de TE-13 (SEQ ID N° : 15) ou leurs séquences nucléotidiques complémentaires ou un dérivé de TE-13 (SEQ ID N° : 15) ou leurs séquences nucléotidiques complémentaires, dans lequel l'acide nucléique, le fragment, le dérivé ou leurs séquences nucléotidiques complémentaires entraînent lors de l'intégration chromosomique, une augmentation de la transcription ou de l'expression d'un gène d'intérêt dans un système d'expression, et dans lequel le dérivé présente au moins 85 % d'identité de séquence.

2. Acide nucléique qui contient TE-08 (SEQ ID N° : 10) ou un fragment de TE-08 (SEQ ID N° : 10) ou leurs séquences nucléotidiques complémentaires ou un dérivé de TE-08 (SEQ ID N° : 10) ou leurs séquences nucléotidiques complémentaires, dans lequel l'acide nucléique, le fragment, le dérivé ou leurs séquences nucléotidiques complémentaires entraînent lors de l'intégration chromosomique, une augmentation de la transcription ou de l'expression d'un gène d'intérêt dans un système d'expression, et dans lequel le dérivé présente au moins 85 % d'identité de séquence.

3. Acide nucléique selon la revendication 1 ou 2, dans lequel ledit acide nucléique contient la SEQ ID N° : 1 ou un fragment de SEQ ID N° : 1, qui comprend un acide nucléique selon la revendication 1 ou 2, ou leurs séquences nucléotidiques complémentaires ou un dérivé de SEQ ID N° : 1 ou leurs séquences nucléotidiques complémentaires, dans lequel l'acide nucléique, le fragment, le dérivé ou leurs séquences nucléotidiques complémentaires entraînent lors de l'intégration chromosomique, une augmentation de la transcription ou de l'expression d'un gène d'intérêt dans un système d'expression, dans lequel le fragment ou le dérivé présente au moins un domaine de séquence du domaine d'acide nucléique entre 1 pb et 1578 pb et dans lequel le dérivé présente au moins 85 % d'identité de séquence.

4. Acide nucléique selon l'une quelconque des revendications 1 à 3, **caractérisé en ce que** l'augmentation de la transcription ou de l'expression d'un gène d'intérêt dans un système d'expression par rapport à un témoin qui ne contient pas d'élément TE est déterminée par la mesure du titre du produit.

5. Acide nucléique selon l'une quelconque des revendications 1 à 4, **caractérisé en ce que** cet acide nucléique présente une longueur d'au moins 511 pb (= longueur TE-13, SEQ ID N° : 15) ou d'au moins 1015 pb (=longueur TE-08, SEQ ID N° : 10).

6. Acide nucléique choisi dans le groupe comprenant : TE-01 (SEQ ID N° : 3), TE-02 (SEQ ID N° : 4), TE-07 (SEQ ID N° : 9), TE-08 (SEQ ID N° : 10) , TE-10 (SEQ ID N° : 12 ) , TE-11 (SEQ ID N° : 13 ) , TE-12 (SEQ ID N° : 14 ) , TE-13 (SEQ ID N° : 15 ) , TE-15 (SEQ ID N° : 17 ) , TE-17 (SEQ ID N° : 19 ) , TE-18 (SEQ ID N° : 20).

7. Acide nucléique selon la revendication 6, dans lequel l'acide nucléique est TE-08 (SEQ ID N° : 10).

8. Acide nucléique selon la revendication 6, dans lequel l'acide nucléique est TE-13 (SEQ ID N° : 15).

9. Vecteur d'expression eucaryote, **caractérisé en ce que** ce vecteur d'expression contient un acide nucléique selon l'une quelconque des revendications 1 à 8.

10. Vecteur d'expression eucaryote selon la revendication 9, **caractérisé en ce qu'**il contient une combinaison de plusieurs acides nucléiques identiques ou différents selon l'une quelconque des revendications 1 à 8, dans lequel un ou plusieurs acides nucléiques sont placés avant (c'est-à-dire en 5') et/ou un ou plusieurs acides nucléiques après (c'est-à-dire en 3') le gène d'intérêt.

11. Vecteur d'expression eucaryote selon la revendication 10, **caractérisé en ce qu'**un ou plusieurs acide (s) nucléique (s) TE-08 (SEQ ID N° : 10) sont placés avant (c'est-à-dire en 5') et un ou plusieurs sont placés après (c'est-à-dire en 3') le gène d'intérêt, de préférence un acide nucléique TE-08 avant et un après.

12. Procédé de production d'un vecteur d'expression eucaryote **caractérisé par** l'intégration d'un acide nucléique selon l'une quelconque des revendications 1 à 8 dans un vecteur d'expression.

13. Cellule hôte eucaryote, **caractérisée en ce qu'**elle contient un vecteur d'expression eucaryote selon la revendication 9 à 11.

14. Cellule hôte eucaryote selon la revendication 13, **caractérisée en ce qu'**elle est à production élevée, c'est-à-dire qu'elle possède une productivité spécifique plus élevée qu'une cellule hôte eucaryote comparable sans élément TE, dans laquelle cette cellule hôte a un taux d'expression de une à deux fois, trois fois, quatre fois, cinq fois, six fois, sept fois ou dix fois supérieur ou un taux d'expression de plus de deux fois, de plus de trois fois, de plus de quatre fois, de plus de cinq fois, de plus de sept fois, de plus de dix fois supérieur.

15. Procédé de développement d'une lignée de cellules hôtes eucaryotes transfectées de façon stable, à production élevée **caractérisé par** les étapes suivantes :
(a) intégration d'un acide nucléique selon l'une quelconque des revendications 1 à 8 dans un vecteur d'expression eucaryote contenant un gène d'intérêt,
(b) transfection d'une cellule hôte eucaryote avec ce vecteur d'expression,
(c) sélection d'une cellule hôte transfectée avec une production élevée.

16. Procédé de production et de sélection de cellules de mammifère recombinantes, **caractérisé par** les étapes suivantes :
(a) transfection des cellules hôtes avec des gènes, modifiant de préférence au moins pour une protéine/un produit d'intérêt, une néomycine phosphotransférase et codant pour le marqueur de sélection amplifiable DHFR, dans lequel au moins le gène (ou les gènes ) d'intérêt est/sont relié (s) fonctionnellement, pour l'augmentation de la transcription ou de l'expression avec au moins un acide nucléique selon l'une quelconque des revendications 1 à 8,
(b) culture des cellules dans des conditions qui permettent une expression des différents gènes,
(c) la sélection de ces gènes co-intégrés par culture des cellules en présence d'un agent de sélection tel que par exemple, G418, dans un milieu dépourvu d'hypoxanthine/ thymidine et
(d) l'amplification de ces gènes co-intégrés par culture des cellules en présence d'un agent de sélection qui permet l'amplification d'au moins le gène marqueur de sélection amplifiable, tel que par exemple, le méthotrexate.

17. Procédé selon l'une quelconque des revendications 15 ou 16, dans lequel la proportion de production élevée est augmentée jusqu'à deux fois, trois fois, quatre fois, cinq fois, six fois, sept fois ou dix fois ou est augmentée de plus de deux fois, de plus de trois fois, de plus de quatre fois, de plus de cinq fois, de plus de sept fois, de plus de dix fois.

18. Procédé de production d'un produit biopharmaceutique **caractérisé par** les étapes suivantes :
(a) intégration d'un acide nucléique selon l'une quelconque des revendications 1 à 8 dans un vecteur d'expression eucaryote contenant un gène d'intérêt,
(b) transfection d'une cellule hôte eucaryote avec ce vecteur d'expression,
(c) sélection d'une cellule hôte transfectée avec une production élevée et
(d) culture des cellules hôtes transfectées avec une production élevée obtenues, dans des conditions qui permettent une expression du (des) gène(s) d'intérêt.

19. Procédé selon la revendication 18, **caractérisé par** l'étape supplémentaire suivants :
(c) récolte et purification de la protéine d'intérêt.

20. Utilisation d'un acide nucléique selon l'une quelconque des revendications 1 à 8 pour la production d'un médicament pour l'utilisation médicale, dans laquelle l'acide nucléique sert d'élément d'augmentation de la transcription pour la production d'un produit biopharmaceutique.

21. Utilisation selon la revendication 20, dans laquelle le produit biopharmaceutique est un anticorps monoclonal.

22. Utilisation d'un acide nucléique selon l'une quelconque des revendications 1 à 8 pour la génération d'animaux ou de plantes transgéniques non humains.

23. Kit consistant en acide (s) nucléique (s) selon l'une quelconque des revendications 1 à 8, en vecteur(s) d'expression, en cellule (s) hôte (s) et éventuellement en réactif(s) de transfection.


### SEQUENCE LISTING

<110> Boehringer Ingelheim Pharma GmbH & Co. KG Boehringer Ingelheim Pharma GmbH & Co. KG
<120> Regulatorische Nukleinsäureelemente
<130> P01-2092
<160> 44
<170> PatentIn version 3.3
<210> 1
   <211> 3788
   <212> DNA
   <213> Artificial
<220>
   <223> Cricetulus griseus derivative, additional 8 nucleotides
<400> 1
   ccatgagagc ctgaagacct gagttgatac ccagaaccca gatcaagatg gaggagagaa     60
   ccagccccac taagctgtcc cctgaccccc ataaatgcct ccctgtccag ttatgccaca    120
   caatgatagg tgaatacaga aaaacaccct tcctttagac actaagcgga ttcctcttac    180
   gcataccagt taagtgatag ttcttaggct tcaactcagc actttaaaaa gtttatattt    240
   tgcaatgctg gggactaaat tagggttgtg cacatgctaa gtaagcactc tacttttgta    300
   tcacatttta ataattgtaa gaattaattc gtgaaatagt agctgagaca atagatttgt    360
   ttctttcatg tgggaactgc tgtgtgtgct tcttgctgat gcaaacaagg tcaaatactt    420
   tattccccag tgtctgccta gccctgtaac acttctctat tatacaatga ccacaaataa    480
   ttaggtgagt gggttttgtt tcattttaaa ttgttgctat tttagagaca ggatttcttg    540
   caaacctggt tggtcttaaa ctccgtatgt agctgagaat gaccttgaaa accttcctgt    600
   cccacccctc aaattccaga attatagaca cccaccacat ggcttaataa gtaaacaaca    660
   acaataaaag catgacttct gggtctggag ggagggcttg ccagttaaga gcaatggata    720
   ctttcccata gaacctgggt ttgactccca gcactaacct acatggtgat agtgatgcag    780
   cagacataca tgagggcaac acacacatgg gcacatacac acgcacccgc ccaccatggc    840
   ttttccccca tcacttagac agccatattt aaacgtagtg gagccaggct ggggtggtgg    900
   cccacacctt taatcccagc actccagaag gcagaggtag gcggatctct gtgggtttga    960
   gaccagcctg gtctacaaga gctagttcca ggacagcctc caaagccata gagaaaccct   1020
   atctcaaaaa actgaaacaa caacaacaac aaaacaaaat aaaaaaacaa caaaagaatc   1080
   ttagtggttc agtggttcca cacacaggaa agtagaaagg gccttgatgg gaaggttttc   1140
   agagggagga gtatggatga gacaggatga tagtgaaaag aactcaaatt aattaaatat   1200
   ttgaaactat ctaagaataa aagctaaaat atttaaaatt acagtcaggt agtggtggtg   1260
   cagagggcta agttggtaga cacagtgaga tccaggccag ccagggctac ctagtgagac   1320
   cttgttcaaa taactaataa aatatacaaa ataaaggaga caccacaata attttgaaat 1380
   gtaaaagact aaatttacct tttatattga tgagttggat aaaaaaatca atttaccaga 1440
   gaacataaag tagtcccatc aaagacaaaa gcaatatatg attaaactct aatttaaaag 1500
   tttgttagag cctggcaacg tggcacatac ctttaatccc agcaccaggg agacagaggc 1560
   catcctggtc taaaaagtga tctccaggac agccatggct attacacaga gaaaccctgt 1620
   ctggaaaaac aaaaaattag tgtccatgtg taaatgtgtg gagtatgctt gtcatgccac 1680
   atacagaggt agagggcagt ttatgggagt cagttcctat tcttccttta tgggggacct 1740
   ggggactgaa ctcaggtcat caggcttggc agaaagtgca ttagctcacg gagccttatc 1800
   attggcgaaa gctctctcaa gtagaaaatc aatgtgtttg ctcatagtgc aatcattatg 1860
   tttcgagagg ggaagggtac aatcgttggg gcatgtgtgg tcacatctga atagcagtag 1920
   ctccctagga gaattaattc caagttcttt ggtggtgtat caatgccctt aaaggggtca 1980
   acaacttttt ttccctctga caaaactatc ttcttatgtc cttgtccctc atatttgaag 2040
   tattttattc tttgcagtgt tgaatatcaa ttctagcacc tcagacatgt taggtaagta 2100
   ccctacaact caggttaact aatttaattt aactaattta accccaacac tttttctttg 2160
   tttatccaca tttgtggagt gtgtgtgtgt gtgtgtgtgt gtgtgtgtgt gtgtgtgtgt 2220
   gtgtgtgtgt gtgtgtgtgt gcgcgcgcgc gcgcgctcgg atcattctac cttttgttta 2280
   aaaaatgtta gtccaggggt ggggtgcact gtgaaagtct gagggtaact tgctggggtc 2340
   agttctttcc actataggac agaactccag gtgtcaactc tttactgaca gaaccatcca 2400
   aatagcccta tctaatttta gttttttatt tatttatttt ttgtttttcg agacagggtt 2460
   tctctgtggc tttggaggct gtcctggaac tagctcttgt agaccaggct ggtctcgaac 2520
   tcagagatcc acctgcctct gcctcctgag tgctgggatt aaaggcatgc gccaccaacg 2580
   cttggctcta cctaatttta aaagagattg tgtgtcacaa gggtgtcatg tcgccctgca 2640
   accacccccc ccccaaaaaa aaaaaaaaaa aaacttcact gaagctgaag cacgatgatt 2700
   tggttactct ggctggccaa tgagctctag ggagtctcct gtcaaacaga atctcaacag 2760
   gcgcagcagt cttttttaaa gtggggttac aacacaggtt tttgcatatc aggcatttta 2820
   tctaagctat ttcccagcca aaaatgtgta ttttggaggc agcagagcta atagattaaa 2880
   atgagggaag agcccacaca ggttattagg aagataagca tcttctttat ataaaacaaa 2940
   accaaaccaa actggaggag gtctaccttt agggatggaa gaaaagacat ttagagggtg 3000
   caatagaaag ggcactgagt ttgtgaggtg gaggactggg agagggcgca accgctttaa 3060
   ctgtcctgtt ttgcctattt tttggggaca gcacatgttc ctatttttcc caggatgggc 3120
   aatctccacg tccaaacttg cggtcgagga ctacagtcat tttgcaggtt tccttactgt 3180
   atggctttta aaacgtgcaa aggtgaccat taaccgtttc acgctgggag ggcacgtgcg 3240
   gctcagatgc ttcctctgac tgagggccag gagggggcta cacggaagag gccacacccg   3300
   cacttgggaa gactcgattt gggcttcagc tggctgagac gccccagcag gctcctcggc   3360
   tacaccttca gccccgaatg ccttccggcc cataaccctt cccttctagg catttccggc   3420
   gaggacccac cctcgcgcca aacattcggc cccatccccc ggtcctcacc tgaatctcta   3480
   actctgactc cagagtttag agactataac cagatagccc ggatgtgtgg aactgcatct   3540
   tgggacgagt agttttagca aaaagaaagc gacgaaaaac tacaattccc agacagactt   3600
   gtgttacctc tcttctcatg ctaaacaagc cccctttaaa ggaaagcccc tcttagtcgc   3660
   atcgactgtg taagaaaggc gtttgaaaca ttttaatgtt gggcacaccg tttcgaggac   3720
   cgaaatgaga aagagcatag ggaaacggag cgcccgagct agtctggcac tgcgttagac   3780
   agccgcgg                                                             3788
<210> 2
   <211> 2210
   <212> DNA
   <213> Cricetulus griseus
<400> 2
   gatctccagg acagccatgg ctattacaca gagaaaccct gtctggaaaa acaaaaaatt      60
   agtgtccatg tgtaaatgtg tggagtatgc ttgtcatgcc acatacagag gtagagggca     120
   gtttatggga gtcagttcct attcttcctt tatgggggac ctggggactg aactcaggtc     180
   atcaggcttg gcagaaagtg cattagctca cggagcctta tcattggcga aagctctctc     240
   aagtagaaaa tcaatgtgtt tgctcatagt gcaatcatta tgtttcgaga ggggaagggt     300
   acaatcgttg gggcatgtgt ggtcacatct gaatagcagt agctccctag gagaattaat     360
   tccaagttct ttggtggtgt atcaatgccc ttaaaggggt caacaacttt ttttccctct     420
   gacaaaacta tcttcttatg tccttgtccc tcatatttga agtattttat tctttgcagt     480
   gttgaatatc aattctagca cctcagacat gttaggtaag taccctacaa ctcaggttaa     540
   ctaatttaat ttaactaatt taaccccaac actttttctt tgtttatcca catttgtgga     600
   gtgtgtgtgt gtgtgtgtgt gtgtgtgtgt gtgtgtgtgt gtgtgtgtgt gtgtgtgtgt     660
   gtgcgcgcgc gcgcgcgctc ggatcattct accttttgtt taaaaaatgt tagtccaggg     720
   gtggggtgca ctgtgaaagt ctgagggtaa cttgctgggg tcagttcttt ccactatagg     780
   acagaactcc aggtgtcaac tctttactga cagaaccatc caaatagccc tatctaattt     840
   tagtttttta tttatttatt ttttgttttt cgagacaggg tttctctgtg gctttggagg     900
   ctgtcctgga actagctctt gtagaccagg ctggtctcga actcagagat ccacctgcct     960
   ctgcctcctg agtgctggga ttaaaggcat gcgccaccaa cgcttggctc tacctaattt    1020
   taaaagagat tgtgtgtcac aagggtgtca tgtcgccctg caaccacccc ccccccaaaa    1080
   aaaaaaaaaa aaaaacttca ctgaagctga agcacgatga tttggttact ctggctggcc    1140
   aatgagctct agggagtctc ctgtcaaaca gaatctcaac aggcgcagca gtctttttta   1200
   aagtggggtt acaacacagg tttttgcata tcaggcattt tatctaagct atttcccagc   1260
   caaaaatgtg tattttggag gcagcagagc taatagatta aaatgaggga agagcccaca   1320
   caggttatta ggaagataag catcttcttt atataaaaca aaaccaaacc aaactggagg   1380
   aggtctacct ttagggatgg aagaaaagac atttagaggg tgcaatagaa agggcactga   1440
   gtttgtgagg tggaggactg ggagagggcg caaccgcttt aactgtcctg ttttgcctat   1500
   tttttgggga cagcacatgt tcctattttt cccaggatgg gcaatctcca cgtccaaact   1560
   tgcggtcgag gactacagtc attttgcagg tttccttact gtatggcttt taaaacgtgc   1620
   aaaggtgacc attaaccgtt tcacgctggg agggcacgtg cggctcagat gcttcctctg   1680
   actgagggcc aggagggggc tacacggaag aggccacacc cgcacttggg aagactcgat   1740
   ttgggcttca gctggctgag acgccccagc aggctcctcg gctacacctt cagccccgaa   1800
   tgccttccgg cccataaccc ttcccttcta ggcatttccg gcgaggaccc accctcgcgc   1860
   caaacattcg gccccatccc ccggtcctca cctgaatctc taactctgac tccagagttt   1920
   agagactata accagatagc ccggatgtgt ggaactgcat cttgggacga gtagttttag   1980
   caaaaagaaa gcgacgaaaa actacaattc ccagacagac ttgtgttacc tctcttctca   2040
   tgctaaacaa gcccccttta aaggaaagcc cctcttagtc gcatcgactg tgtaagaaag   2100
   gcgtttgaaa cattttaatg ttgggcacac cgtttcgagg accgaaatga gaaagagcat   2160
   agggaaacgg agcgcccgag ctagtctggc actgcgttag acagccgcgg              2210
<210> 3
   <211> 3005
   <212> DNA
   <213> Artificial
<220>
   <223> Cricetulus griseus with manipulation of the endogenous EeoR1 site by substitution of 4 bases
<400> 3
   gttgctattt tagagacagg atttcttgca aacctggttg gtcttaaact ccgtatgtag     60
   ctgagaatga ccttgaaaac cttcctgtcc cacccctcaa attccagaat tatagacacc   120
   caccacatgg cttaataagt aaacaacaac aataaaagca tgacttctgg gtctggaggg    180
   agggcttgcc agttaagagc aatggatact ttcccataga acctgggttt gactcccagc    240
   actaacctac atggtgatag tgatgcagca gacatacatg agggcaacac acacatgggc    300
   acatacacac gcacccgccc accatggctt ttcccccatc acttagacag ccatatttaa   360
   acgtagtgga gccaggctgg ggtggtggcc cacaccttta atcccagcac tccagaaggc    420
   agaggtaggc ggatctctgt gggtttgaga ccagcctggt ctacaagagc tagttccagg    480
   acagcctcca aagccataga gaaaccctat ctcaaaaaac tgaaacaaca acaacaacaa    540
   aacaaaataa aaaaacaaca aaagaatctt agtggttcag tggttccaca cacaggaaag 600
   tagaaagggc cttgatggga aggttttcag agggaggagt atggatgaga caggatgata 660
   gtgaaaagaa ctcaaattaa ttaaatattt gaaactatct aagaataaaa gctaaaatat 720
   ttaaaattac agtcaggtag tggtggtgca gagggctaag ttggtagaca cagtgagatc 780
   caggccagcc agggctacct agtgagacct tgttcaaata actaataaaa tatacaaaat 840
   aaaggagaca ccacaataat tttgaaatgt aaaagactaa atttaccttt tatattgatg 900
   agttggataa aaaaatcaat ttaccagaga acataaagta gtcccatcaa agacaaaagc 960
   aatatatgat taaactctaa tttaaaagtt tgttagagcc tggcaacgtg gcacatacct 1020
   ttaatcccag caccagggag acagaggcca tcctggtcta aaaagtgatc tccaggacag 1080
   ccatggctat tacacagaga aaccctgtct ggaaaaacaa aaaattagtg tccatgtgta 1140
   aatgtgtgga gtatgcttgt catgccacat acagaggtag agggcagttt atgggagtca 1200
   gttcctattc ttcctttatg ggggacctgg ggactgaact caggtcatca ggcttggcag 1260
   aaagtgcatt agctcacgga gccttatcat tggcgaaagc tctctcaagt agaaaatcaa 1320
   tgtgtttgct catagtgcaa tcattatgtt tcgagagggg aagggtacaa tcgttggggc 1380
   atgtgtggtc acatctgaat agcagtagct ccctaggaga attaattcca agttctttgg 1440
   tggtgtatca atgcccttaa aggggtcaac aacttttttt ccctctgaca aaactatctt 1500
   cttatgtcct tgtccctcat atttgaagta ttttattctt tgcagtgttg aatatcaatt 1560
   ctagcacctc agacatgtta ggtaagtacc ctacaactca ggttaactaa tttaatttaa 1620
   ctaatttaac cccaacactt tttctttgtt tatccacatt tgtggagtgt gtgtgtgtgt 1680
   gtgtgtgtgt gtgtgtgtgt gtgtgtgtgt gtgtgtgtgt gtgtgtgtgc gcgcgcgcgc 1740
   gcgctcggat cattctacct tttgtttaaa aaatgttagt ccaggggtgg ggtgcactgt 1800
   gaaagtctga gggtaacttg ctggggtcag ttctttccac tataggacag aactccaggt 1860
   gtcaactctt tactgacaga accatccaaa tagccctatc taattttagt tttttattta 1920
   tttatttttt gtttttcgag acagggtttc tctgtggctt tggaggctgt cctggaacta 1980
   gctcttgtag accaggctgg tctcgaactc agagatccac ctgcctctgc ctcctgagtg 2040
   ctgggattaa aggcatgcgc caccaacgct tggctctacc taattttaaa agagattgtg 2100
   tgtcacaagg gtgtcatgtc gccctgcaac cacccccccc ccaaaaaaaa aaaaaaaaaa 2160
   acttcactga agctgaagca cgatgatttg gttactctgg ctggccaatg agctctaggg 2220
   agtctcctgt caaacagaat ctcaacaggc gcagcagtct tttttaaagt ggggttacaa 2280
   cacaggtttt tgcatatcag gcattttatc taagctattt cccagccaaa aatgtgtatt 2340
   ttggaggcag cagagctaat agattaaaat gagggaagag cccacacagg ttattaggaa 2400
   gataagcatc ttctttatat aaaacaaaac caaaccaaac tggaggaggt ctacctttag 2460
   ggatggaaga aaagacattt agagggtgca atagaaaggg cactgagttt gtgaggtgga 2520
   ggactgggag agggcgcaac cgctttaact gtcctgtttt gcctattttt tggggacagc   2580
   acatgttcct atttttccca ggatgggcaa tctccacgtc caaacttgcg gtcgaggact   2640
   acagtcattt tgcaggtttc cttactgtat ggcttttaaa acgtgcaaag gtgaccatta   2700
   accgtttcac gctgggaggg cacgtgcggc tcagatgctt cctctgactg agggccagga   2760
   gggggctaca cggaagaggc cacacccgca cttgggaaga ctcgatttgg gcttcagctg   2820
   gctgagacgc cccagcaggc tcctcggcta caccttcagc cccgaatgcc ttccggccca   2880
   taacccttcc cttctaggca tttccggcga ggacccaccc tcgcgccaaa cattcggccc   2940
   catcccccgg tcctcacctg aatctctaac tctgactcca gagtttagag actataacca   3000
   gatag                                                               3005
<210> 4
   <211> 2517
   <212> DNA
   <213> Cricetulus griseus
<400> 4
   caaagccata gagaaaccct atctcaaaaa actgaaacaa caacaacaac aaaacaaaat 60
   aaaaaaacaa caaaagaatc ttagtggttc agtggttcca cacacaggaa agtagaaagg 120
   gccttgatgg gaaggttttc agagggagga gtatggatga gacaggatga tagtgaaaag 180
   aactcaaatt aattaaatat ttgaaactat ctaagaataa aagctaaaat atttaaaatt 240
   acagtcaggt agtggtggtg cagagggcta agttggtaga cacagtgaga tccaggccag 300
   ccagggctac ctagtgagac cttgttcaaa taactaataa aatatacaaa ataaaggaga 360
   caccacaata attttgaaat gtaaaagact aaatttacct tttatattga tgagttggat 420
   aaaaaaatca atttaccaga gaacataaag tagtcccatc aaagacaaaa gcaatatatg 480
   attaaactct aatttaaaag tttgttagag cctggcaacg tggcacatac ctttaatccc 540
   agcaccaggg agacagaggc catcctggtc taaaaagtga tctccaggac agccatggct 600
   attacacaga gaaaccctgt ctggaaaaac aaaaaattag tgtccatgtg taaatgtgtg 660
   gagtatgctt gtcatgccac atacagaggt agagggcagt ttatgggagt cagttcctat 720
   tcttccttta tgggggacct ggggactgaa ctcaggtcat caggcttggc agaaagtgca 780
   ttagctcacg gagccttatc attggcgaaa gctctctcaa gtagaaaatc aatgtgtttg 840
   ctcatagtgc aatcattatg tttcgagagg ggaagggtac aatcgttggg gcatgtgtgg 900
   tcacatctga atagcagtag ctccctagga gaattaattc caagttcttt ggtggtgtat 960
   caatgccctt aaaggggtca acaacttttt ttccctctga caaaactatc ttcttatgtc 1020
   cttgtccctc atatttgaag tattttattc tttgcagtgt tgaatatcaa ttctagcacc 1080
   tcagacatgt taggtaagta ccctacaact caggttaact aatttaattt aactaattta 1140
   accccaacac tttttctttg tttatccaca tttgtggagt gtgtgtgtgt gtgtgtgtgt 1200
   gtgtgtgtgt gtgtgtgtgt gtgtgtgtgt gtgtgtgtgt gcgcgcgcgc gcgcgctcgg 1260
   atcattctac cttttgttta aaaaatgtta gtccaggggt ggggtgcact gtgaaagtct 1320
   gagggtaact tgctggggtc agttctttcc actataggac agaactccag gtgtcaactc 1380
   tttactgaca gaaccatcca aatagcccta tctaatttta gttttttatt tatttatttt 1440
   ttgtttttcg agacagggtt tctctgtggc tttggaggct gtcctggaac tagctcttgt 1500
   agaccaggct ggtctcgaac tcagagatcc acctgcctct gcctcctgag tgctgggatt 1560
   aaaggcatgc gccaccaacg cttggctcta cctaatttta aaagagattg tgtgtcacaa 1620
   gggtgtcatg tcgccctgca accacccccc ccccaaaaaa aaaaaaaaaa aaacttcact 1680
   gaagctgaag cacgatgatt tggttactct ggctggccaa tgagctctag ggagtctcct 1740
   gtcaaacaga atctcaacag gcgcagcagt cttttttaaa gtggggttac aacacaggtt 1800
   tttgcatatc aggcatttta tctaagctat ttcccagcca aaaatgtgta ttttggaggc 1860
   agcagagcta atagattaaa atgagggaag agcccacaca ggttattagg aagataagca 1920
   tcttctttat ataaaacaaa accaaaccaa actggaggag gtctaccttt agggatggaa 1980
   gaaaagacat ttagagggtg caatagaaag ggcactgagt ttgtgaggtg gaggactggg 2040
   agagggcgca accgctttaa ctgtcctgtt ttgcctattt tttggggaca gcacatgttc 2100
   ctatttttcc caggatgggc aatctccacg tccaaacttg cggtcgagga ctacagtcat 2160
   tttgcaggtt tccttactgt atggctttta aaacgtgcaa aggtgaccat taaccgtttc 2220
   acgctgggag ggcacgtgcg gctcagatgc ttcctctgac tgagggccag gagggggcta 2280
   cacggaagag gccacacccg cacttgggaa gactcgattt gggcttcagc tggctgagac 2340
   gccccagcag gctcctcggc tacaccttca gccccgaatg ccttccggcc cataaccctt 2400
   cccttctagg catttccggc gaggacccac cctcgcgcca aacattcggc cccatccccc 2460
   ggtcctcacc tgaatctcta actctgactc cagagtttag agactataac cagatag 2517
<210> 5
   <211> 1989
   <212> DNA
   <213> Cricetulus griseus
<400> 5
   acctttaatc ccagcaccag ggagacagag gccatcctgg tctaaaaagt gatctccagg      60
   acagccatgg ctattacaca gagaaaccct gtctggaaaa acaaaaaatt agtgtccatg     120
   tgtaaatgtg tggagtatgc ttgtcatgcc acatacagag gtagagggca gtttatggga     180
   gtcagttcct attcttcctt tatgggggac ctggggactg aactcaggtc atcaggcttg     240
   gcagaaagtg cattagctca cggagcctta tcattggcga aagctctctc aagtagaaaa     300
   tcaatgtgtt tgctcatagt gcaatcatta tgtttcgaga ggggaagggt acaatcgttg     360
   gggcatgtgt ggtcacatct gaatagcagt agctccctag gagaattaat tccaagttct     420
   ttggtggtgt atcaatgccc ttaaaggggt caacaacttt ttttccctct gacaaaacta 480
   tcttcttatg tccttgtccc tcatatttga agtattttat tctttgcagt gttgaatatc 540
   aattctagca cctcagacat gttaggtaag taccctacaa ctcaggttaa ctaatttaat 600
   ttaactaatt taaccccaac actttttctt tgtttatcca catttgtgga gtgtgtgtgt 660
   gtgtgtgtgt gtgtgtgtgt gtgtgtgtgt gtgtgtgtgt gtgtgtgtgt gtgcgcgcgc 720
   gcgcgcgctc ggatcattct accttttgtt taaaaaatgt tagtccaggg gtggggtgca 780
   ctgtgaaagt ctgagggtaa cttgctgggg tcagttcttt ccactatagg acagaactcc 840
   aggtgtcaac tctttactga cagaaccatc caaatagccc tatctaattt tagtttttta 900
   tttatttatt ttttgttttt cgagacaggg tttctctgtg gctttggagg ctgtcctgga 960
   actagctctt gtagaccagg ctggtctcga actcagagat ccacctgcct ctgcctcctg 1020
   agtgctggga ttaaaggcat gcgccaccaa cgcttggctc tacctaattt taaaagagat 1080
   tgtgtgtcac aagggtgtca tgtcgccctg caaccacccc ccccccaaaa aaaaaaaaaa 1140
   aaaaacttca ctgaagctga agcacgatga tttggttact ctggctggcc aatgagctct 1200
   agggagtctc ctgtcaaaca gaatctcaac aggcgcagca gtctttttta aagtggggtt 1260
   acaacacagg tttttgcata tcaggcattt tatctaagct atttcccagc caaaaatgtg 1320
   tattttggag gcagcagagc taatagatta aaatgaggga agagcccaca caggttatta 1380
   ggaagataag catcttcttt atataaaaca aaaccaaacc aaactggagg aggtctacct 1440
   ttagggatgg aagaaaagac atttagaggg tgcaatagaa agggcactga gtttgtgagg 1500
   tggaggactg ggagagggcg caaccgcttt aactgtcctg ttttgcctat tttttgggga 1560
   cagcacatgt tcctattttt cccaggatgg gcaatctcca cgtccaaact tgcggtcgag 1620
   gactacagtc attttgcagg tttccttact gtatggcttt taaaacgtgc aaaggtgacc 1680
   attaaccgtt tcacgctggg agggcacgtg cggctcagat gcttcctctg actgagggcc 1740
   aggagggggc tacacggaag aggccacacc cgcacttggg aagactcgat ttgggcttca 1800
   gctggctgag acgccccagc aggctcctcg gctacacctt cagccccgaa tgccttccgg 1860
   cccataaccc ttcccttcta ggcatttccg gcgaggaccc accctcgcgc caaacattcg 1920
   gccccatccc ccggtcctca cctgaatctc taactctgac tccagagttt agagactata 1980
   accagatag 1989
<210> 6
   <211> 1512
   <212> DNA
   <213> Cricetulus griseus
<400> 6
   ctatcttctt atgtccttgt ccctcatatt tgaagtattt tattctttgc agtgttgaat 60
   atcaattcta gcacctcaga catgttaggt aagtacccta caactcaggt taactaattt 120
   aatttaacta atttaacccc aacacttttt ctttgtttat ccacatttgt ggagtgtgtg 180
   tgtgtgtgtg tgtgtgtgtg tgtgtgtgtg tgtgtgtgtg tgtgtgtgtg tgtgtgcgcg 240
   cgcgcgcgcg ctcggatcat tctacctttt gtttaaaaaa tgttagtcca ggggtggggt 300
   gcactgtgaa agtctgaggg taacttgctg ggtcagttcc tttccactat aggacagaac 360
   tccaggtgtc aactctttac tgacagaacc atccaaatag ccctatctaa ttttagtttt 420
   ttatttattt attttttgtt tttcgagaca gggtttctct gtggctttgg aggctgtcct 480
   ggaactagct cttgtagacc aggctggtct cgaactcaga gatccacctg cctctgcctc 540
   ctgagtgctg ggattaaagg catgcgccac caacgcttgg ctctacctaa ttttaaaaga 600
   gattgtgtgt cacaagggtg tcatgtcgcc ctgcaaccac ccccccccca aaaaaaaaaa 660
   aaaaaaaact tcactgaagc tgaagcacga tgatttggtt actctggctg gccaatgagc 720
   tctagggagt ctcctgtcaa acagaatctc aacaggcgca gcagtctttt ttaaagtggg 780
   gttacaacac aggtttttgc atatcaggca ttttatctaa gctatttccc agccaaaaat 840
   gtgtattttg gaggcagcag agctaataga ttaaaatgag ggaagagccc acacaggtta 900
   ttaggaagat aagcatcttc tttatataaa acaaaaccaa accaaactgg aggaggtcta 960
   cctttaggga tggaagaaaa gacatttaga gggtgcaata gaaagggcac tgagtttgtg 1020
   aggtggagga ctgggagagg gcgcaaccgc tttaactgtc ctgttttgcc tattttttgg 1080
   ggacagcaca tgttcctatt tttcccagga tgggcaatct ccacgtccaa acttgcggtc 1140
   gaggactaca gtcattttgc aggtttcctt actgtatggc ttttaaaacg tgcaaaggtg 1200
   accattaacc gtttcacgct gggagggcac gtgcggctca gatgcttcct ctgactgagg 1260
   gccaggaggg ggctacacgg aagaggccac acccgcactt gggaagactc gatttgggct 1320
   tcagctggct gagacgcccc agcaggctcc tcggctacac cttcagcccc gaatgccttc 1380
   cggcccataa cccttccctt ctaggcattt ccggcgagga cccaccctcg cgccaaacat 1440
   tcggccccat cccccggtcc tcacctgaat ctctaactct gactccagag tttagagact 1500
   ataaccagat ag 1512
<210> 7
   <211> 1013
   <212> DNA
   <213> Cricetulus griseus
<400> 7
   caggctggtc tcgaactcag agatccacct gcctctgcct cctgagtgct gggattaaag      60
   gcatgcgcca ccaacgcttg gctctaccta attttaaaag agattgtgtg tcacaagggt 120
   gtcatgtcgc cctgcaacca cccccccccc aaaaaaaaaa aaaaaaaaac ttcactgaag 180
   ctgaagcacg atgatttggt tactctggct ggccaatgag ctctagggag tctcctgtca 240
   aacagaatct caacaggcgc agcagtcttt tttaaagtgg ggttacaaca caggtttttg 300
   catatcaggc attttatcta agctatttcc cagccaaaaa tgtgtatttt ggaggcagca 360
   gagctaatag attaaaatga gggaagagcc cacacaggtt attaggaaga taagcatctt 420
   ctttatataa aacaaaacca aaccaaactg gaggaggtct acctttaggg atggaagaaa 480
   agacatttag agggtgcaat agaaagggca ctgagtttgt gaggtggagg actgggagag 540
   ggcgcaaccg ctttaactgt cctgttttgc ctattttttg gggacagcac atgttcctat 600
   ttttcccagg atgggcaatc tccacgtcca aacttgcggt cgaggactac agtcattttg 660
   caggtttcct tactgtatgg cttttaaaac gtgcaaaggt gaccattaac cgtttcacgc 720
   tgggagggca cgtgcggctc agatgcttcc tctgactgag ggccaggagg gggctacacg 780
   gaagaggcca cacccgcact tgggaagact cgatttgggc ttcagctggc tgagacgccc 840
   cagcaggctc ctcggctaca ccttcagccc cgaatgcctt ccggcccata acccttccct 900
   tctaggcatt tccggcgagg acccaccctc gcgccaaaca ttcggcccca tcccccggtc 960
   ctcacctgaa tctctaactc tgactccaga gtttagagac tataaccaga tag 1013
<210> 8
   <211> 381
   <212> DNA
   <213> Artificial
<220>
   <223> mutant /point mutation in a Cricetulus griseus sequence
<400> 8
   cttgcggtcg aggactacag tcattttgca ggtttcctta ctgtatggct tttaaaacgt 60
   gcaaaggtga ccattaaccg tttcacgctg ggagggcacg tgcggctcag atgcttcctc 120
   tgactgaggg ccaggagggg gctacacgga agaggccaca cccgcacttg ggaagactcg 180
   atttgggctt cagctggctg agacgcccca gcaggctcct cggctacacc ttcagccccg 240
   aatgccttcc ggcccataac ccttcccttc taggcatttc cggcgaggac ccaccctcgc 300
   gccaaacatt cggccccatc ccccggtcct cacctgaatc tctaactctg actccagagt 360
   ttagcgacta taaccagata g 381
<210> 9
   <211> 529
   <212> DNA
   <213> Cricetulus griseus
<400> 9
   gcctgaagac ctgagttgat acccagaacc cagatcaaga tggaggagag aaccagcccc      60
   actaagctgt cccctgaccc ccataaatgc ctccctgtcc agttatgcca cacaatgata     120
   ggtgaataca gaaaaacacc cttcctttag acactaagcg gattcctctt acgcatacca     180
   gttaagtgat agttcttagg cttcaactca gcactttaaa aagtttatat tttgcaatgc     240
   tggggactaa attagggttg tgcacatgct aagtaagcac tctacttttg tatcacattt     300
   taataattgt aagaattaat tcgtgaaata gtagctgaga caatagattt gtttctttca     360
   tgtgggaact gctgtgtgtg cttcttgctg atgcaaacaa ggtcaaatac tttattcccc     420
   agtgtctgcc tagccctgta acacttctct attatacaat gaccacaaat aattaggtga     480
   gtgggttttg tttcatttta aattgttgct attttagaga caggatttc                 529
<210> 10
   <211> 1015
   <212> DNA
   <213> Cricetulus griseus
<400> 10
   gcctgaagac ctgagttgat acccagaacc cagatcaaga tggaggagag aaccagcccc 60
   actaagctgt cccctgaccc ccataaatgc ctccctgtcc agttatgcca cacaatgata 120
   ggtgaataca gaaaaacacc cttcctttag acactaagcg gattcctctt acgcatacca 180
   gttaagtgat agttcttagg cttcaactca gcactttaaa aagtttatat tttgcaatgc 240
   tggggactaa attagggttg tgcacatgct aagtaagcac tctacttttg tatcacattt 300
   taataattgt aagaattaat tcgtgaaata gtagctgaga caatagattt gtttctttca 360
   tgtgggaact gctgtgtgtg cttcttgctg atgcaaacaa ggtcaaatac tttattcccc 420
   agtgtctgcc tagccctgta acacttctct attatacaat gaccacaaat aattaggtga 480
   gtgggttttg tttcatttta aattgttgct attttagaga caggatttct tgcaaacctg 540
   gttggtctta aactccgtat gtagctgaga atgaccttga aaaccttcct gtcccacccc 600
   tcaaattcca gaattataga cacccaccac atggcttaat aagtaaacaa caacaataaa 660
   agcatgactt ctgggtctgg agggagggct tgccagttaa gagcaatgga tactttccca 720
   tagaacctgg gtttgactcc cagcactaac ctacatggtg atagtgatgc agcagacata 780
   catgagggca acacacacat gggcacatac acacgcaccc gcccaccatg gcttttcccc 840
   catcacttag acagccatat ttaaacgtag tggagccagg ctggggtggt ggcccacacc 900
   tttaatccca gcactccaga aggcagaggt aggcggatct ctgtgggttt gagaccagcc 960
   tggtctacaa gagctagttc caggacagcc tccaaagcca tagagaaacc ctatc 1015
<210> 11
   <211> 1541
   <212> DNA
   <213> Cricetulus griseus
<400> 11
   gcctgaagac ctgagttgat acccagaacc cagatcaaga tggaggagag aaccagcccc 60
   actaagctgt cccctgaccc ccataaatgc ctccctgtcc agttatgcca cacaatgata 120
   ggtgaataca gaaaaacacc cttcctttag acactaagcg gattcctctt acgcatacca 180
   gttaagtgat agttcttagg cttcaactca gcactttaaa aagtttatat tttgcaatgc 240
   tggggactaa attagggttg tgcacatgct aagtaagcac tctacttttg tatcacattt 300
   taataattgt aagaattaat tcgtgaaata gtagctgaga caatagattt gtttctttca 360
   tgtgggaact gctgtgtgtg cttcttgctg atgcaaacaa ggtcaaatac tttattcccc 420
   agtgtctgcc tagccctgta acacttctct attatacaat gaccacaaat aattaggtga 480
   gtgggttttg tttcatttta aattgttgct attttagaga caggatttct tgcaaacctg 540
   gttggtctta aactccgtat gtagctgaga atgaccttga aaaccttcct gtcccacccc 600
   tcaaattcca gaattataga cacccaccac atggcttaat aagtaaacaa caacaataaa 660
   agcatgactt ctgggtctgg agggagggct tgccagttaa gagcaatgga tactttccca 720
   tagaacctgg gtttgactcc cagcactaac ctacatggtg atagtgatgc agcagacata 780
   catgagggca acacacacat gggcacatac acacgcaccc gcccaccatg gcttttcccc 840
   catcacttag acagccatat ttaaacgtag tggagccagg ctggggtggt ggcccacacc 900
   tttaatccca gcactccaga aggcagaggt aggcggatct ctgtgggttt gagaccagcc 960
   tggtctacaa gagctagttc caggacagcc tccaaagcca tagagaaacc ctatctcaaa 1020
   aaactgaaac aacaacaaca acaaaacaaa ataaaaaaac aacaaaagaa tcttagtggt 1080
   tcagtggttc cacacacagg aaagtagaaa gggccttgat gggaaggttt tcagagggag 1140
   gagtatggat gagacaggat gatagtgaaa agaactcaaa ttaattaaat atttgaaact 1200
   atctaagaat aaaagctaaa atatttaaaa ttacagtcag gtagtggtgg tgcagagggc 1260
   taagttggta gacacagtga gatccaggcc agccagggct acctagtgag accttgttca 1320
   aataactaat aaaatataca aaataaagga gacaccacaa taattttgaa atgtaaaaga 1380
   ctaaatttac cttttatatt gatgagttgg ataaaaaaat caatttacca gagaacataa 1440
   agtagtccca tcaaagacaa aagcaatata tgattaaact ctaatttaaa agtttgttag 1500
   agcctggcaa cgtggcacat acctttaatc ccagcaccag g 1541
<210> 12
   <211> 2020
   <212> DNA
   <213> Cricetulus griseus
<400> 12
   gcctgaagac ctgagttgat acccagaacc cagatcaaga tggaggagag aaccagcccc      60
   actaagctgt cccctgaccc ccataaatgc ctccctgtcc agttatgcca cacaatgata     120
   ggtgaataca gaaaaacacc cttcctttag acactaagcg gattcctctt acgcatacca     180
   gttaagtgat agttcttagg cttcaactca gcactttaaa aagtttatat tttgcaatgc     240
   tggggactaa attagggttg tgcacatgct aagtaagcac tctacttttg tatcacattt     300
   taataattgt aagaattaat tcgtgaaata gtagctgaga caatagattt gtttctttca     360
   tgtgggaact gctgtgtgtg cttcttgctg atgcaaacaa ggtcaaatac tttattcccc     420
   agtgtctgcc tagccctgta acacttctct attatacaat gaccacaaat aattaggtga 480
   gtgggttttg tttcatttta aattgttgct attttagaga caggatttct tgcaaacctg 540
   gttggtctta aactccgtat gtagctgaga atgaccttga aaaccttcct gtcccacccc 600
   tcaaattcca gaattataga cacccaccac atggcttaat aagtaaacaa caacaataaa 660
   agcatgactt ctgggtctgg agggagggct tgccagttaa gagcaatgga tactttccca 720
   tagaacctgg gtttgactcc cagcactaac ctacatggtg atagtgatgc agcagacata 780
   catgagggca acacacacat gggcacatac acacgcaccc gcccaccatg gcttttcccc 840
   catcacttag acagccatat ttaaacgtag tggagccagg ctggggtggt ggcccacacc 900
   tttaatccca gcactccaga aggcagaggt aggcggatct ctgtgggttt gagaccagcc 960
   tggtctacaa gagctagttc caggacagcc tccaaagcca tagagaaacc ctatctcaaa 1020
   aaactgaaac aacaacaaca acaaaacaaa ataaaaaaac aacaaaagaa tcttagtggt 1080
   tcagtggttc cacacacagg aaagtagaaa gggccttgat gggaaggttt tcagagggag 1140
   gagtatggat gagacaggat gatagtgaaa agaactcaaa ttaattaaat atttgaaact 1200
   atctaagaat aaaagctaaa atatttaaaa ttacagtcag gtagtggtgg tgcagagggc 1260
   taagttggta gacacagtga gatccaggcc agccagggct acctagtgag accttgttca 1320
   aataactaat aaaatataca aaataaagga gacaccacaa taattttgaa atgtaaaaga 1380
   ctaaatttac cttttatatt gatgagttgg ataaaaaaat caatttacca gagaacataa 1440
   agtagtccca tcaaagacaa aagcaatata tgattaaact ctaatttaaa agtttgttag 1500
   agcctggcaa cgtggcacat acctttaatc ccagcaccag ggagacagag gccatcctgg 1560
   tctaaaaagt gatctccagg acagccatgg ctattacaca gagaaaccct gtctggaaaa 1620
   acaaaaaatt agtgtccatg tgtaaatgtg tggagtatgc ttgtcatgcc acatacagag 1680
   gtagagggca gtttatggga gtcagttcct attcttcctt tatgggggac ctggggactg 1740
   aactcaggtc atcaggcttg gcagaaagtg cattagctca cggagcctta tcattggcga 1800
   aagctctctc aagtagaaaa tcaatgtgtt tgctcatagt gcaatcatta tgtttcgaga 1860
   ggggaagggt acaatcgttg gggcatgtgt ggtcacatct gaatagcagt agctccctag 1920
   gagaattaat tccaagttct ttggtggtgt atcaatgccc ttaaaggggt caacaacttt 1980
   ttttccctct gacaaaacta tcttcttatg tccttgtccc 2020
<210> 13
   <211> 2516
   <212> DNA
   <213> Cricetulus griseus
<400> 13
   gcctgaagac ctgagttgat acccagaacc cagatcaaga tggaggagag aaccagcccc 60
   actaagctgt cccctgaccc ccataaatgc ctccctgtcc agttatgcca cacaatgata 120
   ggtgaataca gaaaaacacc cttcctttag acactaagcg gattcctctt acgcatacca 180
   gttaagtgat agttcttagg cttcaactca gcactttaaa aagtttatat tttgcaatgc 240
   tggggactaa attagggttg tgcacatgct aagtaagcac tctacttttg tatcacattt 300
   taataattgt aagaattaat tcgtgaaata gtagctgaga caatagattt gtttctttca 360
   tgtgggaact gctgtgtgtg cttcttgctg atgcaaacaa ggtcaaatac tttattcccc 420
   agtgtctgcc tagccctgta acacttctct attatacaat gaccacaaat aattaggtga 480
   gtgggttttg tttcatttta aattgttgct attttagaga caggatttct tgcaaacctg 540
   gttggtctta aactccgtat gtagctgaga atgaccttga aaaccttcct gtcccacccc 600
   tcaaattcca gaattataga cacccaccac atggcttaat aagtaaacaa caacaataaa 660
   agcatgactt ctgggtctgg agggagggct tgccagttaa gagcaatgga tactttccca 720
   tagaacctgg gtttgactcc cagcactaac ctacatggtg atagtgatgc agcagacata 780
   catgagggca acacacacat gggcacatac acacgcaccc gcccaccatg gcttttcccc 840
   catcacttag acagccatat ttaaacgtag tggagccagg ctggggtggt ggcccacacc 900
   tttaatccca gcactccaga aggcagaggt aggcggatct ctgtgggttt gagaccagcc 960
   tggtctacaa gagctagttc caggacagcc tccaaagcca tagagaaacc ctatctcaaa 1020
   aaactgaaac aacaacaaca acaaaacaaa ataaaaaaac aacaaaagaa tcttagtggt 1080
   tcagtggttc cacacacagg aaagtagaaa gggccttgat gggaaggttt tcagagggag 1140
   gagtatggat gagacaggat gatagtgaaa agaactcaaa ttaattaaat atttgaaact 1200
   atctaagaat aaaagctaaa atatttaaaa ttacagtcag gtagtggtgg tgcagagggc 1260
   taagttggta gacacagtga gatccaggcc agccagggct acctagtgag accttgttca 1320
   aataactaat aaaatataca aaataaagga gacaccacaa taattttgaa atgtaaaaga 1380
   ctaaatttac cttttatatt gatgagttgg ataaaaaaat caatttacca gagaacataa 1440
   agtagtccca tcaaagacaa aagcaatata tgattaaact ctaatttaaa agtttgttag 1500
   agcctggcaa cgtggcacat acctttaatc ccagcaccag ggagacagag gccatcctgg 1560
   tctaaaaagt gatctccagg acagccatgg ctattacaca gagaaaccct gtctggaaaa 1620
   acaaaaaatt agtgtccatg tgtaaatgtg tggagtatgc ttgtcatgcc acatacagag 1680
   gtagagggca gtttatggga gtcagttcct attcttcctt tatgggggac ctggggactg 1740
   aactcaggtc atcaggcttg gcagaaagtg cattagctca cggagcctta tcattggcga 1800
   aagctctctc aagtagaaaa tcaatgtgtt tgctcatagt gcaatcatta tgtttcgaga 1860
   ggggaagggt acaatcgttg gggcatgtgt ggtcacatct gaatagcagt agctccctag 1920
   gagaattaat tccaagttct ttggtggtgt atcaatgccc ttaaaggggt caacaacttt 1980
   ttttccctct gacaaaacta tcttcttatg tccttgtccc tcatatttga agtattttat 2040
   tctttgcagt gttgaatatc aattctagca cctcagacat gttaggtaag taccctacaa 2100
   ctcaggttaa ctaatttaat ttaactaatt taaccccaac actttttctt tgtttatcca 2160
   catttgtgga gtgtgtgtgt gtgtgtgtgt gtgtgtgtgt gtgtgtgtgt gtgtgtgtgt 2220
   gtgtgtgtgt gtgcgcgcgc gcgcgcgctc ggatcattct accttttgtt taaaaaatgt 2280
   tagtccaggg gtggggtgca ctgtgaaagt ctgagggtaa cttgctgggg tcagttcttt 2340
   ccactatagg acagaactcc aggtgtcaac tctttactga cagaaccatc caaatagccc 2400
   tatctaattt tagtttttta tttatttatt ttttgttttt cgagacaggg tttctctgtg 2460
   gctttggagg ctgtcctgga actagctctt gtagaccagg ctggtctcga actcag 2516
<210> 14
   <211> 3148
   <212> DNA
   <213> Cricetulus griseus
<400> 14
   gcctgaagac ctgagttgat acccagaacc cagatcaaga tggaggagag aaccagcccc 60
   actaagctgt cccctgaccc ccataaatgc ctccctgtcc agttatgcca cacaatgata 120
   ggtgaataca gaaaaacacc cttcctttag acactaagcg gattcctctt acgcatacca 180
   gttaagtgat agttcttagg cttcaactca gcactttaaa aagtttatat tttgcaatgc 240
   tggggactaa attagggttg tgcacatgct aagtaagcac tctacttttg tatcacattt 300
   taataattgt aagaattaat tcgtgaaata gtagctgaga caatagattt gtttctttca 360
   tgtgggaact gctgtgtgtg cttcttgctg atgcaaacaa ggtcaaatac tttattcccc 420
   agtgtctgcc tagccctgta acacttctct attatacaat gaccacaaat aattaggtga 480
   gtgggttttg tttcatttta aattgttgct attttagaga caggatttct tgcaaacctg 540
   gttggtctta aactccgtat gtagctgaga atgaccttga aaaccttcct gtcccacccc 600
   tcaaattcca gaattataga cacccaccac atggcttaat aagtaaacaa caacaataaa 660
   agcatgactt ctgggtctgg agggagggct tgccagttaa gagcaatgga tactttccca 720
   tagaacctgg gtttgactcc cagcactaac ctacatggtg atagtgatgc agcagacata 780
   catgagggca acacacacat gggcacatac acacgcaccc gcccaccatg gcttttcccc 840
   catcacttag acagccatat ttaaacgtag tggagccagg ctggggtggt ggcccacacc 900
   tttaatccca gcactccaga aggcagaggt aggcggatct ctgtgggttt gagaccagcc 960
   tggtctacaa gagctagttc caggacagcc tccaaagcca tagagaaacc ctatctcaaa 1020
   aaactgaaac aacaacaaca acaaaacaaa ataaaaaaac aacaaaagaa tcttagtggt 1080
   tcagtggttc cacacacagg aaagtagaaa gggccttgat gggaaggttt tcagagggag 1140
   gagtatggat gagacaggat gatagtgaaa agaactcaaa ttaattaaat atttgaaact 1200
   atctaagaat aaaagctaaa atatttaaaa ttacagtcag gtagtggtgg tgcagagggc 1260
   taagttggta gacacagtga gatccaggcc agccagggct acctagtgag accttgttca 1320
   aataactaat aaaatataca aaataaagga gacaccacaa taattttgaa atgtaaaaga 1380
   ctaaatttac cttttatatt gatgagttgg ataaaaaaat caatttacca gagaacataa 1440
   agtagtccca tcaaagacaa aagcaatata tgattaaact ctaatttaaa agtttgttag 1500
   agcctggcaa cgtggcacat acctttaatc ccagcaccag ggagacagag gccatcctgg 1560
   tctaaaaagt gatctccagg acagccatgg ctattacaca gagaaaccct gtctggaaaa 1620
   acaaaaaatt agtgtccatg tgtaaatgtg tggagtatgc ttgtcatgcc acatacagag 1680
   gtagagggca gtttatggga gtcagttcct attcttcctt tatgggggac ctggggactg 1740
   aactcaggtc atcaggcttg gcagaaagtg cattagctca cggagcctta tcattggcga 1800
   aagctctctc aagtagaaaa tcaatgtgtt tgctcatagt gcaatcatta tgtttcgaga 1860
   ggggaagggt acaatcgttg gggcatgtgt ggtcacatct gaatagcagt agctccctag 1920
   gagaattaat tccaagttct ttggtggtgt atcaatgccc ttaaaggggt caacaacttt 1980
   ttttccctct gacaaaacta tcttcttatg tccttgtccc tcatatttga agtattttat 2040
   tctttgcagt gttgaatatc aattctagca cctcagacat gttaggtaag taccctacaa 2100
   ctcaggttaa ctaatttaat ttaactaatt taaccccaac actttttctt tgtttatcca 2160
   catttgtgga gtgtgtgtgt gtgtgtgtgt gtgtgtgtgt gtgtgtgtgt gtgtgtgtgt 2220
   gtgtgtgtgt gtgcgcgcgc gcgcgcgctc ggatcattct accttttgtt taaaaaatgt 2280
   tagtccaggg gtggggtgca ctgtgaaagt ctgagggtaa cttgctgggg tcagttcttt 2340
   ccactatagg acagaactcc aggtgtcaac tctttactga cagaaccatc caaatagccc 2400
   tatctaattt tagtttttta tttatttatt ttttgttttt cgagacaggg tttctctgtg 2460
   gctttggagg ctgtcctgga actagctctt gtagaccagg ctggtctcga actcagagat 2520
   ccacctgcct ctgcctcctg agtgctggga ttaaaggcat gcgccaccaa cgcttggctc 2580
   tacctaattt taaaagagat tgtgtgtcac aagggtgtca tgtcgccctg caaccacccc 2640
   ccccccaaaa aaaaaaaaaa aaaaacttca ctgaagctga agcacgatga tttggttact 2700
   ctggctggcc aatgagctct agggagtctc ctgtcaaaca gaatctcaac aggcgcagca 2760
   gtctttttta aagtggggtt acaacacagg tttttgcata tcaggcattt tatctaagct 2820
   atttcccagc caaaaatgtg tattttggag gcagcagagc taatagatta aaatgaggga 2880
   agagcccaca caggttatta ggaagataag catcttcttt atataaaaca aaaccaaacc 2940
   aaactggagg aggtctacct ttagggatgg aagaaaagac atttagaggg tgcaatagaa 3000
   agggcactga gtttgtgagg tggaggactg ggagagggcg caaccgcttt aactgtcctg 3060
   ttttgcctat tttttgggga cagcacatgt tcctattttt cccaggatgg gcaatctcca 3120
   cgtccaaact tgcggtcgag gactacag 3148
<210> 15
   <211> 511
   <212> DNA
   <213> Cricetulus griseus
<400> 15
   gttgctattt tagagacagg atttcttgca aacctggttg gtcttaaact ccgtatgtag      60
   ctgagaatga ccttgaaaac cttcctgtcc cacccctcaa attccagaat tatagacacc     120
   caccacatgg cttaataagt aaacaacaac aataaaagca tgacttctgg gtctggaggg     180
   agggcttgcc agttaagagc aatggatact ttcccataga acctgggttt gactcccagc     240
   actaacctac atggtgatag tgatgcagca gacatacatg agggcaacac acacatgggc     300
   acatacacac gcacccgccc accatggctt ttcccccatc acttagacag ccatatttaa     360
   acgtagtgga gccaggctgg ggtggtggcc cacaccttta atcccagcac tccagaaggc     420
   agaggtaggc ggatctctgt gggtttgaga ccagcctggt ctacaagagc tagttccagg     480
   acagcctcca aagccataga gaaaccctat c                                    511
<210> 16
   <211> 549
   <212> DNA
   <213> Cricetulus griseus
<400> 16
   caaagccata gagaaaccct atctcaaaaa actgaaacaa caacaacaac aaaacaaaat      60
   aaaaaaacaa caaaagaatc ttagtggttc agtggttcca cacacaggaa agtagaaagg     120
   gccttgatgg gaaggttttc agagggagga gtatggatga gacaggatga tagtgaaaag     180
   aactcaaatt aattaaatat ttgaaactat ctaagaataa aagctaaaat atttaaaatt     240
   acagtcaggt agtggtggtg cagagggcta agttggtaga cacagtgaga tccaggccag     300
   ccagggctac ctagtgagac cttgttcaaa taactaataa aatatacaaa ataaaggaga     360
   caccacaata attttgaaat gtaaaagact aaatttacct tttatattga tgagttggat     420
   aaaaaaatca atttaccaga gaacataaag tagtcccatc aaagacaaaa gcaatatatg     480
   attaaactct aatttaaaag tttgttagag cctggcaacg tggcacatac ctttaatccc     540
   agcaccagg                                                             549
<210> 17
   <211> 1037
   <212> DNA
   <213> Cricetulus griseus
<400> 17
   gttgctattt tagagacagg atttcttgca aacctggttg gtcttaaact ccgtatgtag      60
   ctgagaatga ccttgaaaac cttcctgtcc cacccctcaa attccagaat tatagacacc     120
   caccacatgg cttaataagt aaacaacaac aataaaagca tgacttctgg gtctggaggg     180
   agggcttgcc agttaagagc aatggatact ttcccataga acctgggttt gactcccagc     240
   actaacctac atggtgatag tgatgcagca gacatacatg agggcaacac acacatgggc     300
   acatacacac gcacccgccc accatggctt ttcccccatc acttagacag ccatatttaa     360
   acgtagtgga gccaggctgg ggtggtggcc cacaccttta atcccagcac tccagaaggc     420
   agaggtaggc ggatctctgt gggtttgaga ccagcctggt ctacaagagc tagttccagg     480
   acagcctcca aagccataga gaaaccctat ctcaaaaaac tgaaacaaca acaacaacaa     540
   aacaaaataa aaaaacaaca aaagaatctt agtggttcag tggttccaca cacaggaaag     600
   tagaaagggc cttgatggga aggttttcag agggaggagt atggatgaga caggatgata     660
   gtgaaaagaa ctcaaattaa ttaaatattt gaaactatct aagaataaaa gctaaaatat     720
   ttaaaattac agtcaggtag tggtggtgca gagggctaag ttggtagaca cagtgagatc     780
   caggccagcc agggctacct agtgagacct tgttcaaata actaataaaa tatacaaaat     840
   aaaggagaca ccacaataat tttgaaatgt aaaagactaa atttaccttt tatattgatg     900
   agttggataa aaaaatcaat ttaccagaga acataaagta gtcccatcaa agacaaaagc     960
   aatatatgat taaactctaa tttaaaagtt tgttagagcc tggcaacgtg gcacatacct    1020
   ttaatcccag caccagg                                                   1037
<210> 18
   <211> 500
   <212> DNA
   <213> Cricetulus griseus
<400> 18
   acctttaatc ccagcaccag ggagacagag gccatcctgg tctaaaaagt gatctccagg      60
   acagccatgg ctattacaca gagaaaccct gtctggaaaa acaaaaaatt agtgtccatg     120
   tgtaaatgtg tggagtatgc ttgtcatgcc acatacagag gtagagggca gtttatggga     180
   gtcagttcct attcttcctt tatgggggac ctggggactg aactcaggtc atcaggcttg     240
   gcagaaagtg cattagctca cggagcctta tcattggcga aagctctctc aagtagaaaa     300
   tcaatgtgtt tgctcatagt gcaatcatta tgtttcgaga ggggaagggt acaatcgttg     360
   gggcatgtgt ggtcacatct gaatagcagt agctccctag gagaattaat tccaagttct     420
   ttggtggtgt atcaatgccc ttaaaggggt caacaacttt ttttccctct gacaaaacta     480
   tcttcttatg tccttgtccc                                                 500
<210> 19
   <211> 1028
   <212> DNA
   <213> Cricetulus griseus
<400> 19
   caaagccata gagaaaccct atctcaaaaa actgaaacaa caacaacaac aaaacaaaat      60
   aaaaaaacaa caaaagaatc ttagtggttc agtggttcca cacacaggaa agtagaaagg     120
   gccttgatgg gaaggttttc agagggagga gtatggatga gacaggatga tagtgaaaag     180
   aactcaaatt aattaaatat ttgaaactat ctaagaataa aagctaaaat atttaaaatt     240
   acagtcaggt agtggtggtg cagagggcta agttggtaga cacagtgaga tccaggccag     300
   ccagggctac ctagtgagac cttgttcaaa taactaataa aatatacaaa ataaaggaga     360
   caccacaata attttgaaat gtaaaagact aaatttacct tttatattga tgagttggat     420
   aaaaaaatca atttaccaga gaacataaag tagtcccatc aaagacaaaa gcaatatatg     480
   attaaactct aatttaaaag tttgttagag cctggcaacg tggcacatac ctttaatccc     540
   agcaccaggg agacagaggc catcctggtc taaaaagtga tctccaggac agccatggct     600
   attacacaga gaaaccctgt ctggaaaaac aaaaaattag tgtccatgtg taaatgtgtg     660
   gagtatgctt gtcatgccac atacagaggt agagggcagt ttatgggagt cagttcctat     720
   tcttccttta tgggggacct ggggactgaa ctcaggtcat caggcttggc agaaagtgca     780
   ttagctcacg gagccttatc attggcgaaa gctctctcaa gtagaaaatc aatgtgtttg     840
   ctcatagtgc aatcattatg tttcgagagg ggaagggtac aatcgttggg gcatgtgtgg     900
   tcacatctga atagcagtag ctccctagga gaattaattc caagttcttt ggtggtgtat     960
   caatgccctt aaaggggtca acaacttttt ttccctctga caaaactatc ttcttatgtc    1020
   cttgtccc                                                             1028
<210> 20
   <211> 1516
   <212> DNA
   <213> Cricetulus griseus
<400> 20
   gttgctattt tagagacagg atttcttgca aacctggttg gtcttaaact ccgtatgtag      60
   ctgagaatga ccttgaaaac cttcctgtcc cacccctcaa attccagaat tatagacacc     120
   caccacatgg cttaataagt aaacaacaac aataaaagca tgacttctgg gtctggaggg     180
   agggcttgcc agttaagagc aatggatact ttcccataga acctgggttt gactcccagc     240
   actaacctac atggtgatag tgatgcagca gacatacatg agggcaacac acacatgggc     300
   acatacacac gcacccgccc accatggctt ttcccccatc acttagacag ccatatttaa     360
   acgtagtgga gccaggctgg ggtggtggcc cacaccttta atcccagcac tccagaaggc     420
   agaggtaggc ggatctctgt gggtttgaga ccagcctggt ctacaagagc tagttccagg     480
   acagcctcca aagccataga gaaaccctat ctcaaaaaac tgaaacaaca acaacaacaa     540
   aacaaaataa aaaaacaaca aaagaatctt agtggttcag tggttccaca cacaggaaag     600
   tagaaagggc cttgatggga aggttttcag agggaggagt atggatgaga caggatgata     660
   gtgaaaagaa ctcaaattaa ttaaatattt gaaactatct aagaataaaa gctaaaatat     720
   ttaaaattac agtcaggtag tggtggtgca gagggctaag ttggtagaca cagtgagatc     780
   caggccagcc agggctacct agtgagacct tgttcaaata actaataaaa tatacaaaat     840
   aaaggagaca ccacaataat tttgaaatgt aaaagactaa atttaccttt tatattgatg     900
   agttggataa aaaaatcaat ttaccagaga acataaagta gtcccatcaa agacaaaagc     960
   aatatatgat taaactctaa tttaaaagtt tgttagagcc tggcaacgtg gcacatacct    1020
   ttaatcccag caccagggag acagaggcca tcctggtcta aaaagtgatc tccaggacag    1080
   ccatggctat tacacagaga aaccctgtct ggaaaaacaa aaaattagtg tccatgtgta    1140
   aatgtgtgga gtatgcttgt catgccacat acagaggtag agggcagttt atgggagtca    1200
   gttcctattc ttcctttatg ggggacctgg ggactgaact caggtcatca ggcttggcag    1260
   aaagtgcatt agctcacgga gccttatcat tggcgaaagc tctctcaagt agaaaatcaa    1320
   tgtgtttgct catagtgcaa tcattatgtt tcgagagggg aagggtacaa tcgttggggc    1380
   atgtgtggtc acatctgaat agcagtagct ccctaggaga attaattcca agttctttgg    1440
   tggtgtatca atgcccttaa aggggtcaac aacttttttt ccctctgaca aaactatctt    1500
   cttatgtcct tgtccc                                                    1516
<210> 21
   <211> 381
   <212> DNA
   <213> Cricetulus griseus
<400> 21
   cttgcggtcg aggactacag tcattttgca ggtttcctta ctgtatggct tttaaaacgt      60
   gcaaaggtga ccattaaccg tttcacgctg ggagggcacg tgcggctcag atgcttcctc     120
   tgactgaggg ccaggagggg gctacacgga agaggccaca cccgcacttg ggaagactcg     180
   atttgggctt cagctggctg agacgcccca gcaggctcct cggctacacc ttcagccccg     240
   aatgccttcc ggcccataac ccttcccttc taggcatttc cggcgaggac ccaccctcgc     300
   gccaaacatt cggccccatc ccccggtcct cacctgaatc tctaactctg actccagagt     360
   ttagagacta taaccagata g                                               381
<210> 22
   <211> 34
   <212> DNA
   <213> Artificial
<220>
   <223> primer
<400> 22
   ctatgaggat ccgcctgaag acctgagttg atac 34
<210> 23
   <211> 37
   <212> DNA
   <213> Artificial
<220>
   <223> primer
<400> 23
   tatgcaggat ccgttgctat tttagagaca ggatttc 37
<210> 24
   <211> 35
   <212> DNA
   <213> Artificial
<220>
   <223> primer
<400> 24
   tatgcaggat cccaaagcca tagagaaacc ctatc 35
<210> 25
   <211> 33
   <212> DNA
   <213> Artificial
<220>
   <223> primer
<400> 25
   tatgcaggat ccacctttaa tcccagcacc agg 33
<210> 26
   <211> 35
   <212> DNA
   <213> Artificial
<220>
   <223> primer
<400> 26
   ctatgaggat ccctatcttc ttatgtcctt gtccc 35
<210> 27
   <211> 32
   <212> DNA
   <213> Artificial
<220>
   <223> primer
<400> 27
   tatgcaggat cccaggetgg tctcgaactc ag 32
<210> 28
   <211> 32
   <212> DNA
   <213> Artificial
<220>
   <223> primer
<400> 28
   ctatgaggat cccttgcggt egaggactac ag 32
<210> 29
   <211> 34
   <212> DNA
   <213> Artificial
<220>
   <223> primer
<400> 29
   ctatgatgta cagcctgaag acctgagttg atac 34
<210> 30
   <211> 39
   <212> DNA
   <213> Artificial
<220>
   <223> primer
<400> 30
   attgcatgta cactatctgg ttatagtctc taaactctg 39
<210> 31
   <211> 37
   <212> DNA
   <213> Artificial
<220>
   <223> primer
<400> 31
   atagcatgta cagaaatcct gtctctaaaa tagcaac 37
<210> 32
   <211> 35
   <212> DNA
   <213> Artificial
<220>
   <223> primer
<400> 32
   atagcatgta cagatagggt ttctctatgg ctttg 35
<210> 33
   <211> 33
   <212> DNA
   <213> Artificial
<220>
   <223> primer
<400> 33
   atacgatgta cacctggtgc tgggattaaa ggt 33
<210> 34
   <211> 35
   <212> DNA
   <213> Artificial
<220>
   <223> primer
<400> 34
   atagcatgta cagggacaag gacataagaa gatag 35
<210> 35
   <211> 32
   <212> DNA
   <213> Artificial
<220>
   <223> primer
<400> 35
   tagttatgta cactgagtte gagaccagcc tg 32
<210> 36
   <211> 32
   <212> DNA
   <213> Artificial
<220>
   <223> primer
<400> 36
   atagcatgta cactgtagtc ctcgaccgca ag 32
<210> 37
   <211> 33
   <212> DNA
   <213> Artificial
<220>
   <223> primer
<400> 37
   atacgaggat cccctggtgc tgggattaaa ggt 33
<210> 38
   <211> 35
   <212> DNA
   <213> Artificial
<220>
   <223> primer
<400> 38
   atagcaggat ccgatagggt ttctetatgg ctttg 35
<210> 39
   <211> 24
   <212> DNA
   <213> Artificial
<220>
   <223> primer
<400> 39
   ctccacacat ttacacatgg acac 24
<210> 40
   <211> 24
   <212> DNA
   <213> Artificial
<220>
   <223> primer
<400> 40
   gggtttctct gtgtaatagc catg 24
<210> 41
   <211> 24
   <212> DNA
   <213> Artificial
<220>
   <223> primer
<400> 41
   atctcactgt gtctaccaac ttag 24
<210> 42
   <211> 22
   <212> DNA
   <213> Artificial
<220>
   <223> primer
<400> 42
   tctgcaccac cactacctga ct 22
<210> 43
   <211> 28
   <212> DNA
   <213> Artificial
<220>
   <223> primer
<400> 43
   ctaagagtac ttgccatgag agcctgaa 28
<210> 44
   <211> 25
   <212> DNA
   <213> Artificial
<220>
   <223> primer
<400> 44
   cattgataca ccaccaaaga acttg 25